# FASCICULE DE BREVET EUROPEEN

(11) **EP 2 268 652 B1**
(45) Date de publication et mention de la délivrance du brevet: **19.12.2012**
(21) Numéro de dépôt: 09724326.5
(22) Date de dépôt: 02.03.2009
(51) Int. Cl.: C07F 15/00, A61K 31/4196, A61K 31/4164, A61P 35/00

(54) **DÉRIVÉS PLATINE - CARBENE N-HETEROCYCLIQUE, LEUR PRÉPARATION ET LEUR APPLICATION EN THÉRAPEUTIQUE**
PLATINUM-N-HETEROCYCLISCHE CARBENDERIVATE, IHRE HERSTELLUNG UND THERAPEUTISCHE VERWENDUNG
PLATINUM-N-HETEROCYCLIC CARBENE DERIVATIVES, PREPARATION THEREOF AND THERAPEUTIC USE THEREOF

(30) Priorité: 03.03.2008 FR 0801155
(43) Date de publication de la demande: 05.01.2011
(73) Titulaire: SANOFI, 75008 Paris (FR); Centre National de la Recherche Scientifique, 75794 Paris Cedex 16 (FR)
(72) Inventeur: MAILLIET, Patrick, F-75013 Paris (FR); MARINETTI, Angela, F-75794 Paris Cedex 16 (FR); SKANDER, Myriem, F-75794 Paris Cedex 16 (FR)
(74) Mandataire: Gaslonde, Aude
(86) Numéro de dépôt international: PCT/FR2009/000220
(87) Numéro de publication internationale: WO 2009/118475

(56) Documents cités:
- RAY S ET AL: "Anticancer and antimicrobial metallopharmaceutical agents based on palladium, gold, and silver N-heterocyclic carbene complexes" JOURNAL OF THE AMERICAN CHEMICAL SOCIETY 20071205 AMERICAN CHEMICAL SOCIETY US, vol. 129, no. 48, 5 décembre 2007 (2007-12-05), pages 15042-15053, XP002496248 cité dans la demande
- FANTASIA S ET AL: "Electronic properties of N-heterocyclic carbene (NHC) ligands: Synthetic, structural, and spectroscopic studies of (NHC)platinum(II) complexes" ORGANOMETALLICS 20071119 AMERICAN CHEMICAL SOCIETY US, vol. 26, no. 24, 19 novembre 2007 (2007-11-19), pages 5880-5889, XP002496249
- ANDREA BIFFIS *, CRISTINA TUBARO, GABRIELLA BUSCEMI, MARINO BASATO: "Highly Efficient Alkyne Hydroarylation with Chelating Dicarbene Palladium(II) and Platinum(II) Complexes" ADVANCED SYNTHESIS & CATALYSIS, vol. 350, no. 1, 23 novembre 2007 (2007-11-23), pages 189-196, XP002496250
- GHEZZI ANNARITA ET AL: "Uptake of antitumor platinum(II)-complexes by cancer cells, assayed by inductively coupled plasma mass spectrometry (ICP-MS)." JOURNAL OF INORGANIC BIOCHEMISTRY, vol. 98, no. 1, janvier 2004 (2004-01), pages 73-78, XP002496251 ISSN: 0162-0134
- KOVALA-DEMERTZI DIMITRA ET AL: "Platinum(II) and palladium(II) complexes with 2-acetyl pyridine 4N-ethyl thiosemicarbazone able to overcome the cis-platin resistance. Structure, antibacterial activity and DNA strand breakage." BIOMETALS : AN INTERNATIONAL JOURNAL ON THE ROLE OF METAL IONS IN BIOLOGY, BIOCHEMISTRY, AND MEDICINE SEP 2003, vol. 16, no. 3, septembre 2003 (2003-09), pages 411-418, XP002496252 ISSN: 0966-0844

## Description

La présente invention se rapporte à des dérivés de platine portant un ligand carbène hétérocyclique, appelés de façon abrégé platine-carbène N-hétérocycliques ou NHC-Pt, à leur préparation et à leur application en thérapeutique.

Certains dérivés du platine sont bien connus comme agent anticancéreux. Les propriétés anticancéreuses du premier d'entre eux, le cisplatine, ont été découvertes en 1965. Le cisplatine a été approuvé par la FDA en 1978, puis ont suivi les accords donnés, toujours par la FDA, au carboplatine en 1989 et à l'oxaliplatine en 2002 et enfin le satraplatine et le picoplatine font actuellement l'objet de demandes de mise sur le marché auprès de la FDA. Le platine se trouve, dans la majorité de ces dérivés, à son degré d'oxydation II, il est toujours coordonné à deux ligands amine et, selon le cas, à des ligands chlorure pour le cisplatine ou à un dérivé d'acide organique dicarboxylique tel que l'acide 1,1-cyclobutanedicarboxylique (appelé "CBDCA") pour le carboplatine, ou l'acide oxalique pour l'oxaliplatine. Le picoplatine est également un dérivé de platine II portant deux ligands azotés, dont l'un est l'α-picoline. Le satraplatine est constitué d'un platine IV lié toujours à deux ligands azotés, à deux chlorures et à deux groupes acétate.

Ces composés peuvent être représentés par les formules suivantes

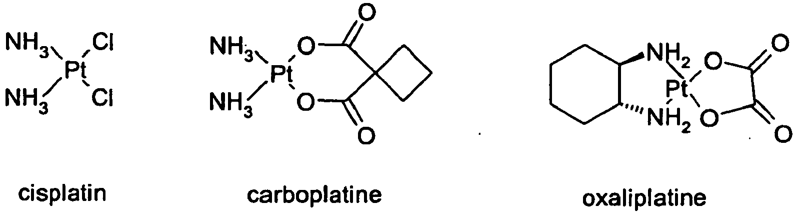

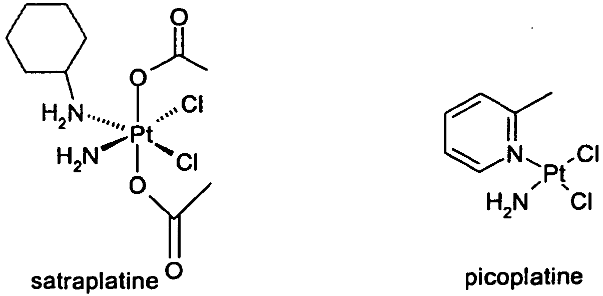

Parmi les différents ligands récemment développés en chimie organométallique, les carbènes riches en électrons et plus précisément les carbènes *N*-hétérocycliques sont très prometteurs. Les premiers complexes de NHCs ont été étudiés vers la fin des années 60 par Öfele (J. Organomet. Chem. 1968, 12, P-42) et Wanzlick (Angew. Chem. Int. Ed. Engl. 1968, 7, 141), mais ce n'est qu'à partir de 1991 que ces composés ont connu un grand développement grâce aux travaux d'Arduengo qui ont permis d'isoler le premier diaminocarbène stable *(*J. Am. Chem. Soc. 1991, 113, 361).

Les carbènes *N*-hétérocycliques sont des composés neutres possédant un carbone divalent à six électrons de valence situé entre deux azotes. Ils sont accessibles entre autres à partir des sels d'imidazolium correspondants ou des oléfines appelées « dimères de Wanzlick ». D'un point de vue électronique, un carbène se compose d'une orbitale σ située dans le plan du cycle et d'une orbitale p_{π} perpendiculaire à celui-ci. Dans le cas des diaminocarbènes, la présence des azotes en a favorise un état électronique de singulet de spin où les deux électrons sont dans l'orbitale σ (Gleiter, R. et al. ; J. Am. Chem. Soc. 1968, 90, 5457).

Les NHCs forment avec les métaux de transition des complexes très stables. Il s'agit de complexes de type Fischer dans lesquels la liaison métal-NHC se caractérise par une forte donation a du carbène vers le métal et d'une faible rétrodonation π (Hu, X. et al. ; Organometallics 2004, 23, 755 ; L. Cavallo et al. J. Organomet Chem. 2005, 690, 5407).

Les premières applications en catalyse organométallique ont concerné des réactions de couplage C-C de type Heck et Suzuki. Pour ces réactions, les complexes palladium-carbène sont des catalyseurs extrêmement intéressants en raison de la forte stabilité de la liaison métal-NHC et de leur préparation facile (Herrmann, W. A. et al. ; Angew. Chem. 1995, 107, 2602). Très souvent les complexes de NHCs se sont révélés plus efficaces en catalyse homogène que les complexes analogues portant des phosphines. L'exemple le plus connu concerne les réactions de métathèse d'oléfines. En effet, les travaux de Grubbs (Acc. Chem. Res. 2001, 34, 18) impliquant le remplacement de ligands phosphine par des NHCs, ont permis d'accéder à des catalyseurs de ruthénium, dits « catalyseurs de Grubbs de seconde génération», très performants en raison de leur grande tolérance aux groupes fonctionnels et leurs conditions de réaction douces. Il existe de nombreuses autres applications en catalyse organométallique et on compte aujourd'hui une grande variété de complexes métal-NHC. En effet, la structure facilement modulable des carbènes *N*-hétérocycliques permet d'introduire assez facilement des groupements chiraux, des groupes fonctionnels, d'effectuer des immobilisations sur support, de créer des ligands bidentés chélatants...etc. (Herrmann, W. A. ; Angew. Chem. Int. Ed. 2002, 41, 1290).

Il existe dans la littérature quelques exemples d'application de complexes carbéniques dans le domaine biomédical. Des complexes d'argent(I) comportant un ligand bis-carbène, ou un dérivé de la caféine (Youngs, W. J. et coll. ; J. Med. Chem. 2006, 49, 6811), ont été synthétisés afin d'obtenir des composés qui possèdent des propriétés antimicrobiennes et antifongiques. Le remplacement du métal, au niveau de ces structures, par un élément radioactif, comme un radio-isotope du Rhodium, de l'Argent, du Gallium ou du Technétium, permet des applications en imagerie médicale ou pour le traitement de cellules cancéreuses. (Youngs, W. J. et coll. ; WO 2005023760 A2)

D'autre part, des complexes carbéniques bis-chélatés d'or(I), ont été synthétisés dans le but d'étudier leur activité antitumorale, par effet antimitochondrial, en comparaison avec les complexes [Au(dppe)₂]⁺ déjà connus. (Bamard, P. J. et al. ; J. Inorg. Biochem. 2004, 10, 1642)

Enfin, un article de S. Ray, R. Mohan, J. K. Singh, M. K. Samantaray, M. M. Shaikh, D. Panda et P. Ghosh, dans le Journal of the American Chemical Society, vol. 48, p. 15042 à 15053, paru le 5 décembre 2007 sous le titre «Anticancer and Antimicrobial Metallopharmaceutical Agents Based on Palladium, Gold, and Silver N-Heterocyclic Carbene Complexes » décrit parmi les dérivés de métaux liés à un carbène N-hétérocyclique, des complexes de palladium, d'or ou d'argent. Dans cet article sont considérés comme actifs en oncologie, uniquement les complexes de palladium et particulièrement les dérivés suivants :

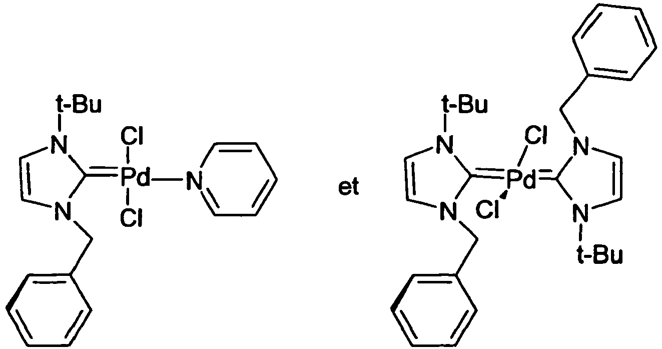

La présente invention a pour objet des complexes platine - carbène N-Hétérocyclique (NHC) portant en trans du carbène un ligand azoté et répondant à la formule générale (I) 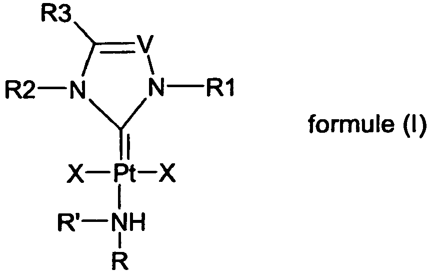 dans laquelle
- R₁ et/ou- R₂ représentent indépendamment l'un de l'autre un groupe aryle ou aralkyle chacun éventuellement substitué, un groupe alkyle en C1-C6 linéaire ou ramifié, cycloalkyle monocyclique en C3-C7, alkényle en C2-C6 linéaire ou ramifié
- ou bien R' représente un atome d'hydrogène et R représente un groupe choisi parmi les groupes cycloalkyle, hétérocycloalkyle mono ou bicyclique en C3-C8 ou benzyle éventuellement substitué
- ou bien R et R' forment ensemble avec NH un hétérocycloalkyle en mono ou bicyclique en C3-C8
- V représente un atome d'azote ou un radical C-R₄
- R₃ et/ou R₄ représentent l'hydrogène, un groupe phényle ou R₃ et R₄ peuvent aussi former ensembles un radical alkylène en C3-C6, hétéroalkylène en C3-C6 avec un ou plusieurs hétéroatomes azotés, les atomes de carbones du radical hétéroalkylène pouvant être modifiés sous forme de radical carbonyle
- X représente l'iode, le brome, le chlore ou un groupe nitrato (-ONO2)

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un groupe alkyle : un groupe aliphatique saturé linéaire ou ramifié en C1-C6. A titre d'exemples, on peut citer les groupes méthyle, éthyle, propyle, isopropyle, butyle, isobutyle, tertbutyle, pentyle, hexyle;
- un groupe cycloalkyle : un groupe alkyle monocyclique en C3-C7 ou bicyclique en C4-C8. A titre d'exemples de groupes alkyles monocyclique en C3-C7 on peut citer les groupes cyclopropyle, cyclobutyle, cyclopentyle, cyclohexyle ; à titre d'exemples de groupes bicycliques en C6-C8 on peut citer le bicyclo[2.2.1]heptyle, le bicyclo[2.2.2]octyle et le bicyclo[3.2.1]octyle ;
- un groupe alkényle : un groupe aliphatique mono- ou polyinsaturé, linéaire ou ramifié, comprenant par exemple une ou deux insaturations éthyléniques et contenant 2 à 6 atomes de carbone;
- un groupe alkylène : un groupe aliphatique saturé linéaire ou ramifié ayant une liaison libre à chaque extrémité ;
- un groupe hétéroalkylène : un groupe aliphatique mono ou polyinsaturé, linéaire ou ramifié en C2-C3 avec un ou plusieurs hétéroatomes azotés ;
- un groupe aryle : un groupe aromatique monocyclique. A titre d'exemples de groupes aryles, on peut citer les groupes phényles. Ces groupes sont éventuellement substitués par un groupe alkoxy en C1-C2 ou par un groupe perfluoroalkyle en C1-C2.
- un groupe aralkyle : un groupe aromatique monocyclique relié par un motif alkylène en C1-C2.

Parmi les composés de formule **(I)** objets de l'invention, un premier groupe de composés est constitué par les composés pour lesquels :
R1 et/ou R2 représentent indépendamment l'un de l'autre un groupe alkyle en C1-C4,
et/ou V représente N ou C-R₄
et/ou R3 et/ou R4 représentent l'hydrogène ou forment ensemble un radical alkénylène
et/ou R' = H et R représente un motif cyclohexyle ou norbornyle ou 4-amino-tétrahydropyranyle
et/ou R et R' forment avec NH une morpholine
et/ou X est l'iode.

Parmi les composés de formule (I) objets de l'invention, un deuxième groupe de composés est constitué par les composés pour lesquels :
R1 et/ou R2 représentent indépendamment l'un de l'autre un benzyle et/ou un alkyle ;
et/ou V représente N ou C-R₄
et/ou R3 et/ou R4 représentent l'hydrogène ou forment ensemble un radical alkénylène ;
et/ou R représente un motif cyclohexyle ou norbornyle ou 4-amino-tétrahydropyranyle et R' est H et/ou R et R' forment avec NH une morpholine
et/ou X est l'iode.

Parmi les composés de formule (I) objets de l'invention, un troisième groupe de composés est constitué par les composés pour lesquels :
R1 et/ou R2 représentent indépendamment l'un de l'autre un groupe cyclohexylméthylène et/ou un groupe alkyle,
et/ou V représente N ou C-R₄
et/ou R3 et/ou R4 représentent l'hydrogène ou forment ensemble un radical alkénylène
et/ou R' = H et R représente un motif cyclohexyle ou norbornyle ou 4-amino-tétrahydropyranyle
et/ou R et R' forment avec NH une morpholine
et/ou X est l'iode.
Parmi les composés du groupe deux on peut substituer le groupe benzyle par un motif CF3 ou méthoxy.

Parmi les composés de formule (I) objets de l'invention, on peut notamment citer les composés suivants :
- *trans-*diiodo(*N*-cyclohexylamine)(1,3-diméthylimidazol-2-ylidène) ptatine(II)
- *trans*-dibromo(*N-*cyclohexylamine)(1,3-diméthylimidazol-2-ylidène) platine(II)
- *trans-*dichloro(*N-*cyclohexylamine)(1,3-diméthylimidazol-2-ylidène) platine(II)
- *trans*-dinitrato(*N*-cyclohexylamine)(1,3-diméthylimidazol-2-ylidène) platine(II)
- *trans*-diiodo(*N*-cyclohexylamine)(1,4-diméthyl-1,2,4-triazol-3-ylidène) platine(II)
- *trans*-dibromo(*N*-cyclohexylamine)(1,4-diméthyl-1,2,4-triazol-3-ylidène) platine(II)
- *trans*-dichloro(*N*-cyclohexylamine)(1,4-diméthyl-1,2,4-triazol-3-ylidène) platine(II)
- *trans*-dinitrato(*N*-cyclohexylamine)(1,4-diméthyl-1,2,4-triazol-3-ylidène) platine(II)
- *trans*-diiodo(*N*-cyclohexylamine)(1-méthyl-3-(cyclohexylméthyl) imidazol-2-ylidène) platine(II)
- *trans-*dibromo(*N*-cyclohexylamine)(1-méthyl-3-(cyclohexylméthyl) imidazol-2-ylidène) platine(II)
- *trans-*dichloro(*N-*cyclohexylamine)(1-méthyl-3-(cyclohexylméthyl) imidazol-2-ylidène) platine(II)
- *trans*-dinitrato(*N*-cyclohexylamine)(1-méthyl-3-(cyclohexylméthyl) imidazol-2-ylidène) platine(II)
- *trans*-diiodo(*N*-cyclohexylamine)(1-méthyl-3-phénylimidazol-2-ylidène) platine(II)
- trans-dibromo(N-cyclohexylamine)(1-méthyl-3-phénylimidazol-2-ylidène) platine(II)
- *trans*-dichloro(*N*-cyclohexylamine)(1-méthyl-3-phénylimidazol-2-ylidène) platine(II)
- *trans*-dinitrato(*N*-cyclohexylamine)(1-méthyl-3-phénylimidazol-2-ylidène) platine(II)
- *trans*-diiodo(*N*-tétrahydropyranyl-4-amine) (1,3-diméthylimidazol-2-ylidène) platine(II)
- *trans*-dibromo(*N-*tétrahydropyranyl-4-amine) (1,3-diméthylimidazol-2-ylidène) platine(II)
- *trans*-dichloro(*N*-tétrahydropyranyl-4-amine) (1,3-diméthylimidazol-2-ylidène) platine(II)
- *trans*-dinitrato(*N-*tétrahydropyranyl-4-amine) (1,3-diméthylimidazol-2-ylidène) platine(II)
- *trans*-diido(*N*-tétrahydropyranyl-4-amine) (1,4-diméthyl-1,2,4-triazol-3-ylidène) platine(II)
- *trans*-dibromo(*N-*tétrahydropyranyl-4-amine) (1,4-diméthyl-1,2,4-triazol-3-ylidène) platine(II)
- trans-dichloro(N-tétrahydropyranyl-4-amine) (1,4-diméthyl-1,2,4-triazol-3-ylidène) platine(II)
- *trans*-dinitrato(*N-*tétrahydropyranyl-4-amine) (1,4-diméthyl-1,2,4-triazol-3-ylidène) platine(II)
- *trans*-diiodo(*N*-tétrahydropyrany)-4-amine) (1-méthyl-3-(cyclohexylméthyl) imidazol-2-ylidène) platine(II)
- trans-dibromo(N-tétrahydropyranyl-4-amine) (1-méthyl-3-(cyclohexylméthyl) imidazol-2-ylidène) platine(II)
- *trans-*dichloro(*N-*tétrahydropyranyl-4-amine) (1-méthyl-3-(cyclohexylméthyl) imidazol-2-ylidène) platine(II)
- trans-dinitrato(N-tétrahydropyranyl-4-amine) (1-méthyl-3-(cyclohexylméthyl) imidazol-2-ylidène) platine(II)
- *trans*-diiodo(*N*-tétrahydropyranyl-4-amine) (1-méthyl-3-phénylimidazol-2-ylidène) platine(II)
- trans-dibromo(N-tétrahydropyranyl-4-amine) (1-méthyl-3-phénylimidazol-2-ylidène) platine(II)
- *trans-*dichloro(*N-*tétrahydropyranyl-4-amine) (1-méthyl-3-phénylimidazol-2-ylidène) platine(II)
- *trans*-dinitrato(*N*-tétrahydropyranyl-4-amine) (1-méthyl-3-phénylimidazol-2-ylidène) platine(II)
- *trans*-diido(*N-*morpholine) (1,3-diméthylimidazol-2-ylidène) platine(II)
- *trans*-dibromo(*N-*morpholine) (1,3-diméthylimidazol-2-ylidène) platine(II)
- *trans*-dichloro(*N*-morpholine) (1,3-diméthylimidazol-2-ylidène) platine(II)
- *trans*-dinitrato(*N*-morpholine) (1,3-diméthylimidazol-2-ylidène) platine(II)
- *trans*-diiodo(*N-*morpholine) (1,4-diméthyl-1,2,4-triazol-3-ylidène) platine(II)
- *trans-*dibromo(*N*-morpholine) (1,4-diméthyt-1,2,4-triazol-3-ylidène) platine(II)
- *trans*-dichloro(*N*-4-amino-tétrahydropyrane) (1,4-diméthyl-1,2,4-triazol-3-ylidène) platine(II)
- *trans*-dinitrato(*N*-morpholine) (1,4-diméthyl-1,2,4-triazol-3-ylidène) platine(II)
- *trans*-diiodo(*N*-morpholine) (1-méthyl-3-(cyclohexylméthyl) imidazol-2-ylidène) platine(II)
- *trans*-dibromo(*N*-morpholine) (1-méthyl-3-(cyclohexylméthyl) imidazol-2-ylidène) platine(II)
- trans-dichloro(*N*-morpholine) (1-méthyl-3-(cyclohexylméthyl) imidazol-2-ylidène) platine(II)
- *trans*-dinitrato(*N*-morpholine) (1-méthyl-3-(cyclohexylméthyl) imidazol-2-ylidène) platine(II)
- *trans-*diiodo(*N-*morpholine) (1-méthyl-3-phénylimidazol-2-ylidène) platine(II)
- *trans*-dibromo(*N*-morpholine) (1-méthyl-3-phénylimidazol-2-ylidène) platine(II)
- trans-dichloro(*N*-morpholine) (1-méthyl-3-phénylimidazol-2-ylidène) platine(II)
- *trans*-dinitrato(*N-*morpholine) (1-méthyl-3-phénylimidazol-2-ylidène) platine(II)
- *trans*-diiodo[*N*-(1-méthyl-pipéridin-4-ylamine)] (1,3-diméthylimidazol-2-ylidène) platine(II)
- *trans*-dibromo[*N*-(1-méthyl-pipéridin-4-ylamine)] (1,3-diméthylimidazol-2-ylidène) platine(II)
- *trans*-dichloro[*N-*(1-méthyl-pipéridin-4-ylamine)] (1,3-diméthylimidazol-2-ylidène) platine(II)
- *trans*-dinitrato[*N*-(1-méthyl-pipéridin-4-ylamine)]] (1,3-diméthylimidazol-2-ylidène) platine(II)
- *trans*-diiodo[*N*-(1-méthyl-pipéridin-4-ylamine)] (1,4-diméthyl-1,2,4-triazol-3-ylidène) platine(II)
- *trans*-dibromo[*N-*(1-méthyl-pipéridin-4-ylamine)] (1,4-diméthyl-1,2,4-triazol-3-ylidène) platine(II)
- *trans*-dichloro[*N*-(1-méthyl-pipéridin-4-ylamine)] (1,4-diméthyl-1,2,4-triazol-3-ylidène) platine(II)
- *trans*-dinitrato[*N-*(1-méthyl-pipéridin-4-ylamine)] (1,4-diméthyl-1,2,4-triazol-3-ylidène) platine(II)
- *trans*-diiodo[*N*-(1-méthyl-pipéridin-4-ylamine)] (1-méthyl-3-(cyclohexylméthyl) imidazol-2-ylidène) platine(II)
- *trans*-dibromo[*N*-(1-méthyl-pipéridin-4-ylamine)] (1-méthyl-3-(cyclohexylméthyl) imidazol-2-ylidène) platine(II)
- *trans*-dichloro[*N*-(1-méthyl-pipéridin-4-ylamine)] (1-méthyl-3-(cyclohexylméthyl) imidazol-2-ylidène) platine(II)
- *trans*-dinitrato[*N*-(1-méthyl-pipéridin-4-ylamine)] (1-méthyl-3-(cyclohexylméthyl) imidazol-2-ylidène) platine(II)
- *trans*-diiodo[*N*-(1-méthyl-pipéridin-4-ylamine)] (1-méthyl-3-phénylimidazol-2-ylidène) platine(II)
- *trans*-dibromo[*N*-(1-méthyl-pipéridin-4-ylamine)] (1-méthyl-3-phénylimidazol-2-ylidène) platine(II)
- *trans*-dichloro[*N-*(1-méthyl-pipéridin-4-ylamine)] (1-méthyl-3-phénylimidazol-2-ylidène) platine(II)
- *trans*-dinitrato[*N*-(1-méthyl-pipéridin-4-ylamine)] (1-méthyl-3-phénylimidazol-2-ylidène) platine(II)
- *trans*-diiodo(*N-exo*(+/-)bicyclo[2.2.1]heptylamine)(1,3-diméthyl-imidazol-2-ylidène)platine (II)
- *trans*-dibromo(*N-exo*(+/-)bicyclo[2.2.1]heptylamine)(1,3-diméthyl-imidazol-2-ylidène)platine (II)
- *trans*-dichloro(*N-exo*(+/-)bicyclo[2.2.1]heptylamine)(1,3-diméthyl-imidazol-2-ylidène)platine (II)
- *trans*-dinitrato(*N-exo*(+/-)bicyclo[2.2.1]heptylamine)(1,3-diméthyl-imidazol-2-ylidène)platine (II)
- *trans*-diiodo(*N*-cyclohexylamine)(1,3-diméthyl-benzimidazol-2-ylidène) platine (II)
- *trans*-dibromo(*N-*cyclohexylamine)(1,3-diméthyl-benzimidazol-2-ylidène) platine (II)
- trans-dichloro(N-cyclohexylamine)(1,3-diméthyl-benzimidazol-2-ylidène) platine (II)
- *trans*-dinitratochloro(*N-*cyclohexylamine)(1,3-diméthyl-benzimidazol-2-ylidène) platine (II)
- *trans*-diiodo(*N*-cyclohexylamine)(1,3,7,9-tétraméthylxanthine-8-ylidène) platine(II)
- *trans-*dibromo(*N*-cyclohexylamine)(1,3,7,9-tétraméthylxanthine-8-ylidène) platine(II)
- *trans*-dichloro(*N*-cyclohexylamine)(1,3,7,9-tétraméthylxanthine-8-ylidène) platine(II)
- *trans*-dinitrato(*N-*cyclohexylamine)(1,3,7,9-tétraméthylxanthine-8-ylidène) platine(II)
- *trans*-diiodo(*N*-cyclohexylamine)[1-méthyl-3-(4-méthoxybenzyl) imidazol-2-ylidène]platine (II)
- *trans*-dibromo(*N*-cyclohexylamine)[1-méthyl-3-(4-méthoxybenzyl) imidazol-2-ylidène]platine (II)
- *trans*-dichloro(*N*-cyclohexylamine)[1-méthyl-3-(4-méthoxybenzyl) imidazol-2-ylidène]platine (II)
- *trans*-dinitrato(*N*-cyclohexylamine)[1-méthyl-3-(4-méthoxybenzyl) imidazol-2-ylidène]platine (II)
- *trans*-diiodo(*N*-cydohexylamine)[1-méthyl-3-(4-trifluorométhylbenzyl) imidazol-2-ylidène]platine (II)
- *trans*-dibromo(*N-*cyclohexylamine)[1-méthyl-3-(4-trifluorométhylbenzyl) imidazol-2-ylidène]platine (II)
- *trans*-dichloro(*N*-cyclohexylamine)[1-méthyl-3-(4-trifluorométhylbenzyl) imidazol-2-ylidène]platine (II)
- *trans*-dinitrato(*N*-cyclohexylamine)[1-méthyl-3-(4-trifluorométhylbenzyl) imidazol-2-ylidène]platine (II)
- *trans-*diiodo(*N-*cyclohexylamine)[1,3-dibenzyl-imidazol-2-ylidène]platine (II)
- *trans*-dibromo(*N*-cyclohexylamine)[1,3-dibenzyl-imidazol-2-ylidène]platine (II)
- *trans*-dichloro(*N*-cyclohexylamine)[1,3-dibenzyl-imidazol-2-ylidène]platine (II)
- *trans*-dinitrato(*N*-cyclohexylamine)[1,3-dibenzyl-imidazol-2-ylidène]platine (II)
- *trans*-diiodo(*N*-benzylamine)(1,3-diméthyl-imidazol-2-ylidène) platine (II)
- *trans*-dibromo(*N-*benzylamine)(1,3-diméthyl-imidazol-2-ylidène) platine (II)
- *trans*-dichloro(*N*-benzylamine)(1,3-diméthyl-imidazol-2-ylidène) platine (II)
- *trans*-dinitrato(*N-*benzylamine)(1,3-diméthyl-imidazol-2-ylidène) platine (II)
- *trans*-diiodo[*N*-4-(trifluorométhyl)benzylamine](1,3-diméthyl-imidazol-2-ylidène)platine (II)
- *trans*-dibromo[*N*-4-(trifluorométhyl)benzylamine](1,3-diméthyl-imidazol-2-ylidène)platine (II)
- *trans*-dichloro[*N*-4-(trifluorométhyl)benzylamine](1,3-diméthyl-imidazol-2-ylidène)platine (II)
- *trans*-dinitrato[*N*-4-(trifluorométhyl)benzylamine](1,3-diméthyl-imidazol-2-ylidène)platine (II)
- *trans-*diiodo(*N*-cyclohexylamine)[1-méthyl-3-vinyl-imidazol-2-ylidène]platine (II)
- *trans-*dibromo(*N-*cyclohexylamine)[1-méthyl-3-vinyl-imidazol-2-ylidène]platine (II)
- *trans*-dichloro(*N-*cyclohexylamine)[1-méthyl-3-vinyl-imidazol-2-ylidène]platine (II)
- *trans*-dinitrato(*N*-cyclohexylamine)[1-méthyl-3-vinyl-imidazol-2-ylidène]platine (II)
- *trans*-diiodo(*N-exo*(+)bicyclo[2.2.1]heptylamine)(1,3-diméthyl-benzimidazol-2-ylidène)platine (II)
- *trans*-dibromo(*N-exo*(+)bicyclo[2.2.1]heptylamine)(1,3-diméthyl-benzimidazol-2-ylidène)platine (II)
- *trans*-dichloro(*N*-*exo*(+)bicyclo[2.2.1]heptylamine)(1,3-diméthyl-benzimidazol-2-ylidène)platine (II)
- *trans*-dinitrato(*N*-*exo*(+)bicyclo[2.2.1]heptylamine)(1,3-diméthyl-benzimidazol-2-ylidène)platine (II)
- *trans*-diiodo(*N-exo*(-)bicyclo[2.2.1]heptylamine)(1,3-diméthyl-benzimidazol-2-ylidène)platine (II)
- *trans*-dibromo(*N-exo*(-)bicydo[2.2.1]heptylamine)(1,3-diméthyl-benzimidazol-2-ylidène)platine (II)
- *trans*-dichloro(*N-exo*(-)bicyclo[2.2. 1]heptylamine)(1,3-diméthyl-benzimidazol-2-ylidène)platine (II)
- *trans*-dinitrato(*N-exo*(-)bicyclo[2.2.1]heptylamine)(1,3-diméthyl-benzimidazol-2-ylidène)platine (II)
- *trans*-diiodo(*N-*cyclohexylamine)(1,3-dicyclohexylimidazol-2-ylidène) platine(II)
- *trans*-dibromo(*N-*cyclohexylamine)(1,3-dicyclohexylimidazol-2-ylidène) platine(II)
- *trans*-dichloro(*N*-cyclohexylamine)(1,3-dicyclohexylimidazol-2-ylidène) platine(II)
- *trans-*dinitrato(*N*-cyclohexylamine)(1,3-dicyclohexylimidazol-2-ylidène) platine(II)
- *trans-*diiodo(*N*-cyclohexylamine)(1-méthyl-3-phényl-1,2,4-triazol-3-ylidène) platine(II)
- *trans*-dibromo(*N-*cyclohexylamine)(1-méthyl-3-phényl-1,2,4-triazol-3-ylidène) platine(II)
- *trans*-dichloro(*N-*cyclohexylamine)(1-méthyl-3-phényl-1,2,4-triazol-3-ylidène) platine(II)
- *trans*-dinitrato(*N*-cyclohexylamine)(1-méthyl-3-phényl-1,2,4-triazol-3-ylidène) platine(II)
- *trans*-diiodo(*N*-cyclohexylamine)[1,3-diméthyl-4-phényl-imidazol-2-ylidène]platine (II)
- *trans*-dibromo(*N*-cyclohexylamine)[1,3-diméthyl-4-phényl-imidazol-2-ylidène]platine (II)
- *trans*-dichoro(*N*-cyclohexylamine)[1,3-diméthyl-4-phényl-imidazol-2-ylidène]platine (II)
- *trans*-dinitato(*N-*cyclohexylamine)[1,3-diméthyl-4-phényl-imidazol-2-ylidène]platine (II)
- *trans*-diiodo(*N*-cyclohexylamine)[3-benzyl-1-méthyl-4-phényl-imidazol-2-ylidène]platine (II)
- *trans*-dibromo(*N-*cyclohexylamine)[3-benzyl-1-méthyl-4-phényl-imidazol-2-ylidène]platine (II)
- *trans*-dichloro(*N*-cyclohexylamine)[3-benzyl-1-méthyl-4-phényl-imidazol-2-ylidène]platine (II)
- *trans*-dinitrato(*N-*cyclohexylamine)[3-benzyl-1-méthyl-4-phényl-imidazol-2-ylidène]platine (II)
- *trans*-diiodo(*N*-cyclohexylamine)[1,3-diméthyl-4,5-diphényl-imidazol-2-ylidène]platine (II)
- *trans*-dibromo(*N*-cyclohexylamine)[1,3-diméthyl-4,5-diphényl-imidazol-2-ylidène]platine (II)
- *trans*-dichloro(*N-*cyclohexylamine)[1,3-diméthyl-4,5-diphényl-imidazol-2-ylidène]platine (II)
- *trans*-dinitato(*N-*cyclohexylamine)[1,3-diméthyl-4,5-diphényl-imidazol-2-ylidène]platine (II)
- *trans*-diiodo(*N*-*exo*(-)bicyclo[2.2.1]heptyl-amine)(1,3-diméthyl-imidazol-2-ylidène)platine (II)
- *trans*-dibromo(*N-exo*(-)bicyclo[2.2.1]heptyl-amine)(1,3-diméthyl-imidazol-2-ylidène)platine (II)
- *trans*-dichloro(*N-exo*(-)bicyclo[2.2.1]heptyl-amine)(1,3-diméthyl-imidazol-2-ylidène)platine (II)
- *trans*-dinitrato(*N-exo*(-)bicyclo[2.2.1]heptyl-amine)(1,3-diméthyl-imidazol-2-ylidène)platine (II)
- *trans*-diiodo(*N*-*exo*(+)bicyclo[2.2.1]heptylamine)(1,3-diméthyl-imidazol-2-ylidène)platine (II)
- *trans*-dibromo(*N-exo*(+)bicyclo[2.2.1]heptylamine)(1,3-diméthyl-imidazol-2-ylidène)platine (II)
- *trans*-dichloro(*N*-*exo*(+)bicyclo[2.2.1]heptylamine)(1,3-diméthyl-imidazol-2-ylidène)platine (II)
- *trans*-dinitrato(*N-exo*(+)bicyclo[2.2.1]heptylamine)(1,3-diméthyl-imidazol-2-ylidène)platine (II)
- *trans*-diiodo(*N-exo*(+/-)bicyclo[2.2.1]heptylamine)(1,3,7,9-tétraméthylxanthine-8-ylidène platine(II)
- *trans*-dibromo(*N-exo*(+/-)bicyclo[2.2.1]heptylamine)(1,3,7,9-tétraméthylxanthine-8-ylidène platine(II)
- *trans*-dichloro(*N*-*exo*(+/-)bicyclo[2.2.1]heptylamine)(1,3,7,9-tétraméthylxanthine-8-ylidène platine(II)
- *trans*-dinitrato(*N-exo*(+/-)bicyclo(2.2.1]heptylamine)(1,3,7,9-tétraméthylxanthine-8-ylidène platine(II)
- *trans*-diiodo(*N*-cyclopropylamine)(1,3-diméthylimidazol-2-ylidène) platine(II)
- *trans*-diiodo(*N*-cyclobutylamine)(1,3-diméthylimidazol-2-ylidène) platine(II)
- *trans*-diiodo(*N*-cyclopentylamine)(1,3-diméthylimidazol-2-ylidène) platine(II)
- *trans*-diiodo(*N*-cycloheptylamine)(1,3-diméthylimidazol-2-ylidène) platine(II)
- *trans*-diiodo(*N*-bicyclo[2.2.2]octylamine)(1,3-diméthylimidazol-2-ylidène) platine(II)
- *trans-*diiodo(*N-*endo(+/-)bicyclo[2.2.1]heptylamine)(1,3-diméthylimidazol-2-ylidène) platine(II)
- *trans*-diiodo(*N-exo*(+/-)bicyclo[3.2.1]octylamine)(1,3-diméthylimidazol-2-ylidène) platine(II)
- *trans*-diiodo(*N-*benzylamine)(1,3-diméthylimidazot-2-ylidène) platine(II)
- *trans*-diiodo(*N*-4-méthoxybenzylamine)(1,3-diméthylimidazol-2-ylidène) platine(II)
- *trans*-diiodo(*N*-4-trifluorométhylebenzylamine)(1,3-diméthylimidazol-2-ylidène) platine(II)
- *trans*-diiodo(*N*-4-chlorobenzylamine)(1,3-diméthylimidazol-2-ylidène) platine(II)
- *trans*-diiodo(*N*-4-fluorobenzylamine)(1,3-diméthylimidazol-2-ylidène) platine(II)
- *trans*-diiodo(*N*-4-bromobenzylamine)(1,3-diméthylimidazol-2-ylidène) platine(II)
- *trans*-diiodo(*N*-4-méthylbenzylamine)(1,3-diméthylimidazol-2-ylidène) platine(II)
- *trans*-diiodo(*N-*3,4-dichlorobenzylamine)(1,3-diméthylimidazol-2-ylidène) platine(II)
- *trans*-diiodo(*N*-3,4-diméthoxybenzylamine)(1,3-diméthylimidazol-2-ylidène) platine(II)
- *trans*-diiodo(*N*-3,4-diméthylbenzylamine)(1,3-diméthylimidazol-2-ylidène) platine(II)
- *trans*-diiodo(*N-*cyclopropylamine)(1,4-diméthyl-1,2,4-triazol-3-ylidène) platine(II)
- *trans*-diiodo(*N*-cyclobutylamine)(1,4-diméthyl-1,2,4-triazol-3-ylidène) platine(II)
- *trans*-diiodo(*N*-cyclopentylamine)(1,4-diméthyt-1,2,4-triazol-3-ylidène) platine(II)
- *trans-*diiodo(*N*-cycloheptylamine)(1,4-diméthyl-1,2,4-triazol-3-ylidène) platine(II)
- *trans*-diiodo(*N-*bicyclo[2.2.2]octylamine)(1,4-diméthyl-1,2,4-triazol-3-ylidène) platine(II)
- *trans*-diiodo(*N*-endo(+/-)bicyclo[2.2.1]heptylamine)(1,4-diméthyl-1,2,4-triazol-3-ylidène) platine(II)
- *trans*-diiodo(*N*-exo(+/-)bicyclo[3.2.1]octylamine)(1,4-diméthyl-1,2,4-triazol-3-ylidène) platine(II)
- *trans*-diiodo(*N-*benzylamine)(1,4-diméthyl-1,2,4-triazol-3-ylidène) platine(II)
- *trans*-diiodo(*N*-4-méthoxybenzylamine)(1,4-diméthyl-1,2,4-triazol-3-ylidène) platine(II)
- *trans*-diiodo(*N*-4-trifluorométhylebenzylamine)(1,4-diméthyl-1,2,4-triazol-3-ylidène) platine(II)
- *trans-*diiodo(*N*-4-chlorobenzylamine)(1,4-diméthyl-1,2,4-triazol-3-ylidène) platine(II)
- *trans*-diiodo(*N*-4-fluorobenzylamine)(1,4-diméthyl-1,2,4-triazol-3-ylidène) platine(II)
- *trans*-diiodo(*N*-4-bromobenzylamine)(1,4-diméthyl-1,2,4-triazol-3-ylidène) platine(II)
- *trans*-diiodo(*N*-4-méthylbenzylamine)(1,4-diméthyl-1,2,4-triazol-3-ylidène) platine(II)
- *trans*-diiodo(*N*-3,4-dichlorobenzylamine)(1,4-diméthyl-1,2,4-triazol-3-ylidène) platine(II)
- *trans*-diiodo(*N*-3,4-diméthoxybenzylamine)(1,4-diméthyl-1,2,4-triazol-3-ylidène) platine(II)
- *trans*-diiodo(*N*-3,4-diméthylbenzylamine)(1,4-diméthyl-1,2,4-triazol-3-ylidène) platine(II)
- *trans*-diiodo(*N*-cyclopropylamine)(1-méthyl-3-(cyclohexylméthyl) imidazol-2-ylidène) platine(II)
- *trans*-diiodo(*N*-cyclobutylamine)(1-méthyl-3-(cyclohexylméthyl) imidazol-2-ylidène) platine(II)
- *trans*-diiodo(*N*-cyclopentylamine)(1-méthyl-3-(cyclohexylméthyl) imidazol-2-ylidène) platine(II)
- *trans*-diiodo(*N*-cycloheptylamine)(1-méthyl-3-(cyclohexylméthyl) imidazol-2-ylidène) platine(II)
- *trans*-diiodo(*N-*bicyclo[2.2.2]octylamine)(1-méthyl-3-(cyclohexylméthyl) imidazol-2-ylidène) platine(II)
- *trans*-diiodo(*N*-endo(+/-)bicyclo[2.2.1]heptylamine)(1-méthyl-3-(cyclohexylméthyl) imidazol-2-ylidène) platine(II)
- *trans*-diiodo(*N*-exo(+/-)bicyclo[3.2.1]octylamine)(1-méthyl-3-(cyclohexylméthyl) imidazol-2-ylidène) platine(II)
- *trans*-diiodo(*N*-benzylamine)(1-méthyl-3-(cyclohexylméthyl) imidazol-2-ylidène) platine(II)
- *trans*-diiodo(*N*-4-méthoxybenzylamine)(1-méthyl-3-(cyclohexylméthyl) imidazol-2-ylidène) platine(II)
- *trans-*diiodo(*N*-4-trifluorométhylebenzylamine)(1-méthyl-3-(cyclohexylméthyl) imidazol-2-ylidène) platine(Il)
- *trans*-diiodo(*N*-4-chlorobenzylamine)(1-méthyl-3-(cyclohexylméthyl) imidazol-2-ylidène) platine(II)
- *trans*-diiodo(*N*-4-fluorobenzylamine)(1-méthyl-3-(cyclohexylméthyl) imidazol-2-ylidène) platine(II)
- *trans*-diiodo(*N*-4-bromobenzylamine)(1-méthyl-3-(cyclohexylméthyl) imidazol-2-ylidène) platine(II)
- *trans*-diiodo(*N*-4-méthylbenzylamine)(1-méthyl-3-(cyclohexylméthyl) imidazol-2-ylidène) platine(II)
- *trans*-diiodo(*N*-3,4-dichlorobenzylamine)(1-méthyl-3-(cyclohexylméthyl) imidazol-2-ylidène) platine(II)
- *trans*-diiodo(*N*-3,4-diméthoxybenzylamine)(1-méthyl-3-(cyclohexylméthyl) imidazol-2-ylidène) platine(II)
- *trans*-diiodo(*N-*3,4-diméthylbenzylamine)(1-méthyl-3-(cyclohexylméthyl) imidazol-2-ylidène) platine(II)
- *trans*-diiodo(*N*-cyclopropylamine)(1-méthyl-3-phénylimidazol-2-ylidène) platine(II)
- *trans*-diiodo(*N*-cyclobutylamine)(1-méthyl-3-phénylimidazol-2-ylidène) platine(II)
- *trans*-diiodo(*N*-cyclopentylamine)(1-méthyl-3-phénylimidazol-2-ylidène) platine(II)
- *trans*-diiodo(*N*-cycloheptylamine)(1-méthyl-3-phénylimidazol-2-ylidène) platine(II)
- *trans*-diiodo(*N*-bicyclo[2.2.2]octylamine)(1-méthyl-3-phénylimidazol-2-ylidène) platine(Il)
- *trans*-diiodo(*N*-endo(+/-)bicyclo[2.2.1]heptylamine)(1-méthyl-3-phénylimidazol-2-ylidène) platine(II)
- *trans*-diiodo(*N*-exo(+/-)bicyclop.2.1]octylamine)(1-méthyl-3-phénylimidazol-2-ylidène) platine(II)
- *trans*-diiodo(*N*-benzylamine)(1-méthyl-3-phénylimidazol-2-ylidène) platine(II)
- *trans*-diiodo(*N*-4-méthoxybenzylamine)(1-méthyl-3-phénylimidazol-2-ylidène) platine(II)
- *trans*-diiodo(*N*-4-trifluorométhylebenzylamine)(1-méthyl-3-phénylimidazol-2-ylidène) platine(II)
- *trans-*diiodo(*N*-4-chlorobenzylamine)(1-méthyl-3-phénylimidazol-2-ylidène) platine(II)
- *trans*-diiodo(*N*-4-fluorobenzylamine)(1-méthyl-3-phénylimidazol-2-ylidène) platine(II)
- *trans*-diiodo(*N*-4-bromobenzylamine)(1-méthyl-3-phénylimidazol-2-ylidène) platine(II)
- *trans*-diiodo(*N*-4-méthylbenzylamine)(1-méthyl-3-phénylimidazol-2-ylidène) platine(II) .
- *trans*-diiodo(*N*-3,4-dichlorobenzylamine)(1-méthyl-3-phénylimidazol-2-ylidène) platine(II)
- *trans*-diiodo(*N*-3,4-diméthoxybenzylamine)(1-méthyl-3-phénylimidazol-2-ylidène) platine(II)
- *trans-*diiodo(*N*-3,4-diméthylbenzylamine)(1-méthyl-3-phénylimidazol-2-ylidène) platine(II)
- *trans*-diiodo(*N*-cyclopropylamine)(1,3-diméthyl-benzimidazol-2-ylidène) platine(II)
- *trans*-diiodo(*N*-cyclobutylamine)(1,3-diméthyl-benzimidazol-2-ylidène) platine(II)
- *trans*-diiodo(*N*-cyclopentylamine)(1,3-diméthyl-benzimidazol-2-ylidène) platine(II)
- *trans-*diiodo(N-cyclo.heptylamine)( 1,3-diméthyl-benzimidazol-2-ylidène) platine(II)
- *trans*-diiodo(*N-*bicyclo[2.2.2]octylamine)(1,3-diméthyl-benzimidazol-2-ylidène) platine(II)
- *trans*-diiodo(*N*-endo(+/-)bicyclo[2.2.1]heptylamine)(1,3-diméthyl-benzimidazol-2-ylidène) platine(II)
- *trans-*diiodo(*N*-exo(+/-)bicyclo[3.2.1]octylamine)(1,3-diméthyl-benzimidazol-2-ylidène) platine(II)
- *trans-*diiodo(*N*-benzylamine)(1,3-diméthyl-benzimidazol-2-ylidène) platine(II)
- *trans*-diiodo(*N*-4-méthoxybenzylamine)(1,3-diméthyl-benzimidazol-2-ylidène) platine(II)
- *trans-*diiodo(*N*-4-trifluorométhylebenzylamine)(1,3-diméthyl-benzimidazol-2-ylidène) platine(II)
- *trans*-diiodo(*N*-4-chlorobenzylamine)(1,3-diméthyl-benzimidazol-2-ylidène) platine(II)
- *trans*-diiodo(*N*-4-fluorobenzylamine)(1,3-diméthyl-benzimidazol-2-ylidène) platine(II)
- *trans*-diiodo(*N*-4-bromobenzylamine)(1,3-diméthyl-benzimidazol-2-ylidène) platine(II)
- *trans*-diiodo(*N*-4-méthylbenzylamine)(1,3-diméthyl-benzimidazol-2-ylidène) platine(II)
- *trans*-diiodo(*N*-3,4-dichlorobenzylamine)(1,3-diméthyl-benzimidazol-2-ylidène) platine(II)
- *trans*-diiodo(*N*-3,4-diméthoxybenzylamine)(1,3-diméthyl-benzimidazol-2-ylidène) platine(II)
- *trans-*diiodo(*N*-3,4-diméthylbenzylamine)(1,3-diméthyl-benzimidazol-2-ylidène) platine(II)
- *trans*-diiodo(*N*-cyclopropylamine)(1,3,7,9-tétraméthylxanthine-8-ylidène) platine(II)
- *trans*-diiodo(*N-*cyclobutylamine)(1,3,7,9-tétraméthylxanthine-8-ylidène) platine(II)
- *trans*-diiodo(*N*-cyclopentylamine)(1,3,7,9-tétraméthylxanthine-8-ylidène) platine(II)
- *trans*-diiodo(*N*-cycloheptylamine)(1,3,7,9-tétraméthylxanthine-8-ylidène) platine(II)
- *trans-*diiodo(*N-*bicyclo[2.2.2]octylamine)(1,3,7,9-tétraméthylxanthine-8-ylidène) platine(II)
- *trans*-diiodo(*N*-endo(+/-)bicyclo[2.2.1]heptylamine)(1,3,7,9-tétraméthylxanthine-8-ylidène) platine(II)
- *trans*-diiodo(*N*-exo(+/-)bicyclo[3.2.1]octylamine)(1,3,7,9-tétraméthylxanthine-8-ylidène) platine(II)
- *trans*-diiodo(*N*-benzylamine)(1,3,7,9-tétraméthylxanthine-8-ylidène) platine(II)
- *trans*-diiodo(*N*-4-méthoxybenzylamine)(1,3,7,9-tétraméthylxanthine-8-ylidène) platine(II)
- *trans*-diiodo(*N*-4-trifluorométhylebenzylamine)(1,3,7,9-tétraméthylxanthine-8-ylidène) platine(II)
- *trans*-diiodo(*N*-4-chlorobenzylamine)(1,3,7,9-tétraméthylxanthine-8-ylidène) platine(II)
- *trans*-diiodo(*N*-4-fluorobenzylamine)(1,3,7,9-tétraméthylxanthine-8-ylidène) platine(II)
- *trans*-diiodo(*N*-4-bromobenzylamine)(1,3,7,9-tétraméthylxanthine-8-ylidène) platine(II)
- *trans*-diiodo(*N*-4-méthylbenzylamine)(1,3,7,9-tétraméthylxanthine-8-ylidène) platine(II)
- *trans*-diiodo(*N*-3,4-dichlorobenzylamine)(1,3,7,9-tétraméthylxanthine-8-ylidène) platine(II)
- *trans*-diiodo(*N*-3,4-diméthoxybenzylamine)(1,3,7,9-tétraméthylxanthine-8-ylidène) platine(II)
- *trans*-diiodo(*N*-3,4-diméthylbenzylamine)( 1,3,7,9-tétraméthylxanthine-8-ylidène) platine(II)
- *trans*-diiodo(*N*-cyclypropylamine)[1-méthyl-3-(4-méthoxybenzyl) imidazol-2-ylidène platine(II)
- *trans*-diiodo(*N*-cyclobutylamine)[1-méthyl-3-(4-méthoxybenzyl) imidazol-2-ylidène platine(II)
- *trans-*diiodo(*N*-cyclopentylamine)[1-méthyl-3-(4-méthoxybenzyl) imidazol-2-ylidène platine(II)
- *trans*-diiodo(*N*-cycloheptylamine)[1-méthyl-3-(4-méthoxybenzyl) imidazol-2-ylidène platine(II)
- *trans*-diiodo(*N*-bicyclo[2.2.2]octylamine)[1-méthyl-3-(4-méthoxybenzyl) imidazol-2-ylidène platine(II)
- *trans*-diiodo(*N*-endo(+/-)bicyclo[2.2.1]heptylamine)[1-méthyl-3-(4-méthoxybenzyl) imidazol-2-ylidène platine(II)
- *trans*-diiodo(*N*-exo(+/-)bicyclo[3.2.1]octylamine)[1-méthyl-3-(4-méthoxybenzyl) imidazol-2-ylidène platine(II)
- *trans*-diiodo(*N*-benzylamine)[1-méthyl-3-(4-méthoxybenzyl) imidazol-2-ylidène platine(II)
- *trans*-diiodo(*N*-4-méthoxybenzylamine)[1-méthyl-3-(4-méthoxybenzyl) imidazol-2-ylidène platine(II)
- *trans*-diiodo(*N*-4-trifluorométhylebenzylamine)[1-méthyl-3-(4-méthoxybenzyl) imidazol-2-ylidène platine(II)
- *trans-*diiodo(*N*-4-chlorobenzylamine)[1-méthyl-3-(4-méthoxybenzyl) imidazol-2-ylidène platine(II)
- *trans*-diiodo(*N*-4-fluorobenzylamine)[1-méthyl-3-(4-méthoxybenzyl) imidazole-2-ylidène platine(II)
- *trans*-diiodo(*N*-4-bromobenzylamine)[1-méthyl-3-(4-méthoxybenzyl) imidazol-2-ylidène platine(II)
- *trans*-diiodo(*N*-4-méthylbenzylamine)[1-méthyl-3-(4-méthoxybenzyl) imidazol-2-ylidène platine(II)
- *trans*-diiodo(*N*-3,4-dichlorobenzylamine)[1-méthyl-3-(4-méthoxybenzyl) imidazol-2-ylidène platine(II)
- *trans*-diiodo(*N*-3,4-diméthoxybenzylamine)[1-méthyl-3-(4-méthoxybenzyl) imidazol-2-ylidène platine(II)
- *trans*-diiodo(*N*-3,4-diméthylbenzylamine)[1-méthyl-3-(4-méthoxybenzyl) imidazol-2-ylidène platine(II)
- *trans*-diiodo(*N*-cyclopropylamine)[1-méthyl-3-(4-trifluorométhylbenzyl) imidazol-2-ylidène platine(II)
- *trans*-diiodo(*N*-cyclobutylamine)[1-méthyl-3-(4-trifluorométhylbenzyl) imidazol-2-ylidène platine(II)
- *trans*-diiodo(*N*-cyclopentylamine)[1-méthyl-3-(4-trifluorométhylbenzyl) imidazol-2-ylidène platine(II)
- *trans*-diiodo(*N*-cycloheptylamine)[1-méthyl-3-(4-trifluorométhylbenzyl I) imidazol-2-ylidène platine(II)
- *trans*-diiodo(*N*-bicyclo[2.2.2]octylamine)[1-méthyl-3-(4-trifluorométhylbenzyl) imidazol-2-ylidène platine(II)
- *trans*-diiodo(*N*-endo(+/-)bicyclo[2.2.1]heptylamine)[1-méthyl-3-(4-trifluorométhylbenzyl) imidazol-2-ylidène platine(II)
- *trans*-diiodo(*N*-exo(+/-)bicyclo[3.2.1]octylamine)[1-méthyl-3-(4-trifluorométhylbenzyl) imidazol-2-ylidène platine(II)
- *trans*-diiodo(*N*-benzylamine)[1-méthyl-3-(4-trifluorométhylbenzyl) imidazol-2-ylidène platine(II)
- *trans*-diiodo(*N*-4-méthoxybenzylamine)[1-méthyl-3-(4-trifluorométhylbenzyl I) imidazol-2-ylidène platine(II)
- *trans-*diiodo(*N*-4-trifluorométhylebenzylamine)[1-méthyl-3-(4-trifluorométhylbenzyl) imidazol-2-ylidène platine(II)
- *trans*-diiodo(*N*-4-chlorobenzylamine)[1-méthyl-3-(4-trifluorométhylbenzyl) imidazol-2-ylidène platine(II)
- *trans*-diiodo(*N*-4-fluorobenzylamine)[1-méthyl-3-(4-trifluorométhylbenzyl) imidazol-2-ylidène platine(II)
- *trans*-diiodo(*N*-4-bromobenzylamine)[1-méthyl-3-(4-trifluorométhylbenzyl) imidazol-2-ylidène platine(II)
- *trans*-diiodo(*N*-4-méthylbenzylamine)[1-méthyl-3-(4-trifluorométhylbenzyl) imidazol-2-ylidène platine(II)
- *trans*-diiodo(*N*-3,4-dichlorobenzylamine)[1-méthyl-3-(4-trifluorométhylbenzyl) imidazol-2-ylidène platine(II)
- *trans*-diiodo(*N*-3,4-diméthoxybenzylamine)[1-méthyl-3-(4-trifluorométhylbenzyl) imidazol-2-ylidène platine(II)
- *trans*-diiodo(*N*-3,4-diméthylbenzylamine)[1-méthyl-3-(4-trifluorométhylbenzyl) imidazol-2-ylidène platine(II)
- *trans*-diiodo(*N*-cyclopropylamine)[1-méthyl-3-(3-méthoxybenzyl) imidazol-2-ylidène platine(II)
- *trans*-diiodo(*N*-cyclobutylamine)[1-méthyl-3-(3-méthoxybenzyl) imidazol-2-ylidène platine(II)
- *trans*-diiodo(*N*-cyclopentylamine)[1-méthyl-3-(3-méthoxybenzyl) imidazol-2-ylidène platine(II)
- *trans*-diiodo(*N*-cycloheptylamine)[1-méthyl-3-(3-méthoxybenzyl) imidazol-2-ylidène platine(II)
- *trans*-diiodo(*N*-bicyclo[2.2.2]octylamine)[1-méthyl-3-(3-méthoxybenzyl) imidazol-2-ylidène platine(II)
- *trans*-diiodo(*N*-endo(+/-)bicyclo[2.2.1]heptylamine)[1-méthyl-3-(3-méthoxybenzyl) imidazol-2-ylidène platine(II)
- *trans*-diiodo(*N*-exo(+/-)bicyclo[3.2.1]octylamine)[1-méthyl-3-(3-méthoxybenzyl) imidazol-2-ylidène platine(II)
- *trans*-diiodo(*N*-benzylamine)[1-méthyl-3-(3-méthoxybenzyl) imidazol-2-ylidène platine(II)
- *trans*-diiodo(*N*-4-méthoxybenzylamine)[1-méthyl-3-(3-méthoxybenzyl) imidazol-2-ylidène platine(II)
- *trans*-diiodo(*N*-4-trifluorométhylebenzylamine)[1-méthyl-3-(3-méthoxybenzyl) imidazol-2-ylidène platine(II)
- *trans*-diiodo(*N*-4-chlorobenzylamine)[1-méthyl-3-(3-méthoxybenzyl) imidazol-2-ylidène platine(II)
- *trans*-diiodo(*N*-4-fluorobenzylamine)[1-méthyl-3-(3-méthoxybenzyl) imidazol-2-ylidène platine(II)
- *trans*-diiodo(*N*-4-bromobenzylamine)[1-méthyl-3-(3-méthoxybenzyl) imidazol-2-ylidène platine(II)
- *trans*-diiodo(*N*-4-méthylbenzylamine)[1-méthyl-3-(3-méthoxybenzyl) imidazol-2-ylidène platine(II)
- *trans*-diiodo(*N*-3,4-dichlorobenzylamine)[1-méthyl-3-(3-méthoxybenzyl) imidazol-2-ylidène platine(II)
- *trans*-diiodo(*N*-3,4-diméthoxybenzylamine)[1-méthyl-3-(3-méthoxybenzyl) imidazol-2-ylidène platine(II)
- *trans-*diiodo(*N*-3,4-diméthylbenzylamine)[1-méthyl-3-(3-méthoxybenzyl) imidazol-2-ylidène platine(II)
- *trans*-diiodo(*N*-cyclopropylamine)[1-méthyl-3-(3-trifluorométhylbenzyl) imidazol-2-ylidène platine(II)
- *trans*-diiodo(*N*-cyclobutytamine)[1-méthyl-3-(3-trifluorométhylbenzyl) imidazol-2-ylidène platine(II)
- *trans*-diiodo(*N*-cyclopentylamine)[1-méthyl-3-(3-trifluorométhylbenzyl) imidazol-2-ylidène platine(II)
- *trans*-diiodo(*N*-cycloheptylamine)[1-méthyl-3-(3-trifluorométhylbenzyl I) imidazol-2-ylidène platine(II)
- *trans*-diiodo(*N*-bicyclo[2.2.2]octylamine)[1-méthyl-3-(3-trifluorométhylbenzyl) imidazol-2-ylidène platine(II)
- *trans-*diiodo(*N*-endo(+/-)bicyclo[2.2.1]heptylamine)[1-méthyl-3-(3-trifluorométhylbenzyl) imidazol-2-ylidène platine(II)
- *trans*-diiodo(*N*-exo(+/-)bicyclo[3.2.1]octylamine)[1-méthyl-3-(3-trifluorométhylbenzyl) imidazol-2-ylidène platine(II)
- *trans*-diiodo(*N*-benzylamine)[1-méthyl-3-(3-trifluorométhylbenzyl) imidazol-2-ylidène platine(II)
- *trans*-diiodo(*N*-4-méthoxybenzylamine)[1-méthyl-3-(3-trifluorométhylbenzyl I) imidazol-2-ylidène platine(II)
- *trans*-diiodo(*N*-4-trifluorométhylebenzylamine)[1-méthyl-3-(3-trifluorométhylbenzyl) imidazol-2-ylidène platine(II)
- *trans*-diiodo(*N*-4-chlorobenzylamine)[1-méthyl-3-(3-trifluorométhylbenzyl) imidazol-2-ylidène platine(II)
- *trans*-diiodo(*N*-4-fluorobenzylamine)[1-méthyl-3-(3-trifluorométhylbenzyl) imidazol-2-ylidène platine(II)
- *trans*-diiodo(*N*-4-bromobenzylamine)[1-méthyl-3-(3-trifluorométhylbenzyl) imidazol-2-ylidène platine(II)
- *trans*-diiodo(*N*-4-méthylbenzylamine)[1-méthyl-3-(3-trifluorométhylbenzyl) imidazol-2-ylidène platine(II)
- *trans*-diiodo(*N*-3,4-dichlorobenzylamine)[1-méthyl-3-(3-trifluorométhylbenzyl) imidazol-2-ylidène platine(II)
- *trans*-diiodo(*N*-3,4-diméthoxybenzylamine)[1-méthyl-3-(3-trifluorométhylbenzyl) imidazol-2-ylidène platine(II)
- *trans*-diiodo(*N*-3,4-diméthylbenzylamine)[1-méthyl-3-(3-trifluorométhylbenzyl) imidazol-2-ylidène platine(II)
- *trans*-diiodo(*N*-cyclopropylamine)[1-méthyl-3-(3,4-diméthoxybenzyl) imidazol-2-ylidène platine(II)
- *trans*-diiodo(*N*-cyclobutylamine)[1-méthyl-3-((3,4-diméthoxybenzyl) imidazol-2-ylidène platine(II)
- *trans*-diiodo(*N*-cyclopentylamine)[1-méthyl-3-((3,4-diméthoxybenzyl) imidazol-2-ylidène platine(II)
- *trans-*diiodo(*N*-cycloheptylamine)[1-méthyl-3-((3,4-diméthoxybenzyl) imidazol-2-ylidène platine(II)
- *trans*-diiodo(*N*-bicyclo[2.2.2]octylamine)[1-méthyl-3-((3,4-diméthoxybenzyl) imidazol-2-ylidène platine(II)
- *trans*-diiodo(*N*-endo(+/-)bicyclo[2.2.1]heptylamine)[1-méthyl-3-((3,4-diméthoxybenzyl) imidazol-2-ylidène platine(II)
- *trans*-diiodo(*N*-exo(+/-)bicyclo[3.2.1]octylamine)[1-méthyl-3-((3,4-diméthoxybenzyl) imidazol-2-ylidène platine(II)
- *trans*-diiodo(*N*-benzylamine)[1-méthyl-3-((3,4-diméthoxybenzyl) imidazol-2-ylidène platine(II)
- *trans*-diiodo(*N*-4-méthoxybenzylamine)[1-méthyl-3-((3,4-diméthoxybenzyl) imidazol-2-ylidène platine(II)
- *trans*-diiodo(*N*-4-trifluorométhylebenzylamine)[1-méthyl-3-((3,4-diméthoxybenzyl) imidazol-2-ylidène platine(II)
- *trans*-diiodo(*N*-4-chlorobenzylamine)[1-méthyl-3-((3,4-diméthoxybenzyl) imidazol-2-ylidène platine(II)
- *trans*-diiodo(*N*-4-fluorobenzylamine)[1-méthyl-3-((3,4-diméthoxybenzyl) imidazol-2-ylidène platine(II)
- *trans*-diiodo(*N*-4-bromobenzylamine)[1-méthyl-3-((3,4-diméthoxybenzyl) imidazol-2-ylidène platine(II)
- *trans*-diiodo(*N*-4-méthylbenzylamine)[1-méthyl-3-((3,4-diméthoxybenzyl) imidazol-2-ylidène platine(II)
- *trans*-diiodo(*N*-3,4-dichlorobenzylamine)[1-méthyl-3-((3,4-diméthoxybenzyl) imidazol-2-ylidène platine(II)
- *trans*-diiodo(*N*-3,4-diméthoxybenzylamine)[1-méthyl-3-((3,4-diméthoxybenzyl) imidazol-2-ylidène platine(II)
- *trans*-diiodo(*N*-3,4-diméthylbenzylamine)[1-méthyl-3-((3,4-diméthoxybenzyl) imidazol-2-ylidène platine(II)
- *trans*-diiodo(*N*-cyclopropylamine)(1,3-dibenzyl-imidazol-2-ylidène) platine(II)
- *trans-*diiodo(*N*-cyclobutylamine)(1,3-dibenzyl-imidazol-2-ylidène) platine(II)
- *trans-*diiodo(*N*-cyclopentylamine)(1,3-dibenzyl-imidazol-2-ylidène) platine(II)
- *trans*-diiodo(*N*-cycloheptylamine)(1,3-dibenzyl-imidazol-2-ylidène) platine(II)
- *trans*-diiodo(*N*-bicyclo[2.2.2]octylamine)(1,3-dibenzyl-imidazol-2-ylidène) platine(II)
- *trans*-diiodo(*N*-endo(+/-)bicyclo[2.2.1]heptylamine)(1,3-dibenzyl-imidazol-2-ylidène) platine(II)
- *trans*-diiodo(*N*-exo(+/-)bicyclo[3.2.1]octylamine)(1,3-dibenzyl-imidazol-2-ylidène) platine(II)
- *trans*-diiodo(*N*-benzylamine)(1,3-dibenzyl-imidazol-2-ylidène) platine(II)
- *trans*-diiodo(*N*-4-méthoxybenzylamine)(1,3-dibenzyl-imidazol-2-ylidène) platine(II)
- *trans*-dilodo(*N*-4-trifluorométhylebenzylamine)(1,3-dibenzyl-imidazol-2-ylidène) platine(II)
- *trans*-diiodo(*N*-4-chlorobenzylamine)(1,3-dibenzyl-imidazol-2-ylidène) platine(II)
- *trans*-diiodo(*N*-4-fluorobenzylamine)(1,3-dibenzyl-imidazol-2-ylidène) platine(II)
- *trans*-diiodo(*N*-4-bromobenzylamine)(1,3-dibenzyl-imidazol-2-ylidène) platine(II)
- *trans*-diiodo(*N*-4-méthylbenzylamine)(1,3-dibenzyl-imidazol-2-ylidène) platine(II)
- *trans*-diiodo(*N*-3,4-dichlorobenzylamine)(1,3-dibenzyl-imidazol-2-ylidène) platine(II)
- *trans*-diiodo(*N*-3,4-diméthoxybenzylamine)(1,3-dibenzyl-imidazol-2-ylidène) platine(II)
- *trans*-diiodo(*N*-3,4-diméthylbenzylamine)(1,3-dibenzyl-imidazol-2-ylidène) platine(II)
- *trans*-diiodo(*N*-cyclopropylamine)(1-méthyl-3-vinyl-imidazol-2-ylidène) platine(II)
- *trans-*dilodo(*N*-cyclobutylamine)(1-méthyl-3-vinyl-imidazol-2-ylidène) platine(II)
- *trans*-diiodo(*N*-cyclopentylamine)(1-méthyl-3-vinyl-imidazol-2-ylidène) platine(II)
- *trans*-diiodo(*N*-cycloheptylamine)(1-méthyl-3-vinyl-imidazol-2-ylidène) platine(II)
- *trans*-diiodo(*N*-bicyclo[2.2.2]octylamine)(1-méthyl-3-vinyl-imidazol-2-ylidène) platine(II)
- *trans*-diiodo(*N*-endo(+/-)bicyclo[2.2.1]heptylamine)(1-méthyl-3-vinyl-imidazol-2-ylidène) platine(II)
- *trans*-diiodo(*N*-exo(+/-)bicyclo[3.2.1]octylamine)(1-méthyl-3-vinyl-imidazol-2-ylidène) platine(II)
- *trans*-diiodo(*N*-benzylamine)(1-méthyl-3-vinyl-imidazol-2-ylidène) platine(II)
- *trans*-diiodo(*N*-4-méthoxybenzylamine)(1-méthyl-3-vinyl-imidazol-2-ylidène) platine(II)
- *trans*-diiodo(*N*-4-trifluorométhylebenzylamine)(1-méthyl-3-vinyl-imidazol-2-ylidène) platine(II)
- *trans*-diiodo(*N*-4-chlorobenzylamine)(1-méthyl-3-vinyl-imidazol-2-ylidène) platine(II)
- *trans*-diiodo(*N*-4-fluorobenzylamine)(1-méthyl-3-vinyl-imidazol-2-ylidène) platine(II)
- *trans*-diiodo(*N*-4-bromobenzylamine)(1-méthyl-3-vinyl-imidazol-2-ylidène) platine(II)
- trans-diiodo(N-4-méthylbenzylamine)(1-méthyl-3-vinyl-imidazol-2-ylidène) platine(II)
- trans-diiodo(N-3,4-dichlorobenzylamine)(1-méthyl-3-vinyl-imidazol-2-ylidène) platine(II)
- trans-diiodo(N-3,4-diméthoxybenzylamine)(1-méthyl-3-vinyl-imidazol-2-ylidène) platine(II)
- *trans*-diiodo(*N*-3,4-diméthylbenzylamine)(1-méthyl-3-vinyl-imidazol-2-ylidène) platine(II)
- trans-diiodo(N-cyclopropylamine)(1-méthyl-3-éthyl-imidazol-2-ylidène) platine(II)
- trans-diiodo(N-cyclobutylamine)(1-méthyl-3- éthyl-imidazol-2-ylidène) platine(II)
- *trans*-diiodo(*N*-cyclopentylamine)(1-méthyl-3- éthyl-imidazol-2-ylidène) platine(II)
- *trans*-diiodo(*N*-cycloheptylamine)(1-méthyl-3- éthyl-imidazol-2-ylidène) platine(II)
- *trans*-diiodo(*N*-bicyclo[2.2.2]octylamine)(1-méthyl-3-éthyl-imidazol-2-ylidène) platine(II)
- *trans*-diiodo(*N*-endo(+/-)bicyclo[2.2.1]heptylamine)(1-méthyl-3- éthyl-imidazol-2-ylidène) platine(II)
- *trans*-diiodo(*N*-exo(+1-)bicyclo[3.2.1]octylamine)(1-méthyl-3- éthyl-imidazol-2-ylidène) platine(II)
- *trans*-diiodo(*N*-benzylamine)(1-méthyl-3- éthyl-imidazol-2-ylidène) platine(II)
- *trans*-diiodo(*N*-4-méthoxybenzylamine)(1-méthyl-3- éthyl-imidazol-2-ylidène) platine(II)
- *trans*-diiodo(*N*-4-trifluorométhylebenzylamine)(1-méthyl-3- éthyl-imidazol-2-ylidène) platine(II)
- *trans*-diiodo(*N*-4-chlorobenzylamine)(1-méthyl-3- éthyl-imidazol-2-ylidène) platine(II)
- *trans*-diiodo(*N*-4-fluorobenzylamine)(1-méthyl-3- éthyl-imidazol-2-ylidène) platine(II)
- *trans*-diiodo(*N*-4-bromobenzylamine)(1-méthyl-3- éthyl-imidazol-2-ylidène) platine(II)
- *trans*-diiodo(*N*-4-méthylbenzylamine)(1-méthyl-3- éthyl-imidazol-2-ylidène) platine(II)
- *trans*-diiodo(*N*-3,4-dichlorobenzylamine)(1-méthyl-3-éthyl-imidazol-2-ylidène) platine(II)
- *trans*-diiodo(*N*-3,4-diméthoxybenzylamine)(1-méthyl-3 éthyl-imidazol-2-ylidène) platine(II)
- *trans*-diiodo(*N*-3,4-diméthylbenzylamine)(1-méthyl-3- éthyl-imidazol-2-ylidène) platine(II)
- *trans*-diiodo(*N*-cyclopropylamine)(1-méthyl-3-isopropyl-2-ylidène) platine(II)
- *trans*-diiodo(*N*-cyclobutylamine)(1-méthyl-3- isopropyl-imidazol-2-ylidène) platine(II)
- *trans*-diiodo(*N*-cyclopentylamine)(1-méthyl-3-isopropyl-imidazol-2-ylidène) platine(II)
- *trans-*diiodo(*N*-cycloheptylamine)(1-méthyl-3-isopropyl-imidazol-2-ylidène) platine(II)
- *trans*-diiodo(*N*-bicyclo[2.2.2]octylamine)(1-méthyl-3-isopropyl-imidazol-2-ylidène) platine(II)
- *trans*-diiodo(*N*-endo(+/-)bicyclo[2.2.1]heptylamine)(1-méthyl-3-isopropy -imidazol-2-ylidène) platine(II)
- *trans*-diiodo(*N*-exo(+/-)bicyclo[3.2.1]octylamine)(1-méthyl-3-isopropyl-imidazol-2-ylidène) platine(II)
- *trans*-diiodo(*N*-benzylamine)(1-méthyl-3-isopropyl-imidazol-2-ylidène) platine(II)
- *trans*-diiodo(*N*-4-méthoxybenzylamine)(1-méthyl-3-isopropyl-imidazol-2-ylidène) platine(II)
- *trans*-diiodo(*N*-4-trifluorométhylebenzylamine)(1-méthyl-3-isopropyl-imidazol-2-ylidène) platine(II)
- *trans*-diiodo(*N*-4-chlorobenzylamine)(1-méthyl-3-isopropyl-imidazol-2-ylidène) platine(II)
- *trans*-diiodo(*N*-4-fluorobenzylamine)(1-méthyl-3-isopropyl-imidazol-2-ylidène) platine(II).
- *trans*-diiodo(*N*-4-bromobenzylamine)(1-méthyl-3-isopropyl-imidazol-2-ylidène) platine(II)
- *trans*-diiodo(*N*-4-méthylbenzylamine)(1-méthyl-3-isopropyl-imidazol-2-ylidène) platine(II)
- *trans-*diiodo(*N*-3,4-dichlorobenzylamine)(1-méthyl-3-isopropyl-imidazol-2-ylidène) platine(II)
- *trans*-diiodo(*N*-3,4-diméthoxybenzylamine)(1-méthyl-3-isopropyl-imidazol-2-ylidène) platine(II)
- *trans*-diiodo(*N*-3,4-diméthylbenzylamine)(1-méthyl-3-isopropyl-imidazol-2-ylidène) platine(II)
- *trans*-diiodo(*N*-cyclopropylamine)(1-méthyl-3-allyl-imidazol-2-ylidène) platine(II)
- *trans*-diiodo(*N*-cyclobutylamine)(1-méthyl-3-allyl-imidazol-2-ylidène) platine(II)
- *trans*-diiodo(*N*-cyclopentylamine)(1-méthyl-3-allyl-imidazol-2-ylidène) platine(II)
- *trans*-diiodo(*N*-cycloheptylamine)(1-méthyl-3-allyl-imidazol-2-ylidène) platine(II)

- *trans*-diiodo(*N*-bicyclo[2.2.2]octylamine)(1-méthyl-3-allyl-imidazol-2-ylidène) platine(II)
- *trans-*diiodo(*N*-endo(+/-)bicyclo[2.2.1]heptylamine)(1-méthyl-3-allyl-imidazol-2-ylidène) platine(II)
- *trans*-diiodo(*N*-exo(+/-)bicyclo[3.2.1]octylamine)(1-méthyl-3-allyl-imidazol-2-ylidène) platine(II)
- *trans*-diiodo(*N*-benzylamine)(1-méthyl-3-allyl-imidazol-2-ylidène) platine(II)
- *trans*-diiodo(*N*-4-méthoxybenzylamine)(1-méthyl-3-allyl-imidazol-2-ylidène) platine(II)
- *trans*-diiodo(*N*-4-trifluorométhylebenzylamine)(1-méthyl-3-allyl-imidazol-2-ylidène) platine(II)
- *trans*-diiodo(*N*-4-chlorobenzylamine)(1-méthyl-3-allyl-imidazol-2-ylidène) platine(II)
- *trans*-diiodo(*N*-4-fluorobenzylamine)(1-méthyl-3-allyl-imidazol-2-ylidène) platine(II)
- *trans*-diiodo(*N*-4-bromobenzylamine)(1-méthyl-3-vinyl-imidazol-2-ylidène) platine(II)
- *trans*-diiodo(*N*-4-méthylbenzylamine)(1-méthyl-3-allyl-imidazol-2-ylidène) platine(II)
- *trans*-diiodo(*N*-3,4-dichlorobenzylamine)(1-méthyl-3-allyl-imidazol-2-ylidène) platine(II)
- *trans*-diiodo(*N*-3,4-diméthoxybenzylamine)(1-méthyl-3-allyl-imidazol-2-ylidène) platine(II)
- *trans*-diiodo(*N*-3,4-diméthylbenzylamine)(1-méthyl-3-allyl-imidazol-2-ylidène) platine(II)
- *trans*-diiodo(*N*-cyclopropylamine)(1-méthyl-3-tertbutyl-imidazol-2-ylidène) platine(II)
- *trans*-diiodo(*N*-cyclobutylamine)(1-méthyl-3-*tert*butyl-imidazol-2-ylidène) platine(II)
- *trans*-diiodo(*N*-cyclopentylamine)(1-méthyl-3-tertbutyl-imidazol-2-ylidène) platine(II)
- *trans*-diiodo(*N*-cycloheptylamine)(1-méthyl-3-tertbutyl-imidazol-2-ylidène) platine(II)
- *trans*-diiodo(*N*-bicyclo[2.2.2]octylamine)(1-méthyl-3-tertbutyl-imidazol-2-ylidène) platine(II)
- *trans*-diiodo(*N*-endo(+/-)bicyclo[2.2.1]heptylamine)(1-méthyl-3-terbutyl-imidazol-2-ylidène) platine(II)
- *trans*-diiodo(*N*-exo(+/-)bicyclo[3.2.1]octylamine)(1-méthyl-3-terbutyl-imidazol-2-ylidène) platine(II)
- *trans*-diiodo(*N*-benzylamine)(1-méthyl-3-tertbutyl-imidazol-2-ylidène) platine(II)
- *trans*-diiodo(*N*-4-méthoxybenzylamine)(1-méthyl-3-tertbutyl-imidazol-2-ylidène) platine(II)
- *trans*-diiodo(*N*-4-trifluorométhylebenzylamine)(1-méthyl-3-tertbutyl-imidazol-2-ylidène) platine(II)
- *trans*-diiodo(*N*-4-chlorobenzylamine)(1-méthyl-3-tertbutyl-imidazol-2-ylidène) platine(II)
- *trans*-diiodo(*N*-4-fluorobenzylamine)(1-méthyl-3-tertbutyl-imidazol-2-ylidène) platine(II)
- *trans*-diiodo(*N*-4-bromobenzylamine)(1-méthyl-3-tertbutyl-imidazol-2-ylidène) platine(II)
- *trans*-diiodo(*N*-4-méthylbenzylamine)(1-méthyl-3-tertbutyl-imidazol-2-ylidène) platine(II)
- *trans*-diiodo(*N*-3,4-dichlorobenzylamine)(1-méthyl-3-*tert*butyl-imidazol-2-ylidène) platine(II)
- *trans*-diiodo(*N*-3,4-diméthoxybenzylamine)(1-méthyl-3-tertbutyl-imidazol-2-ylidène) platine(II)
- *trans*-diiodo(*N*-3,4-diméthylbenzylamine)(1-méthyl-3-tertbutyl-imidazol-2-ylidène) platine(II)
- *trans*-diiodo(*N*-cyclopropylamine)(1-méthyl-3-cyclohexyl-imidazol-2-ylidène) platine(II)
- *trans*-diiodo(*N*-cyclobutylamine)(1-méthyl-3-cyclohexyl-imidazol-2-ylidène) platine(II)
- *trans*-diiodo(*N*-cyclopentylamine)(1-méthyl-3-cyclohexyl-imidazol-2-ylidène) platine(II)
- *trans*-diiodo(*N*-cycloheptylamine)(1-méthyl-3-cyclohexyl-imidazol-2-ylidène) platine(II)
- *trans*-diiodo(*N*-bicyclo[2.2.2]octylamine)(1-méthyl-3-cyclohexyl-imidazol-2-ylidène) platine(II)
- *trans*-diiodo(*N*-endo(+/-)bicyclo[2.2.1]heptylamine)(1-méthyl-3-cyclohexyl-imidazol-2-ylidène) platine(II)
- *trans*-diiodo(*N*-exo(+/-)bicyclo[3.2.1]octylamine)(1-méthyl-3-cyclohexyl-imidazol-2-ylidène)platine(II)
- *trans*-diiodo(*N*-benzylamine)(1-méthyl-3-cyclohexyl-imidazol-2-ylidène) platine(II)
- *trans*-diiodo(*N*-4-méthoxybenzylamine)(1-méthyl-3-cyclohexyl-imidazol-2-ylidène) platine(II)
- *trans*-diiodo(*N*-4-trifluorométhylebenzylamine)(1-méthyl-3-cyclohexyl-imidazol-2-ylidène) platine(II)
- *trans*-diiodo(*N*-4-chlorobenzylamine)(1-méthyl-3-cyclohexyl-imidazol-2-ylidène) platine(II)
- *trans*-diiodo(*N*-4-fluorobenzylamine)(1-méthyl-3-cyclohexyl-imidazol-2-ylidène)platine(II)
- *trans*-diiodo(*N*-4-bromobenzylamine)(1-méthyl-3-cyclohexyl-imidazol-2-ylidène) platine(II)
- *trans*-diiodo(*N*-4-méthylbenzylamine)(1-méthyl-3-cyclohexyl-imidazol-2-ylidène) platine(II)
- *trans*-diiodo(*N*-3,4-dichlorobenzylamine)(1-méthyl-3-cyclohexyl-imidazol-2-ylidène) platine(II)
- *trans*-diiodo(*N*-3,4-diméthoxybenzylamine)(1-méthyl-3-cyclohexyl-imidazol-2-ylidène) platine(II)
- *trans*-diiodo(*N*-3,4-diméthylbenzylamine)(1-méthyl-3-cyclohexyl-imidazol-2-ylidène)platine(II)
- *trans*-diiodo(*N*-cyclopropylamine)(1-méthyl-3-cyclohexylméthyl-imidazol-2-ylidène) platine(II)
- *trans*-diiodo(*N*-cyclobutylamine)(1-méthyl-3-cyclohexylméthyl-imidazol-2-ylidène) platine(II)
- *trans*-diiodo(*N*-cyclopentylamine)(1-méthyl-3-cyclohexylméthyl-imidazol-2-ylidène) platine(II)
- *trans*-diiodo(*N*-cycloheptylamine)(1-méthyl-3-cyclohexylméthyl-imidazol-2-ylidène) platine(II)
- *trans*-diiodo(*N*-bicyclo[2.2.2]octylamine)(1-méthyl-3-cyclohexylméthyl-imidazol-2-ylidène) platine(II)
- *trans-*diiodo(*N*-endo(+/-)bicyclo[2.2.1]heptylamine)(1-méthyl-3-cyclohexylméthyl-imidazol-2-ylidène) platine(II)
- *trans*-diiodo(*N*-exo(+/-)bicyclo[3.2.1]octylamine)(1-méthyl-3-cyclohexylméthyl-imidazol-2-ylidène)platine(II)
- *trans*-diiodo(*N*-benzylamine)(1-méthyl-3-cyclohexyméthyll-imidazol-2-ylidène) platine(II)
- *trans*-diiodo(*N*-4-méthoxybenzylamine)(1-méthyl-3-cyclohexyméthyl-imidazol-2-ylidène) platine(II)
- *trans*-diiodo(*N*-4-trifluorométhylebenzylamine)(1-méthyl-3-cyclohexylméthyl-imidazol-2-ylidène) platine(II)
- *trans*-diiodo(*N*-4-chlorobenzylamine)(1-méthyl-3-cyclohexylméthyl-imidazol-2-ylidène) platine(II)
- *trans*-diiodo(*N*-4-fluorobenzylamine)(1-méthyl-3-cyclohexylméthyl-imidazol-2-ylidène)platine(II)
- *trans*-diiodo(*N*-4-bromobenzylamine)(1-méthyl-3-cyclohexylméthyl-imidazol-2-ylidène) platine(II)
- *trans*-diiodo(*N*-4-méthylbenzylamine)(1-méthyl-3-cyclohexylméthyl-imidazol-2-ylidène) platine(II)
- *trans*-diiodo(*N*-3,4-dichlorobenzylamine)(1-méthyl-3-cyclohexylméthyl-imidazol-2-ylidène) platine(II)
- *trans*-diiodo(*N*-3,4-diméthoxybenzylamine)(1-méthyl-3-cyclohexylméthyl-imidazol-2-ylidène) platine(II)
- *trans*-diiodo(*N*-3,4-diméthylbenzylamine)(1-méthyl-3-cyclohexylméthyl-imidazol-2-ylidène)platine(II)
- *trans*-diiodo(*N*-cyclopropylamine)(1-méthyl-3-cydopropyl-imidazol-2-ylidène) platine(II)
- *trans*-diiodo(*N*-cyclobutylamine)(1-méthyl-3-cyclopropyl-imidazol-2-ylidène) platine(II)
- *trans*-diiddo(*N*-cyclopentylamine)(1-méthyl-3-cyclopropyl imidazol-2-ylidène) platine(II)
- *trans*-diiodo(*N*-cycloheptylamine)(1-méthyl-3-cyclopropyl-imidazol-2-ylidène) platine(II)
- *trans*-diiodo(*N*-bicyclo[2.2.2]octylamine)(1-méthyl-3-cyclopropyl-imidazol-2-ylidène) platine(II)
- *trans*-diiodo(*N*-endo(+/-)bicyclo[2.2.1]heptylamine)(1-méthyl-3-cyclopropyl-imidazol-2-ylidène) platine(II)
- *trans*-diiodo(*N*-exo(+/-)bicyclo[3.2.1]octylamine)(1-méthyl-3-cyclopropyl-imidazol-2-ylidène)platine(II)
- *trans*-diiodo(*N*-benzylamine)(1-méthyl-3-cyclopropyl-imidazol-2-ylidène) platine(II)
- *trans*-diiodo(*N*-4-méthoxybenzylamine)(1-méthyl-3-cyclopropyl-imidazol-2-ylidène) platine(II)
- *trans-*diiodo(*N*-4-trifluorométhylebenzylamine)(1-méthyl-3-cyclopropyl-imidazol-2-ylidène) platine(II)
- *trans*-diiodo(*N*-4-chlorobenzylamine)(1-méthyl-3-cyclopropyl-imidazol-2-ylidène) platine(II)
- *trans*-diiodo(*N*-4-fluorobenzylamine)(1-méthyl-3-cyclopropyl-imidazol-2-ylidène)platine(II)
- *trans*-diiodo(*N*-4-bromobenzylamine)(1-méthyl-3-cyclopropyl-imidazol-2-ylidène) platine(II)
- *trans*-diiodo(*N*-4-méthylbenzylamine)(1-méthyl-3-cyclopropyl-imidazol-2-ylidène) platine(II)
- *trans*-diiodo(*N-*3,4-dichlorobenzylamine)(1-méthyl-3-cyclopropyl-imidazol-2-ylidène) platine(II)
- *trans*-diiodo(*N*-3,4-diméthoxybenzylamine)(1-méthyl-3-cyclopropyl-imidazol-2-ylidène) platine(II)
- *trans*-diiodo(*N*-3,4-diméthylbenzylamine)(1-méthyl-3-cyclopropyl-imidazol-2-ylidène)platine(II)
- *trans-*diiodo(*N*-cyclopropylamine)(1-méthyl-3-cyclopropylméthyl-imidazol-2-ylidène) platine(II)
- *trans*-diiodo(*N*-cyclobutylamine)(1-méthyl-3-cyclopropylméthyl-imidazol-2-ylidène) platine(II)
- *trans*-diiodo(*N*-cyclopentylamine)(1-méthyl-3-cyclopropylméthyl-imidazol-2-ylidène) platine(II)
- *trans*-diiodo(*N*-cycloheptylamine)(1-méthyl-3-cyclopropylméthyl-imidazol-2-ylidène) platine(II)
- *trans*-diiodo(*N*-bicyclo[2.2.2]octylamine)(1-méthyl-3-cyclopropylméthyl-imidazol-2-ylidène) platine(II)
- *trans*-diiodo(*N*-endo(+/-)bicyclo[2.2.1]heptylamine)(1-méthyl-3-cyclopropylméthyl-imidazol-2-ylidène) platine(II)
- *trans*-diiodo(*N*-exo(+/-)bicyclo[3.2.1]octylamine)(1-méthyl-3-cyclopropylméthyl-imidazol-2-ylidène)platine(II)
- *trans*-diiodo(*N*-benzylamine)(1-méthyl-3-cyclopropyméthyl-imidazol-2-ylidène) platine(II)
- *trans*-diiodo(*N*-4-méthoxybenzylamine)(1-méthyl-3-cyclopropyméthyl-imidazol-2-ylidène) platine(II)
- *trans*-diiodo(*N*-4-trifluorométhylebenzylamine)(1-méthyl-3-cyclopropylméthyl-imidazol-2-ylidène) platine(II)
- *trans*-diiodo(*N*-4-chlorobenzylamine)(1-méthyl-3-cyclopropylméthyl-imidazol-2-ylidène) platine(II)
- *trans*-diiodo(*N*-4-fluorobenzylamine)(1-méthyl-3-cyclopropylméthyl-imidazol-2-ylidène)platine(II)
- *trans*-diiodo(*N*-4-bromobenzylamine)(1-méthyl-3-cyclopropylméthyl-imidazol-2-ylidène) platine(II)
- *trans-*diiodo(*N*-4-méthylbenzylamine)(1-méthyl-3-cyclopropylméthyl-imidazol-2-ylidène) platine(II)
- *trans-*diiodo(*N*-3,4-dichlorobenzylamine)(1-méthyl-3-cyclopropylméthyl-imidazol-2-ylidène) platine(II)
- *trans*-diiodo(*N*-3,4-diméthoxybenzylamine)(1-méthyl-3-cyclopropylméthyl-imidazol-2-ylidène) platine(II)
- *trans*-diiodo(*N*-3,4-diméthylbenzylamine)(1-méthyl-3-cyclopropylméthyl-imidazol-2-ylidène)platine(II)
- *trans*-diiodo(*N*-cyclopropylamine)(1,3-dicyclohexylimidazol-2-ylidène) platine(II)
- *trans*-diiodo(*N*-cyclobutylamine)(1,3-dicyclohexylimidazol-2-ylidène) platine(II)
- *trans-*diiodo(*N*-cyclopentylamine)(1,3-dicyclohexylimidazol-2-ylidène) platine(II)
- *trans*-diiodo(*N*-cycloheptylamine)(1,3-dicyclohexylimidazol-2-ylidène) platine(II)
- *trans*-diiodo(*N*-bicyclo[2.2.2]octylamine)(1,3-dicyclohexylimidazol-2-ylidène) platine(II)
- *trans-*diiodo(*N*-endo(+/-)bicyclo[2.2.1]heptylamine)(1,3-dicyclohexylimidazol-2-ylidène) platine(II)
- *trans*-diiodo(*N*-exo(+/-)bicyclo[3.2.1]octylamine)(1,3-dicyclohexylimidazol-2-ylidène) platine(II)
- *trans*-diiodo(*N*-benzylamine)(1,3-dicyclohexylimidazol-2-ylidène) platine(II)
- *trans*-diiodo(*N*-4-méthoxybenzylamine)(1,3-dicyclohexylimidazol-2-ylidène) platine(II)
- *trans*-diiodo(*N*-4-trifluorométhylebenzylamine)(1,3-dicyclohexylimidazol-2-ylidène) platine(II)
- *trans*-diiodo(*N*-4-chlorobenzylamine)(1,3-dicyclohexylimidazol-2-ylidène) platine(II)
- *trans*-diiodo(*N*-4-fluorobenzylamine)(1,3-dicyclohexylimidazol-2-ylidène) platine(II)
- *trans-*diiodo(*N*-4-bromobenzylamine)(1,3-dicyclohexylimidazol-2-ylidène) platine(II)
- *trans*-diiodo(*N*-4-méthylbenzylamine)(1,3-dicyclohexylimidazol-2-ylidène) platine(II)
- *trans*-diiodo(*N*-3,4-dichlorobenzylamine)(1,3-dicyclohexylimidazol-2-ylidène) platine(II)
- *trans*-diiodo(*N*-3,4-diméthoxybenzylamine)(1,3-dicyclohexylimidazol-2-ylidène) platine(II)
- *trans*-diiodo(*N*-3,4-diméthylbenzylamine)(1,3-dicyclohexylimidazol-2-ylidène) platine(II)
- *trans*-diiodo(*N*-cyclopropylamine)(1,3-dicyclohexylméthyl-imidazol-2-ylidène) platine(II)
- *trans*-diiodo(*N*-cyclobutylamine)(1,3-dicyclohexyméthyl-limidazol-2-ylidène) platine(II)
- *trans*-diiodo(*N*-cyclopentylamine)(1,3-dicyclohexylméthyl-imidazol-2-ylidène) platine(II)
- *trans*-diiodo(*N*-cycloheptylamine)(1,3-dicyclohexylméthyl-imidazol-2-ylidène) platine(II)
- *trans*-diiodo(*N*-bicyclo[2.2.2]octylamine)(1,3-dicyclohexylméthyl-imidazol-2-ylidène) platine(II)
- *trans*-diiodo(*N*-endo(+/-)bicyclo[2.2.1]heptylamine)(1,3-dicyclohexylméthyl-imidazol-2-ylidène) platine(II)
- *trans*-diiodo(*N*-exo(+/-)bicyclo[3.2.1]octylamine)(1,3-dicyclohexylméthyl-imidazol-2-ylidène) platine(II)
- *trans*-diiodo(*N*-benzylamine)(1,3-dicyclohexylméthyl-imidazol-2-ylidène) platine(II)
- *trans*-diiodo(*N*-4-méthoxybenrylamine)(1,3-dicyclohexylimidazol-2-ylidène) platine(II)
- *trans*-diiodo(*N*-4-trifluorométhylebenzylamine)(1,3-dicyclohexylméthyl-imidazol-2-ylidène) platine(II)
- *trans*-diiodo(*N*-4-chlorobenzylamine)(1,3-dicyclohexylméthyl-imidazol-2-ylidène) platine(II)
- *trans*-diiodo(*N*-4-fluorobenryfamine)(1,3-dicyclohexylméthyl-imidazol-2-ylidène) platine(II)
- *trans*-diiodo(*N*-4-bromobenzylamine)(1,3-dicyclohexylméthyl-imidazol-2-ylidène) platine(II)
- *trans-*diiodo(*N*-4-méthylbenzylamine)(1,3-dicyclohexylméthyl-imidazol-2-ylidène) platine(II)
- *trans*-diiodo(*N*-3,4-dichlorobenzylamine)(1,3-dicyclohexylméthyl-imidazol-2-ylidène) platine(II)
- *trans-*diiodo(*N*-3,4-diméthoxybenzylamine)(1,3-dicyclohexylméthyl-imidazol-2-ylidène) platine(II)
- *trans*-diiodo(*N*-3,4-diméthylbenzylamine)(1,3-dicyclohexylméthyl-imidazol-2-ylidène) platine(II)
- *trans*-diiodo(*N*-cyclopropylamine)(1,3-dicyclopropylméthyl-imidazol-2-ylidène) platine(II)
- *trans-*diiodo(*N*-cyclobutylamine)(1,3-dicyclopropyméthyl-limidazol-2-ylidène) platine(II)
- *trans*-diiodo(*N*-cyclopentylamine)(1,3-dicyclopropylméthyl-imidazol-2-ylidène) platine(II)
- *trans*-diiodo(*N*-cycloheptylamine)(1,3-dicyclopropylméthyl-imidazol-2-ylidène) platine(II)
- *trans*-diiodo(*N*-bicyclo[2.2.2]octylamine)(1,3-dicyclopropylméthyl-imidazol-2-ylidène) platine(II)
- *trans*-diiodo(*N*-endo(+/-)bicyclo[2.2.1]heptylamine)(1,3-dicyclopropylméthyl-imidazol-2-ylidène) platine(II)
- *trans*-diiodo(*N*-exo(+/-)bicyclo[3.2.1]octylamine)(1,3-dicyclopropylméthyl-imidazol-2-ylidène) platine(II)
- *trans*-diiodo(*N*-benzylamine)(1,3-dicyclopropylméthyl-imidazol-2-ylidène) platine(II)
- *trans*-diiodo(*N*-4-méthoxybenzylamine)(1,3-dicyclopropylimidazol-2-ylidène) platine(II)
- *trans*-diiodo(*N*-4-trifluorométhylebenzylamine)(1,3-dicyclopropylméthyl-imidazol-2-ylidène) platine(II)
- *trans*-diiodo(*N*-4-chlorobenzylamine)(1,3-dicyclopropylméthyl-imidazol-2-ylidène) platine(II)
- *trans-*diiodo(*N*-4-fluorobenzylamine)(1,3-dicyclopropylméthyl-imidazol-2-ylidène) platine(II)
- *trans*-diiodo(*N*-4-bromobenzylamine)(1,3-dicyclopropylméthyl-imidazol-2-ylidène) platine(II)
- *trans*-diiodo(*N*-4-méthylbenzylamine)(1,3-dicyclohexylméthyl-imidazol-2-ylidène) platine(II)
- *trans*-diiodo(*N*-3,4-dichlorobenzylamine)(1,3-dicyclohexylméthyl-imidazol-2-ylidène) platine(II)
- *trans*-diiodo(*N*-3,4-diméthoxybenzylamine)(1,3-dicyclohexylméthyl-imidazol-2-ylidène) platine(II)
- *trans*-diiodo(*N*-3,4-diméthylbenzylamine)(1,3-dicyclohexylméthyl-imidazol-2-ylidène) platine(II)
- *trans*-diiodo(*N*-cyclopropylamine)(1-méthyl-3-phényl-1,2,4-triazol-5-ylidène) platine(II)
- *trans*-diiodo(*N*-cyclobutylamine)(1-méthyl-3-phényl-1,2,4-triazol-5-ylidène) platine(II)
- *trans-*diiodo(*N*-cyclopentylamine)(1-méthyl-3-phényl-1,2,4-triazol-5-ylidène) platine(II)
- *trans*-diiodo(*N*-cydoheptylamine)(1-méthyl-3-phényl-1,2,4-triazol-5-ylidène) platine(II)
- *trans*-diiodo(*N*-bicyclo[2.2.2]octylamine)(1-méthyl-3-phényl-1,2,4-triazol-5-ylidène) platine(II)
- *trans*-diiodo(*N*-endo(+/-)bicyclo[2.2.1]heptylamine)(1-méthyl-3-phényl-1,2,4-triazol-5-ylidène) platine(II)
- *trans*-diiodo(*N*-exo(+/-)bicyclo[3.2.1]octylamine)(1-méthyl-3-phényl-1,2,4-triazol-5-ylidène) platine(II)
- *trans*-diiodo(*N*-benzylamine)(1-méthyl-3-phényl-1,2,4-triazol-5-ylidène) platine(II)
- trans-diiodo(N-4-méthoxybenzylamine)(1-méthyl-3-phényl-1,2,4-triazol-5-ylidène) platine(II)
- *trans*-diiodo(*N*-4-trifluorométhylebenzylamine)(1-méthyl-3-phényl-1,2,4-triazol-5-ylidène) platine(II)
- *trans-*diiodo(*N*-4-chlorobenzylamine)(1 -méthyl-3-phényl-1,2,4-triazol-5-ylidène) platine(II)
- *trans*-diiodo(*N*-4-fluorobenzylamine)(1-méthyl-3-phényl-1,2,4-triazol-5-ylidène) platine(II)
- *trans*-diiodo(*N*-4-bromobenzylamine)(1 -méthyl-3-phényl-1,2,4-triazol-5-ylidène) platine(II)
- *trans*-diiodo(*N*-4-méthylbenzylamine)(1-méthyl-3-phényl-1,2,4-triazol-5-ylidène) platine(II)
- *trans*-diiodo(*N*-3,4-dichlorobenzylamine)(1 -méthyl-3-phényl-1,2,4-triazol-5-ylidène) platine(II)
- *trans*-diiodo(*N*-3,4-diméthoxybenzylamine)(1-méthyl-3-phényl-1,2,4-triazol-5-ylidène) platine(II) *trans*-diiodo(*N*-3,4-diméthylbenzylamine)(1-méthyl-3-phényl-1,2,4-triazol-5-ylidène) platine(II)
- *trans*-diiodo(*N*-cyclopropylamine)(1-méthyl-3-cyclohexyl-1,2,4-triazol-5-ylidène) platine(II)
- *trans*-diiodo(*N*-cyclobutylamine)(1-méthyl-3-cyclohexyl-1,2,4-triazol-5-ylidène) platine(II)
- *trans*-diiodo(*N*-cyclopentylamine)(1-méthyl-3-cyclohexyl-1,2,4-triazol-5-ylidène) platine(II)
- *trans*-diiodo(*N*-cycloheptylamine (1-méthyl-3-cyclohexyl-1,2,4-triazol-5-ylidène)platine(II)
- *trans*-diiodo(*N*-bicyclo[2.2.2]octylamine)(1-méthyl-3-cyclohexyl-1,2,4-triazol-5-ylidène) platine(II)
- *trans*-diiodo(*N*-endo(+/-)bicyclo[2.2.1]heptylamine)(1-méthyl-3-cyclohexyl-1,2,4-triazol-5-ylidène) platine(II)
- *trans*-diiodo(*N*-exo(+/-)bicyclo[3.2.1]octylamine)(1-méthyl-3-cyclohexyl-1,2,4-triazol-5-ylidène) platine(II)
- *trans*-diiodo(*N*-benzylamine)(1-méthyl-3-cyclohexyl-1,2,4-triazol-5-ylidène) platine(II)
- *trans*-diiodo(*N*-4-méthoxybenzylamine)(1-méthyl-3-cyclohexyl-1,2,4-triazol-5-ylidène) platine(II)
- *trans*-diiodo(*N*-4-trifluorométhylebenzylamine)(1-méthyl-3-cyclohexyl-1,2,4-triazol-5-ylidène) platine(II)
- *trans*-diiodo(*N*-4-chlorobenzylamine) (1-méthyl-3-cyclohexyl-1,2,4-triazol-5-ylidène) platine(II)
- *trans*-diiodo(*N*-4-fluorobenzylamine)(1-méthyl-3-cyclohexyl-1,2,4-triazol-5-ylidène) platine(II)
- *trans*-diiodo(*N*-4-bromobenzylamine)(1-méthyl-3-cyclohexyl-1,2,4-triazol-5-ylidène) platine(II)
- *trans*-diiodo(*N*-4-méthylbenzylamine)(1-méthyl-3-cyclohexyl-1,2,4-triazol-5-ylidène) platine(II)
- *trans*-diiodo(*N*-3,4-dichlorobenzylamine)(1-méthyl-3-cyclohexyl-1,2,4-triazol-5-ylidène) platine(II)
- *trans*-diiodo(*N*-3,4-diméthoxybenzylamine)(1-méthyl-3-cyclohexyl-1,2,4-triazol-5-ylidène) platine(II)
- *trans*-diiodo(*N*-cyclopropylamine)(1-méthyl-3-benzyl-1,2,4-triazol-5-ylidène) platine(II)
- *trans*-diiodo(*N-*cyclobutylamine)(1-méthyl-3-benzyl-1,2,4-triazol-5-ylidène) platine(II)
- *trans*-diiodo(*N*-cyclopentylamine)(1-méthyl-3-benzyl-1,2,4-triazol-5-ylidène) platine(II)
- *trans*-diiodo(*N*-cycloheptylamine)(1-méthyl-3-benzyl-1,2,4-triazol-5-ylidène) platine(II)
- *trans*-diiodo(*N*-bicyclo[2.2.2]octylamine)(1-méthyl-3-benzyl-1,2,4-triazol.5-ylidène) platine(II)
- *trans*-diiodo(*N*-endo(+/-)bicyclo[2.2.1]heptylamine)(1-méthyl-3-benzyl-1,2,4-triazol-5-ylidène) platine(II)
- *trans*-diiodo(*N*-exo(+/-)bicyclo[3.2.1]octylamine)(1 -méthyl-3-benzyl-1,2,4-triazol-5-ylidène) platine(II)
- *trans*-diiodo(*N*-benzylamine)(1-méthyl-3-benzyl-1,2,4-triazol-5-ylidène) platine(II)
- *trans*-diiodo(*N*-4-méthoxybenzylamine) (1-méthyl-3-benzyl-1,2,4-triazol-5-ylidène)platine(II)
- *trans*-diiodo(*N*-4-trifluorométhylebenzylamine) (1-méthyl-3-benzyl-1,2,4-triazol-5-ylidène) platine(II)
- *trans*-diiodo(*N*-4-chlorobenzylamine)(1 -méthyl-3-benzyl-1,2,4-triazol-5-ylidène) platine(II)
- *trans*-diiodo(*N*-4-fluorobenzylamine)(1-méthyl-3-benzyl-1,2,4-triazol-5-ylidène) platine(II)
- *trans*-diiodo(*N*-4-bromobenzylamine)(1-méthyl-3-benzyl-1,2,4-triazol-5-ylidène) platine(II)
- *trans*-diiodo(*N*-4-méthylbenzylamine)(1-méthyl-3-benzyl-1,2,4-triazol-5-ylidène) platine(II)
- *trans*-diiodo(*N*-3,4-dichlorobenzylamine)(1-méthyl-3-benzyl-1,2,4-triazol-5-ylidène) platine(II)
- *trans*-diiodo(*N*-3,4-diméthoxybenzylamine)(1-méthyl-3-benzyl-1,2,4-triazol-5-ylidène) platine(II)
- *trans*-diiodo(*N*-cyclopropylamine)[1-méthyl-3-(4-trifluorométhylbenzyl)-1,2,4-triazol-5-ylidène] platine(II)
- *trans*-diiodo(*N*-cyclobutylamine)[1-méthyl-3-(4-trifluorométhylbenzyl)-1,2,4-triazol-5-ylidène] platine(II)
- *trans*-diiodo(*N*-cyclopentylamine)[1-méthyl-3-(4-trifluorométhylbenzyl)-1,2,4-triazol-5-ylidène] platine(II)
- *trans*-diiodo(*N*-cycloheptylamine)[1-méthyl-3-(4-trifluorométhylbenzyl)-1,2,4-triazol-5-ylidène] platine(II)
- *trans*-diiodo(*N*-bicyclo[2.2.2]octylamine)[1-méthyl-3-(4-trifluorométhylbenzyl)-1,2,4-triazol-5-ylidène] platine(II)
- *trans*-diiodo(*N*-endo(+/-)bicyclo[2.2.1]heptylamine)[1-méthyl-3-(4-trifluorométhylbenzyl)-1,2,4-triazol-5-ylidène] platine(II)
- *trans*-diiodo(*N*-exo(+/-)bicyclo[3.2.1]octylamine)[1-méthyl-3-(4-trifluorométhylbenzyl)-1,2,4-triazol-5-ylidène) platine(II)
- *trans*-diiodo(*N*-benzylamine)[1-méthyl-3-(4-trifluorométhylbenzyl)-1,2,4-triazol-5-ylidène] platine(II)
- *trans*-diiodo(*N*-4-méthoxybenzylamine)[1-méthyl-3-(4-trifluorométhylbenzyl)-1,2,4-triazol-5-ylidène] platine(II)
- *trans*-diiodo(N-4-trifluorométhylebenzylamine)[1-méthyl-3-(4-trifluorométhylbenzyl)-1,2,4-triazol-5-ylidène] platine(II)
- *trans*-diiodo(*N*-4-chlorobenzylamine)[1-méthyl-3-(4-trifluorométhylbenzyl)-1,2,4-triazol-5-ylidène] platine(II)
- *trans*-diiodo(*N*-4-fluorobenzylamine)[1-méthyl-3-(4-trifluorométhylbenzyl)-1,2,4-triazol-5-ylidène] platine(II)
- *trans*-diiodo(*N*-4-bromobenzylamine)[1-méthyl-3-(4-trifluorométhylbenzyl)-1,2,4-triazol-5-ylidène] platine(II)
- *trans*-diiodo(N-4-méthylbenzylamine)[1-méthyl-3-(4-trifluorométhylbenzyl)-1,2,4-triazol-5-ylidène] platine(II)
- *trans*-diiodo(N-3,4-dichlorobenzylamine)[1-méthyl-3-(4-trifluorométhylbenzyl)-1,2,4-triazol-5-ylidène] platine(II)
- *trans*-diiodo(*N*-3,4-diméthoxybenzylamine)[1-méthyl-3-(4-trifluorométhylbenzyl)-1,2,4-triazol-5-ylidène] platine(II)
- *trans*-diiodo(*N*-cyclopropylamine)(1,3-dibenzyl-1,2,4-triazol-5-ylidène) platine(II)
- *trans*-diiodo(*N*-cyclobutylamine)(1,3-dibenzyl-1,2,4-triazol-5-ylidène) platine(II)
- *trans*-diiodo(*N*-cyclopentylamine)(1,3-dibenzyl-1,2,4-triazol-5-ylidène)platine(II)
- *trans*-diiodo(*N*-cycloheptylamine)(1,3-dibenzyl-1,2,4-triazol-5-ylidène) platine(II)
- *trans*-diiodo(*N*-bicyclo[2.2.2]octylamine)(1,3-dibenzyl-1,2,4-triazol-5-ylidène) platine(II)
- *trans*-diiodo(*N*-endo(+/-)bicyclo[2.2.1]heptylamine)(1,3-dibenzyl-1,2,4-triazol-5-ylidène) platine(II)
- *trans*-diiodo(*N*-exo(+/-)bicyclo[3.2.1]octylamine)(1,3-dibenzyl-1,2,4-triazol-5-ylidène) platine(II)
- *trans*-diiodo(*N*-benzylamine)(1,3-dibenzyl-1,2,4-triazol-5-ylidène) platine(II)
- *trans*-diiodo(*N*-4-méthoxybenzylamine)(1,3-dibenzyl-1,2,4-triazol-5-ylidène) platine(II)
- *trans*-diiodo(N-4-trifluorométhylebenzylamine)(1,3-dibenzyl-1,2,4-triazol-5-ylidène) platine(II)
- *trans*-diiodo(*N*-4-chlorobenzylamine)(1,3-dibenzyl-1,2,4-triazol-5-ylidène) platine(II)
- t*rans*-diiodo(N-4-fluorobenzylamine)(1,3-dibenzyl-1,2,4-triazol-5-ylidène) platine(II)
- *trans*-diiodo(*N*-4-bromobenzylamine)(1,3-dibenzyl-1,2,4-triazol-5-ylidène) platine(II)
- *trans*-diiodo(*N*-4-méthylbenzylamine)(1,3-dibenzyl-1.2,4-triazol-5-ylidène) platine(II)
- *trans*-diiodo(*N*-3,4-dichlorobenzylamine)(1,3-dibenzyl-1,2,4-triazol-5-ylidène) platine(II)
- *trans*-diiodo(*N*-3,4-diméthoxybenzylamine)(1,3-dibenzyl-1,2,4-triazol-5-ylidène) platine(II)
- *trans*-diiodo(*N*-cyclopropylamine)[1,3-diméthyl-4-phényl-imidazol-2-ylidène] platine(II)
- *trans*-diiodo(*N*-cyclobutylamine)[1,3-diméthyl-4-phényl-imidazol-2-ylidène] platine(II)
- *trans*-diiodo(*N*-cyclopentylamine)[1,3-diméthyl-4-phényl-imidazol-2-ylidène] platine(II)
- *trans*-diiodo(*N*-cycloheptylamine)[1,3-diméthyl-4-phényl-imidazol-2-ylidène] platine(II)
- *trans*-diiodo(*N*-bicyclo[2.2.2]octylamine)[1,3-diméthyl-4-phényl-imidazol-2-ylidène]platine(II)
- *trans*-diiodo(*N*-endo(+/-)bicyclo[2.2.1]heptylamine)[1,3-diméthyl-4-phényl-imidazol-2-ylidène] platine(II)
- *trans*-diiodo(*N*-exo(+/-)bicyclo[3.2.1]octylamine)[1,3-diméthyl-4-phényl-imidazol-2-ylidène] platine(II)
- *trans*-diiodo(*N*-benzylamine)[1,3-diméthyl-4-phényl-imidazol-2-ylidène] platine(II)
- *trans*-diiodo(*N*-4-méthoxybenzylamine)[1,3-diméthyl-4-phényl-imidazol-2-ylidène] platine(II)
- *trans*-diiodo(*N*-4-trifluorométhylebenzylamine)[1,3-diméthyl-4-phényl-imidazol-2-ylidène] platine(II)
- *trans*-diiodo(*N*-4-chlorobenzylamine)[1,3-diméthyl-4-phényl-imidazol-2-ylidène] platine(II)
- *trans*-diiodo(*N*-4-fluorobenzylamine)[1,3-diméthyl-4-phényl-imidazol-2-ylidène] platine(II)
- *trans*-diiodo(*N*-4-bromobenzylamine)[1,3-diméthyl-4-phényl-imidazol-2-ylidène] platine(II)
- *trans*-diiodo(*N*-4-méthylbenzylamine)[1,3-diméthyl-4-phényl-imidazol-2-ylidène] platine(II)
- *trans*-diiodo(*N*-3,4-dichlorobenzylamine)[1,3-diméthyl-4-phényl-imidazol-2-ylidène] platine(II)
- *trans*-diiodo(*N*-3,4-diméthoxybenzylamine)[1,3-diméthyl-4-phényl-imidazol-2-ylidène] platine(II)
- *trans*-diiodo(*N*-3,4-diméthylbenzylamine)[1,3-diméthyl-4-phényl-imidazol-2-ylidène] platine(II)
- *trans*-diiodo(*N*-cyclopropylamine)[3-benzyl-1-méthyl-4-phényt-imidazol-2-ylidène] platine(II)
- *trans*-diiodo(*N*-cyclobutylamine)[3-benzyl-1-méthyl-4-phényl-imidazol-2-ylidène] platine(II)
- *trans*-diiodo(*N*-cyclopentylamine)[3-benzyl-1-méthyl-4-phényi-imidazol-2-ylidène] platine(II)
- *trans*-diiodo(*N*-cycloheptylamine)[3-benzyl-1-méthyl-4-phényl-imidazol-2-ylidène] platine(II)
- *trans*-diiodo(*N*-bicyclo[2.2.2]octylamine)[3-benzyl-1-méthyl-4-phényl-imidazol-2-ylidène] platine(II)
- trans-diiodo(N-endo(+/-)bicyclo[2.2.1]heptylamine)[3-benzyl-1-méthyl-4-phényl-imidazol-2-ylidène] platine(II)
- *trans*-diiodo(*N*-exo(+/-)bicyclo[3.2.1]octylamine)[3-benzyl-1-méthyl-4-phényl-imidazol-2-ylidène] platine(II)
- *trans*-diiodo(*N*-benzylamine)[3-benzyl-1-méthyl-4-phényl-imidazol-2-ylidène] platine(II)
- *trans*-diiodo(*N*-4-méthoxybenzylamine)[3-benzyl-1-méthyl-4-phényl-imidazol-2-ylidène] platine(II)
- *trans*-diiodo(*N*-4-trifluorométhylebenzylamine)[3-benzyl-1-méthyl-4-phényl-imidazol-2-ylidène] platine(II)
- *trans*-diiodo(*N*-4-chlorobenzylamine)[3-benzyl-1-méthyl-4-phényl-imidazol-2-ylidène] platine(II)
- *trans*-diiodo(*N*-4-fluorobenzylamine)[3-benzyl-1-méthyl-4-phényl-imidazol-2-ylidène] platine(II)
- trans-diiodo(N-4-bromobenzylamine)[3-benzyl-1-méthyl-4-phényl-imidazol-2-ylidène] platine(II)
- trans-diiodo(N-4-méthylbenzylamine)[3-benzyl-1-méthyl-4-phényi-imidazol-2-ylidène] platine(II)
- *trans*-diiodo(*N*-3,4-dichlorobenzylamine)[3-benzyl-1-méthyl-4-phényl-imidazol-2-ylidène] platine(II)
- *trans-*diiodo(*N*-3,4-diméthoxybenzylamine [3-benzyl-1-méthyl-4-phényl-imidazol-2-ylidène] platine(II)
- *trans*-diiodo(*N*-3,4-diméthylbenzylamine)[3-benzyl-1-méthyl-4-phényl-imidazol-2-ylidène] platine(II)
- trans-diiodo(N-cyclopropylamine)[1,3-diméthyl-4,5-diphényi-imidazol-2-ylidène] platine(II)
- *trans*-diiodo(N-cyclobutylamine)[1,3-diméthyl-4,5-diphényl-imidazol-2-ylidène] platine(II)
- *trans*-diiodo(*N*-cyclopentylamine)[1,3-diméthyl-4,5-diphényl-imidazol-2-ylidène] platine(II)
- *trans*-düodo(*N*-cycloheptylamine)[1,3-diméthyl-4,5-diphényl-imidazol-2-ylidène] platine(II)
- *trans*-diiodo(*N*-bicyclo[2.2.2]octylamine)[1,3-diméthyl-4,5-diphényl-imidazol-2-ylidène] platine(II)
- *trans*-diiodo(*N*-endo(+/-)bicyclo[2.2.1]heptylamine)[1,3-diméthyl-4,5-diphényl-imidazol-2-ylidène] platine(II)
- *trans*-diiodo(*N*-exo(+/-)bicyclo[3.2.1]octylamine)[1,3-diméthyl-4,5-diphényl-imidazol-2-ylidène] platine(II)
- *trans*-diiodo(*N*-benzylamine)[1,3-diméthyl-4,5-diphényl-imidazol-2-ylidène] platine(II)
- *trans*-diiodo(*N*-4-méthoxybenzylamine)[1,3-diméthyl-4,5-diphényl-imidazol-2-ylidène] platine(II)
- *trans*-diiodo(*N*-4-trifluorométhylebenzylamine)[1,3-diméthyl-4,5-diphényl-imidazol-2-ylidène] platine(II)
- *trans*-diiodo(*N*-4-chlorobenzylamine)[1,3-diméthyl-4,5-diphényl-imidazol-2-ylidène] platine(il)
- *trans*-düodo(N-4-fluorobenzylamine)[1,3-diméthyl-4,5-diphényl-imidazol-2-ylidène] platine(II)
- *trans*-diiodo(*N*-4-bromobenzylamine)[1,3-diméthyl-4,5-diphényl-imidazol-2-ylidène] platine(II)
- *trans*-diiodo(*N*-4-méthylbenzylamine)[1,3-diméthyl-4,5-diphényl-imidazol-2-ylidène] platine(II)
- *trans*-diiodo(*N*-3,4-dichlorobenzylamine)[1,3-diméthyl-4,5-diphényl-imidazol-2-ylidène] platine(II)
- *trans*-diiodo(N-3,4-diméthoxybenzylamine)[1,3-diméthyl-4,5-diphényl-imidazol-2-ylidène] platine(II)
- *trans*-diiodo(*N*-3,4-diméthylbenzylamine)[1,3-diméthyl-4,5-diphényl-imidazol-2-ylidène] platine(II)

Conformément à l'invention, on peut préparer les composés de formule générale (I) selon le procédé qui suit. 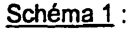 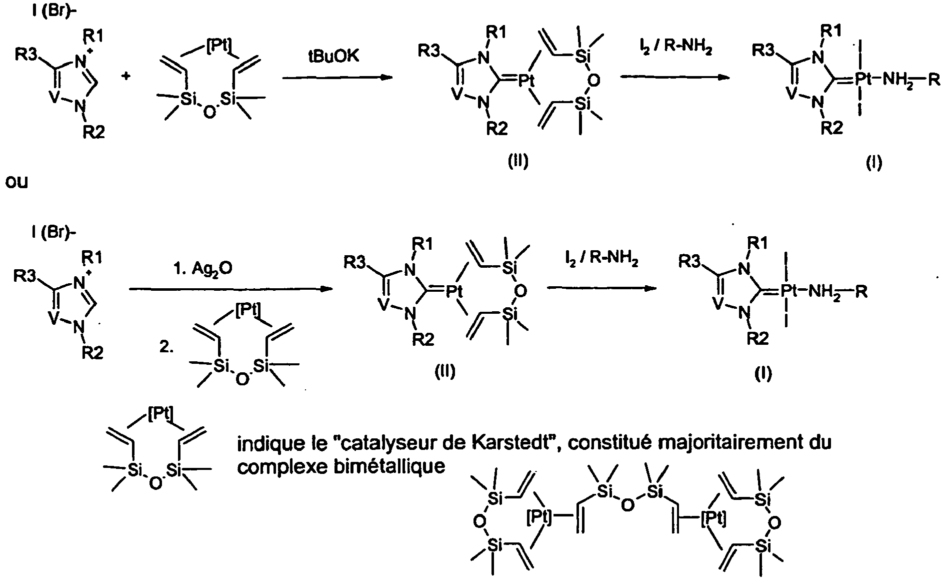 Ou selon le schéma 2 suivant : 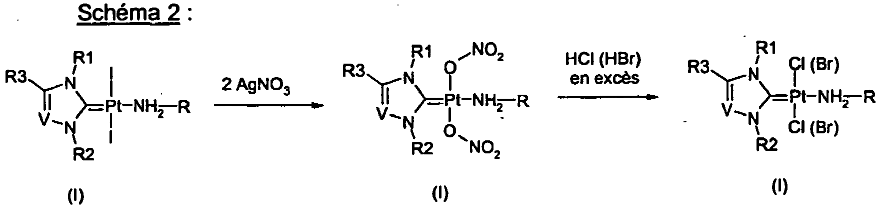 Ou selon le schéma 3 suivant :

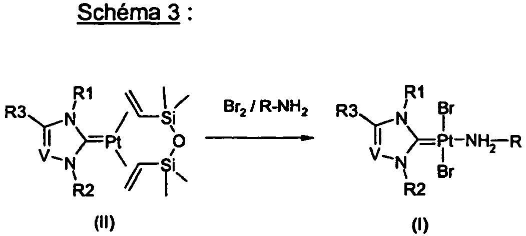

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les composés de formule (II) sont utiles comme intermédiaires de synthèse des composés de formule (I). Ils sont décrits, notamment en tant que catalyseurs par exemple dans le brevet WO 02/098888, ou dans Journal of Organometallic Chemistry (2005), 290(24-25), 6156-68, ou dans Organometallics (2007), 26(24), 5782-85 ou dans Organometallics (2007), 26(24), 5782-85 ou dans Journal of Materials Chemistry (2007), 17(15), 1476-84.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : trans-diiodo(N-cyclohexylamine)(1,3-diméthylimidazol-2-ylidène) platine(II)

A une solution de complexe (1,3-diméthylimidazol-2-ylidène)(1,3-divinyl-1,1,3,3-tetraméthyldisiloxane) platine(0) (0,3 g ; 0,68 mmol) - qui a été obtenu selon G. Berthon Gelloz et coll, Journal of Organometallic Chemistry (2005), 290(24-25), 6156-68 - dans 40 mL de toluène à 0°C est additionnée une solution de diiode (0,17 g ; 0,68 mmol) dans 20 mL de toluène. Par la suite 78 µL de cyclohexylamine (0,68 mmol) sont additionnés au mélange. Le mélange réactionnel est agité pendant 4h à température ambiante puis évaporé à sec. Le résidu obtenu est purifié par chromatographie flash par élution avec un mélange d'heptane et d'acétate d'éthyle (70/30 en volumes), on recueille ainsi 0,27 g (61%) de *trans*-diiodo(*N*-cyclohexylamine)(1,3-diméthylimidazol-2-ylidène) platine(II), sous forme d'une poudre jaune dont les carctéristiques sont Is suivantes :
- spectre de RMN ¹H (300 MHz, CDCl₃, 20°C) δ [ppm] = 1.1-1.5 (5H), 1.64 (m, 1 H), 1.78 (m, 3H), 2.29 (m, 2H), 2.90 (br, 2H), 3.25 (m, 1 H), 3.85 (s, 6H), 6.79 (s, 2H).
- Analyse élémentaire, % mesuré (calc.) pour C₁₁H₂₁I₂N₃Pt : C, 20.79 (20.51); H, 3.21 (3.29); N, 6.26 (6.48).

### Exemple 2: trans-diiodo(N-cyclohexylamine)(1,4-diméthyl-1,2,4-triazol-5-ylidène) platine (II)

### Etape 1 : [1,4-diméthyl-1,2,4-triazol-5-ylidène (1,3-divinyl-1,1,3,3-tetraméthyl-disiloxane)] platine(0)

A une solution de 1,3-divinyl-1,1,3,3-tetraméthyldisiloxane platine(0) 0,1 M dans le xylène (4,2 mL ; 0,42 mmol) est ajouté l'iodure de 1,4-diméthyl-1,2,4-triazol-1-ium (0,10 g ; 0,42 mmol). Le mélange est agité pendant 1 h30 à température ambiante puis refroidi à 0°C. Par la suite 0,06 g de *tert*-butylate de potassium (0,56 mmol). Le mélange réactionnel est agité pendant 2 jours à température ambiante puis évaporé à sec. Le résidu obtenu est purifié par chromatographie flash par élution avec un mélange d'heptane et d'acétate d'éthyle (80/20 en volumes), on recueille ainsi 0,18 g (93 %) de [1,4-diméthyl-1,2,4-triazol-5-ylidène (1,3-divinyl-1,1,3,3-tetraméthyldisiloxane)] platine(0), dont les caractéristiques sont les suivantes :
- Spectre de RMN ¹H (300 MHz, CDCl₃, 20°C) δ [ppm] = -0.28 (s, 6H), 0.32 (s, 6H), 1.8-2.1 (m, 4H), 2.2-2.4 (m, 2H), 3.55 (s, 3H), 3.73 (s, 3H), 8.02 (s, 1H).

### Etape 2: trans-diiodo(N-cyclohexylamine)(1,4-diméthyl-1,2,4-triazol-5-ylidène) platine(II)

A une solution de complexe [1,4-diméthyl-1,2,4-triazol-5-ylidène (1,3-divinyl-1,1,3,3-tetraméthyldisiloxane)] platine(0) (0,22 g ; 0, 5 mmol) dans 20 mL de toluène à 0°C est additionnée une solution de diiode (0,13 g ; 0,5 mmol) dans 30 mL de toluène. Par la suite 60 µL de cyclohexylamine (0,5 mmol) sont additionnés au mélange. Le mélange réactionnel est agité pendant 16h à température ambiante puis évaporé à sec. Le résidu obtenu est purifié par chromatographie flash par élution avec un mélange d'heptane et d'acétate d'éthyle (70/30 en volumes), on recueille ainsi 0,19 g (60%) de *trans*-diiodo(*N*-cyclohexylamine)(1,4-diméthyl-1,2,4-triazol-5-ylidène) platine(II), sous forme de cristaux jaunes dont les caractéristique sont les suivantes ;
- spectre de RMN ¹H (500 MHz, CDCl₃, 20°C) δ [ppm] = 1.1-1.4 (5H), 1.65 (m, 1H), 1.79 (m, 2H), 2.28 (m, 2H), 3.01 (br, 2H), 3.27 (m, 1H), 3.86 (s, 3H), 4.05 (s, 3H), 7.82 (s, 1H).

### Exemple 3 : trans-diiodo(N-cyclohexylamine)(1-méthyl-3-cyclohexylméthyl-imidazol-2-ylidène) platine(II)

### Etape 1: iodure de 1-methyl-3-cyclohexylmethyl-imidazolium

Une solution de 1-méthylimidazole (0,9 mL ; 10,8 mmol) dans le bromure de cyclohexylméthyle (1,7 mL ; 11,9 mmol) est chauffée à 100°C pendant 16 heures, puis les volatiles sont évaporés sous vide. Le résidu obtenu est recristallisé dans un 5 mL d'acétate d'éthyle permettant ainsi d'obtenir 2,8 g d'iodure de 1-méthyl-3-cyclohexylméthyl-imidazolium, dont les caractéristiques sont les suivantes :
Spectre de RMN ¹H (300 MHz, CDCl₃, 20°C) δ [ppm] = 1.0-1.9 (11H), 4.13 (s, 3H), 4.15 (d, 2H), 7.26 (s, 1H), 7.42 (s, 1H), 10.48 (s, 1H).

### Etape 2: [1-méthyl-3-cyclohexylméthyl-imidazol-2-ylidène (1,3-divinyl-1,1,3,3-tetraméthyldisiloxane)] platine(0)

A un mélange de 1,3-divinyl-1,1,3,3-tetraméthyldisiloxane platine(0) 0,1 M dans le xylène (6 mL; 0,60 mmol) et de 1-méthyl-3-méthylcyclohexylimidazolium, obtenu à l'étape 1, (0,15 g ; 0,60 mmol), on ajoute du *tert*-butylate de potassium (0,09 g ; 0,80 mmol) à 0°C. Le mélange réactionnel est agité pendant 16h à température ambiante puis évaporé à sec. Le résidu obtenu est purifié par chromatographie flash par élution avec un mélange d'heptane et d'acétate d'éthyle (90/10 en volumes), on recueille ainsi 0,30 g (83 %) de [1-méthyl-3-cyclohexylméthyl-imidazol-2-ylidène (1,3-divinyl-1, 1,3,3-tetraméthyldisiloxane)] platine(0), dont les caractéristiques sont les suivantes :
- Spectre de RMN ¹H (300 MHz, CDCl₃, 20°C) δ [ppm] = -0.26 (s, 6H), 0.33 (s, 6H), 0.8-0.9 (m, 3H), 1.1-1.2 (m, 4H), 1.5-2.0 (m, 8H), 2.21 (d, 2H), 3.50 (s, 3H), 3.68 (m, 2H), 6.97 (m, 2H).

### Etape 3: trans-diiodo(N-cyclohexylamine)(1-méthyl-3-cyclohexylméthyl-imidazol-2-ylidène) platine(II)

A une solution de complexe de [1-méthyl-3-cyclohexylméthyl-imidazol-2-ylidène (1,3-divinyl-1,1,3,3-tetraméthyldisiloxane)] platine(0) (0,28 g ; 0,5 mmol) dans 20 mL de toluène à 0°C est additionnée une solution de diiode (0,13 g ; 0,5 mmol) dans 30 mL de toluène. Par la suite 60 µL de cyclohexylamine (0,5 mmol) sont additionnés au mélange. Le mélange réactionnel est agité pendant 16h à température ambiante puis évaporé à sec. Le résidu obtenu est purifié par chromatographie flash par élution avec un mélange d'heptane et d'acétate d'éthyle (90/10 en volumes), on recueille ainsi 0,23 g (62%) de *trans*-diiodo(*N*-cyclohexylamine)(1-méthyl-3-cyclohexylméthyl-imidazol-2-ylidène) platine(II), sous forme de crisatux jaunes dont les caractéristiques sont les suivantes :
- RMN ¹H (500 MHz, CDCl₃, 20°C) δ [ppm] = 1.0-1.4 (12H), 1.6-1.9 (m, 8H), 2.30 (m, 2H), 2.55 (m, 1H), 2.92 (br, 2H), 3.26 (m, 1H), 3.86 (s, 3H), 4.07 (d, 2H, J = 7.5 Hz), 6.74 (d, 1H), 6.77 (d, 1H).
- Analyse élémentaire (%) trouvé (calc.) pour C₁₇H₃₁I₂N₃Pt : C, 28.12 (28.11); H, 4.11 (4.30); N, 5.59 (5.79).
- Diagramme ORTEP - diffraction de rayons X. Cristaux obtenus à partir d'une solution du complexe dans le chloroforme, par évaporation lente du solvant.

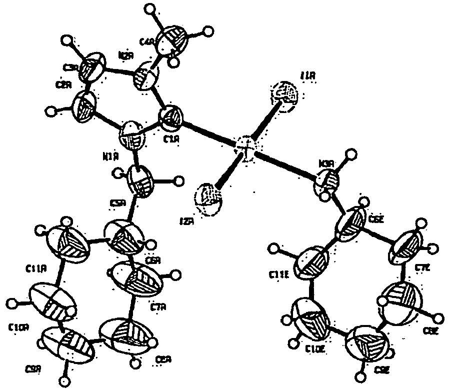

### Exemple 4 : trans-diiodo(N-cyclohexylamine)(1-méthyl-3-phénylimidazol-2-ylidène) platine(II)

A une solution de complexe [1-méthyl-3-(phenyl)imidazol-2-ylidène (1,3-divinyl-1,1,3,3-tetraméthyldisiloxane)] platine(0) (0,14 g ; 0,26 mmol) - qui a été obtenu selon D. Brissy et coll., Organometallics (2007), 26(24), 5782-85 - dans 10 mL de toluène à 0°C est additionnée une solution de diiode (0,07 g ; 0,26 mmol) dans 15 mL de toluène. Par la suite 33 µL de cyclohexylamine (0,29 mmol) sont additionnés au mélange. Le mélange réactionnel est agité pendant 16h à température ambiante puis évaporé à sec. Le résidu obtenu est purifié par chromatographie flash par élution avec un mélange d'heptane et d'acétate d'éthyle (90/10 en volumes), on recueille ainsi 0,07 g (38%) de *trans*-diiodo(*N*-cyclohexylamine)(1-méthyl-3-phénylimidazol-2-ylidène) platine(II), sous forme d'une poudre jaune dont les caractéristiques sont les suivantes :.
- spectre de RMN ¹H (300 MHz, CDCl₃, 20°C) δ [ppm] = 1.0-1.4 (5H), 1.5-1.8 (3H), 2.07 (m, 2H), 2.78 (br, 2H), 3.08 (m, 1H), 3.97 (s, 3H), 6.97 (d, 2H), 7.06 (d, 2H), 7.4-7.6 (3H), 7.85 (m, 2H).
- spectre de masse (HRMS, ESI mode positif) calculé pour C₁₆H₂₃I₂N₃Pt.Na : 728.9527 ; trouvé pour C₁₆H₂₃I₂N₃NaPt : 728.9527.

### Exemple 5 : trans-diiodo(N-cyclohexylamine)(1-méthyl-3-benzyl-imidazol-2-ylidène) platine(II)

### Etape 1 : [1-méthyl-3-benzyl-imidazol-2-ylidène (1,3-divinyl-1,1,3,3-tetraméthyldisiloxane)] platine(0)

A un mélange de 1,3-divinyl-1,1,3,3-tetraméthyldisiloxane platine(0) 0,1 M dans le xylène (3,3 mL ; 0,33 mmol) et d'iodure de 1-méthyl-3-benzyl-imidazolium (0,10 g ; 0,33 mmol) - qui a été obtenu selon A. M. Magill et coll., Journal of Organometallic Chemistry (2001), 617-618, 546-60 - on ajoute du *tert*-butylate de potassium (0,06 g ; 0,52 mmol) à 0°C. Le mélange réactionnel est agité pendant 16h à température ambiante puis évaporé à sec. Le résidu obtenu est purifié par chromatographie flash par élution avec un mélange d'heptane et d'acétate d'éthyle (90/10 en volumes), on recueille ainsi 0,15 g (82 %) de [1-méthyl-3-benzyl-imidazol-2-ylidène (1,3-divinyl-1,1,3,3-tetraméthyldisiloxane)] platine(0), dont les caractéristiques sont les suivantes :
- Spectre RMN ¹H (300 MHz, CDCl₃, 20°C) δ [ppm] = -0.33 (s, 6H), 0.34 (s, 6H,), 1.8-2.0 (m, 4H), 2.23 (d, 2H), 3.56 (s, 3H), 5.14 (s, 2H), 6.91 (d, 1H), 7.02 (d, 1H), 7.1-7.2 (m, 2H), 7.2-7.4 (m, 3H).

### Etape 2 : trans-diiodo(N-cyclohexylamine)(1-méthyl-3-benzyl-imidazol-2-ylidène) platine(II)

A une solution de complexe [1-méthyl-3-benzyl-imidazol-2-ylidène](1,3-divinyi-1,1,3,3-tetraméthyldisiloxane)] platine(0) (0.11 g, 0.2 mmol) dans 8 mL de toluène à 0°C est additionnée une solution de diiode (0.05 g, 0.2 mmol) dans 12 mL de toluène. Par la suite 25 µL de cyclohexylamine (0.22 mmol) sont additionnés au mélange. Le mélange réactionnel est agité pendant 16h à température ambiante puis évaporé à sec. Le résidu obtenu est purifié par chromatographie flash par élution avec un mélange d'heptane et d'acétate d'éthyle (90/10 en volumes), on recueille ainsi 0.07 g (49%) de *trans*-diiodo(*N-*cyclohexylamine)(1-méthyl-3-benzyl-imidazol-2-ylidène) platine(II), sous forme de cristaux jaunes dont les caractéristiques sont les suivantes ;
- spectre de RMN ¹H (300 MHz, CDCl₃, 20°C) δ [ppm] = 1.1-1.9 (8H), 2.28 (m, 2H), 2.93 (br, 2H), 3.25 (m, 1H), 3.89 (s, 3H), 5.59 (s, 2H), 6.56 (d, 2H), 6.77 (s, 2H), 7.3-7.5 (5H).
- Diagramme ORTEP - diffraction de rayons X. Cristaux obtenus à partir d'une solution du complexe dans le chloroforme, par évaporation lente du solvant.

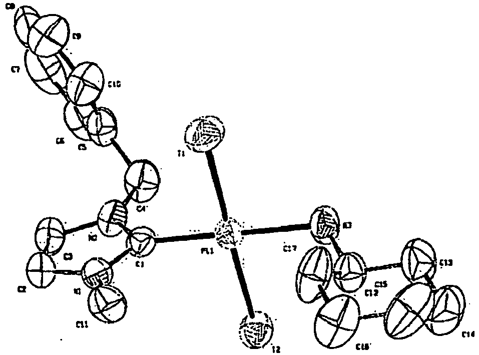

### Exemple 6 : trans-diiodo(N-exo(+/-)bicyclo[2.2.1]heptylamine)(1.3-diméthyl-imidazol-2-ylidène)platine (II)

Dans un tricol de 100 mL sous argon, on dissout 0,310 g (0,649 mmol) de (1,3-diméthyl-imidazol-2-ylidène)(1,3-divinyl-1,1,3,3-tétraméthyldisiloxane) platine (0) - qui a été obtenu selon G Berthon-Gelloz et coll., Journal of Organometallic Chemistry (2005), 690(24-25), 6156-68 - dans 50 mL de toluène puis on refroidit à 0°C. On ajoute alors, goutte à goutte à 0°C, successivement une solution de 0,167 g (0,649 mmol) de diiode dans 10 mL de toluène puis 72,2 mg (0,649 mmol) d'exo-norbornylamine racémique. Le milieu réactionnel est ensuite agité à température ambiante pendant 4 h. Le milieu réactionnel est évaporé à sec. Le résidu ainsi obtenu est purifié par flash-chromatographie en éluant avec un mélange de cyclohexane et d'acétate d'éthyle (85/15 en volumes). On obtient ainsi 188 mg (44%) de *trans*-diiodo(*N*-exo(+/-)bicyclo[2.2.1]heptylamine)[1,3-diméthyl-imidazol-2-ylidène]platine (II), sous forme de fins cristaux jaune-pâle, dont les caractéristiques sont les suivantes :
- Rf = 0,22 (gel de silice ; mélange de 80% de cyclohexane et 20% d'acétate d'éthyle)
- Spectre de RMN¹H (CDCl₃, 20°C), δ [ppm] : 1.13 (m, 1 H), 1.25 (m, 2 H), 1.42 - 1.60 (m, 3 H), 1.73 (m, 1 H), 1.78 (m, 1 H), 2.33 (t, J=4.6 Hz, 1 H), 2.44 (d, J=4.6 Hz, 1 H), 2.88 (m, 2 H), 3.44 (m, 1 H) 3.85 (s, 6 H) 6.79 (s, 2 H).

### Exemple 7 : trans-diiodo(N-exo(+/-)bicyclo[2.2.1]heptylamine)(1,3-diméthyl-benzimidazol-2-ylidène)platine (II)

Dans un tricol de 100 mL sous argon, on dissout 0,316 g (0,6 mmol) de (1,3-diméthyl-benzimidazol-2-ylidène)(1,3-divinyl-1,1,3,3-tétraméthyldisiloxane) platine (0) - qui a été obtenu selon G Berthon-Gelloz et coll., Journal of Organometalic Chemistry (2005), 690(24-25), 6156-68 - dans 55 mL de toluène puis on refroidit à 0°C. On ajoute alors, goutte à goutte à 0°C, successivement une solution de 0,152 g (0,6 mmol) de diiode dans 10 mL de toluène puis 66,7 mg (0,6 mmol) d'exo-norbornylamine racémique. Le milieu réactionnel est ensuite agité à température ambiante pendant 6 h. Le milieu réactionnel est évaporé à sec. Le résidu ainsi obtenu est purifié par flash-chromatographie en éluant avec un mélange de cyclohexane et d'acétate d'éthyle (90/10 en volumes). On obtient ainsi 207 mg (49%) de *trans*-diiodo(*N*-exo(+/- )bicyclo[2.2.1]heptylamine)[1,3-diméthyl-benzimidazol-2-ylidène]platine (II), sous forme de cristaux jaunes, dont les caractéristiques sont les suivantes :
- Rf = 0,27 (gel de silice ; mélange de 85% de cyclohexane et 15% d'acétate d'éthyle)
- Spectre de RMN¹H (CDCl₃, 20°C), δ [ppm]: 1.15 (m, 1 H), 1.27 (m, 2 H), 1.42-1.64 (m, 3 H), 1.76 (m, 1 H), 1.85 (m, 1 H), 2.37 (t, J=4.6 Hz, 1 H), 2.48 (d, J=4.6 Hz, 1 H), 2.91 - 3.13 (m étalé, 2 H), 3.51 (m, 1 H), 4.08 (s, 6 H), 7.23 - 7.29 (m, 2 H), 7.30 - 7.37 (m, 2 H).

### Exemple 8 : trans-diiodo(N-cyclohexylamine)(1,3-diméthyl-benzimidazol-2-ylidène) platine (II)

Dans un tricol de 100 mL sous argon, on dissout 0,316 g (0,6 mmol) de (1,3-diméthyl-benzimidazol-2-ylidène)(1,3-divinyl-1,1,3,3-tétraméthyldisiloxane) platine (0) - qui a été obtenu selon G Berthon-Gelloz et coll., Journal of Organometalic Chemistry (2005), 690(24-25). 6156-68 - dans 55 mL de toluène puis on refroidit à 0°C. On ajoute alors, goutte à goutte à 0°C, successivement une solution de 0,152 g (0,6 mmol) de diiode dans 10 mL de toluène puis 68,6 µL (0,6 mmol) de cyclohexylamine. Le milieu réactionnel est ensuite agité à température ambiante pendant 6 h. Le milieu réactionnel est évaporé à sec. Le résidu ainsi obtenu est purifié par flash-chromatographie en éluant avec un mélange de cyclohexane et d'acétate d'éthyle (90/10 en volumes). On obtient ainsi 308 mg (74%) de *trans*-diiodo(*N*-cyclohexylamine)[1,3-diméthyl-benzimidazol-2-ylidène]platine (II), sous forme de cristaux jaunes, dont les caractéristiques sont les suivantes :
- Rf = 0,26 (gel de silice ; mélange de 80% de cyclohexane et 20% d'acétate d'éthyle)
- Spectre de RMN¹H (CDCl₃, 20°C), δ [ppm] :1,02 - 1,33 (m, 5 H), 1,56 (m, 1 H), 1,71 (m, 2 H), 2,22 (m, 2H), 3,10 (m, 1 H), 3,82 - 3,94 (m, 2 H), 4,00 (s, 6 H), 7,31 (m, 2 H), 7,62 (m, 2 H).

### Exemple 9 : trans-diiodo(N-cyclohexylamine)(1,3,7,9-tétraméthylxanthine-8-ylidène) platine(II)

### Etape 1 : [1,3,7,9-tétraméthylxanthine-8-ylidène (1,3-divinyl-1,1,3,3-tetraméthyldisiloxane)] platine(0)

A une solution de méthyle carbonate de bis(1,3,7,9-tétraméthylxanthine-8-ylidene) argent (I) - qui a été obtenu selon J. Youngs et coll., Journal of Medicinal Chemistry (2006), 49, 6811-6818 - (4.1 g, 6.8 mmol) dans 240 mL de diméthylformamide est ajoutée une solution de 1,3-divinyl-1,1,3,3-tetraméthyldisiloxane de platine(0) 0,1 M dans le xylène (136 mL; 13.6 mmol). Le mélange réactionnel est agité pendant 16h à température ambiante puis évaporé à sec. Le résidu obtenu est filtré sur Clarcel puis est ensuite purifié par chromatographie flash par élution avec un gradient de mélanges d'heptane et d'acétate d'éthyle (70/30 à 50/50 en volumes), on recueille ainsi 4.2 g (52 %) de [1,3,7,9-tétraméthylxanthine-8-ylidène (1,3-divinyl-1,1,3,3-tetraméthyldisiloxane)] platine(0), dont les caractéristiques sont les suivantes :
- Spectre de RMN ¹H (300 MHz, CDCl₃, 20°C) δ [ppm] = -0.27 (s, 6H), 0.33 (s, 6H), 1.96 (m, 4H), 2.2-2.4 (m, 2H), 3.42 (s, 3H), 3.82 (s, 3H), 3.83 (s, 3H), 3.96 (s, 3H).

### Etape 2: trans-diiodo(N-cyclohexylamine)(1,3,7,9-tétraméthylxanthine-8-ylidène) platine(II)

A une solution de complexe [1,3,7,9-tétraméthylxanthine-8-ylidène (1,3-divinyl-1,1,3,3-tetraméthyldisiloxane)] platine(0) (0,13 g ; 0,22 mmol) dans 11 mL de toluène à 0°C est ajoutée une solution de diiode (0,06 g ; 0,24 mmol) dans 19 mL de toluène. Par la suite 27 µL de cyclohexylamine (0,24 mmol) sont additionnés au mélange. Le mélange réactionnel est agité pendant 16h à température ambiante puis évaporé à sec. Le résidu obtenu est purifié par chromatographie flash par élution avec un mélange d'heptane et d'acétate d'éthyle (70/30 en volumes), on recueille ainsi 0,07 g (39%) de *trans*-diiodo(*N*-cyclohexylamine)(1,3,7,9-tétraméthylxanthine-8-ylidène) platine(II), sous forme de cristaux jaunes dont les caractéristiques sont les suivantes :
- spectre de RMN ¹H (500 MHz, CDCl₃, 20°C) δ [ppm] = 1.1-1.4 (5H), 1.65 (m, 1H), 1.79 (m, 2H), 2.27 (m, 2H), 3.0 (br, 2H), 3.26 (m, 1H), 3.38 (s, 3H), 3.78 (s, 3H), 4.21 (s, 3H), 4.32 (s, 3H).
- spectre de masse HRMS (ESI mode positif) calculée pour C₁₅H₂₅I₂N₅O₂Pt.Na : 778.9643 ; trouvée pour C₁₅H₂₅I₂N₅O₂NaPt : 778.9643.
- Diagramme ORTEP - diffraction de rayons X. Cristaux obtenus à partir d'une solution du complexe dans le chloroforme, par évaporation lente du solvant.

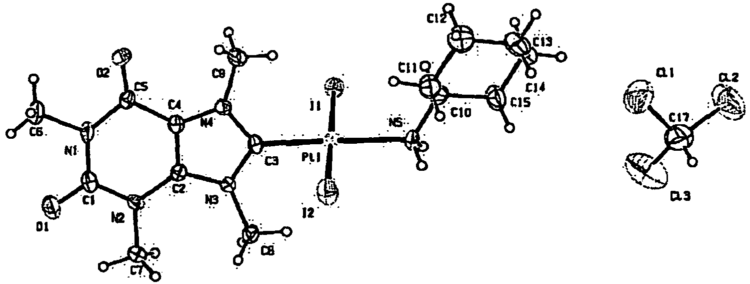

### Exemple 10 : trans-diiodo(N-cyclohexylamine)[1-méthyl-3-(4-méthoxybenzyl) imidazol-2-ylidène]platine (II)

### Etape 1: bromure de 3-(4-méthoxybenzyl)-1-méthyl-imidazolium

Dans un monocol de 50 mL surmonté d'un réfrigérant, sont introduits successivement 1 mL (12,5 mmol) de N-méthylimidazole, puis 5 g (18,8 mmol) de bromure de 4-méthoxybenzyle en solution dans 25 mL de toluène. Le milieu réactionnel est chauffé à reflux, sous agitation, pendant 2h30. Après refroidissement à température ambiante, l'huile visqueuse formée est décantée puis séchée sous vide à température ambiante, on obtient ainsi 4,65 g d'un solide jaune pâle, dont les caractéristiques sont les suivantes :
- Spectre de RMN¹H (DMSO): δ 9.3 ppm (s, 1 H), 7.82 (t, 1H) , 7.74 (t, 1H), 7.43 (d, 2H), 6.98 (d, 2H), 5.38 (s, 2H), 3.87 (s, 3H), 3.77 (s, 3H).

### Etape 2 : [1-méthyl-3-(4-méthoxybenzyl)imidazo-2-ylidène](1,3-divinyl-1,1,3,3-tétraméthyldisiloxane)platine (0).

Dans un monocol de 50 mL sous argon, sont introduits successivement 0.354 g (1,25 mmol) du composé obtenu à l'étape1 et 12.5 mL (1,25 mmol) d'une solution 0,1 M de (1,3-divinyl-1,1,3,3-tétraméthyldisiloxane) platine (0) dans le xylène. Le mélange est refroidit à 0°C, puis on ajoute, goutte à goutte, 1,75 mL (1,75 mmol) d'une solution 1M de *tert*-butylate de potassium dans le tétrahydrofurane. Le milieu réactionnel est agité à température ambiante pendant une nuit. Le milieu réactionnel est évaporé à sec. Le résidu ainsi obtenu est purifié par flash-chromatographie en éluant avec un mélange de cyclohexane et d'acétate d'éthyle (97/3 en volumes). On obtient ainsi 0,47 g (64%) de [1-méthyl-3-(4-méthoxybenzyl)imidazol-2-ylidène](1,3-divinyl-1,1,3,3-tétraméthyl-disiloxane)platine (0), sous forme d'une meringue légèrement jaune, dont les caractéristiques sont les suivantes :
- Spectre de RMN¹H (DMSO): δ 7.44 (t, 2H) , 7.65 (d, 2H), 7.10 (d, 2H), 5.02 (m, 2H), 3.72 (s, 3H), 3.47 (s, 3H), 2.33-1.62 (m, 6H), 0.22 (m, 6H), - 0.30 (m, 6H).

*Etape* 3 : *trans*-diiodo(*N*-cyclohexylamine)[1-méthyl-3-(4-méthoxybenzyl) imidazol-2-ylidène]platine (II).

Dans un tricol de 100 mL sous argon, on dissout 0,230 g (0,394 mmol) du composé obtenu à l'étape 2 dans 30 mL de toluène puis on refroidit à 0°C. On ajoute alors, goutte à goutte à 0°C, successivement une solution de 0,1 g (0,394 mmol) de diiode dans 10 mL de toluène puis 45,1 µL (0,394 mmol) de cyclohexylamine. Le milieu réactionnel est ensuite agité à température ambiante pendant 4 h. Le milieu réactionnel est évaporé à sec. Le résidu ainsi obtenu est purifié par flash-chromatographie en éluant avec un mélange de cyclohexane et d'acétate d'éthyle (80/20 en volumes). On obtient ainsi 51 mg (17%) de *trans*-diiodo(*N*-cyclohexylamine)[1-méthyl-3-(4-méthoxybenzyl) imidazol-2-ylidène]platine (II), sous forme d'une poudre jaune, dont les caractéristiques sont les suivantes :
- Spectre de RMN¹H (CDCl₃): δ 7.45 (d, 2H), 6.95 (d, 2H), 6.82 (d, 1H) , 6.60 (d, 1H), 5.58 (s, 2H), 3.95 (s, 3H), 3.87 (s, 3H), 3.30 (m, 1H), 3.00 (d, 2H), 2.14 (d, 2H), 1.53 (d, 2H), 1.38 (d, 1H), 1.10 (q, 2H), 0.98 (q, 2H), 0.89 (d, 1H)

### Exemple 11 : trans-diiodo(N-cyclohexylamine)[1-méthyl-3-(4-trifluorométhylbenzyl) imidazol-2-ylidène]platine (II)

### Etape 1 : bromure de 1-(4-trifluorométhylbenzyl)-3-méthyl-imidazolium

En opérant comme à l'étape 1 de l'exemple 10, mais à partir de 2 g N-méthylimidazole et 8,73 g de bromure de 4-trifluorométhylbenzyle, au reflux du toluène pendant 2 heures, on obtient 8,4 g de bromure de 1-(4-trifluorométhylbenzyl)-3-méthyl-imidazolium, sous forme d'un solide jaune-orangé, dont les caractéristiques sont les suivantes :
- Spectre de RMN¹H (DMSO): δ 9.33 ppm (s, 1H), 7.88 (t, 1H) , 7.78 (t, 1H), 7.80 (d, 2H), 7.68 (d, 2H), 5.60 (s, 2H), 3.90 (s, 3H)

### Etape 2 : [1-méthyl-3-(4-trifluorométhylbenzyl)imidazol-2-ylidène](1,3-divinyl-1,1,3,3-tétraméthyldisiloxane)platine (0).

En opérant comme à l'étape 2 de l'exemple 10, mais à partir de 0.401 g du composé obtenu à l'étape1 et 12.5 mL (1,25 mmol) d'une solution 0,1 M de (1,3-divinyl-1,1,3,3-tétraméthyldisiloxane) platine (0) dans le xylène et 1,75 mL (1,75 mmol) d'une solution 1 M de *tert*-butylate de potassium dans le tétrahydrofurane, on obtient, après purification par flash-chromatographie en éluant avec un mélange de cyclohexane et d'acétate d'éthyle (97/3 en volumes), 0,4 g (58%) de [1-méthyl-3-(4-trifluorométhylbenzyl)imidazol-2-ylidène](1,3-d ivinyl-1,1,3,3-tétraméthyl-disiloxane) platine (0), sous forme d'une meringue jaune-pâle, dont les caractéristiques sont les suivantes :
- Spectre de RMN¹H (DMSO): δ 7.70 (d, 2H), 7.50 (m, 2H), 7.25 (m, 2H), 5.25 (m, 2H), 3.50 (s, 3H), 2.30-1.60 (m, 6H), 0.20 (m, 6H), - 0.35 (m, 6H).

### Etape 3: trans-diiodo(N-cyclohexylamine)[1-méthyl-3-(4-trifluorométhylbenzyl) imidazol-2-ylidène]platine (II).

En opérant comme à l'étape 3 de l'exemple 11, mais à partir de 0,230 g du composé obtenu à l'étape 2, dans 30 mL de toluène, 0,094 g (0,394 mmol) de diiode dans 10 mL de toluène et 42,4 µL (0,394 mmol) de cyclohexylamine, à température ambiante pendant 4 h, on obtient, après purification par flash-chromatographie en éluant avec un mélange de cyclohexane et d'acétate d'éthyle (85/15 en volumes), 40 mg (14%) de *trans*-diiodo(*N*-cyclohexylamine)[1-méthyl-3-(4-trifluorométhylbenzyl)imidazol-2-ylidène]platine (II), sous forme d'une poudre jaune, dont les caractéristiques sont les suivantes :
- Spectre de RMN¹H (CDCl₃): δ 7.69 (d, 2H), 7.62 (d, 2H), 6.88 (d, 1H), 6.65 (d, 1H), 5.73 (s, 2H), 3.98 (s, 3H), 3.30 (m, 1H), 2.98 (d, 2H), 2.32 (d, 2H), 1.82 (d, 2H), 1.78 (d, 1H), 1.38 (q, 2H), 1.26 (q, 2H), 1.18 (d, 1H).

### Exemple 12 : trans-diiodo(N-cyclohexylamine)[1,3-dibenzyl-imidazol-2-ylidène]platine (II)

### Etape 1 : [1,3-dibenzyl-imidazol-2-ylidène](1,3-divinyl-1,1,3,3-tétraméthyl-disiloxane)platine (0).

En opérant comme à l'étape 2 de l'exemple 10, mais à partir de 0.433 g (1,25 mmol.) de bromure de 1,3-dibenzyl-imidazolium - qui a été obtenu selon E Diaz-Barra et coll., Organometallics (2007), 26(24), 5782-85- 12.5 mL (1,25 mmol) d'une solution 0,1 M de (1,3-divinyl-1,1,3,3-tétraméthyldisiloxane) platine (0) dans le xylène et 1,75 mL (1,75 mmol) d'une solution 1 M de *tert*-butylate de potassium dans le tétrahydrofurane, on obtient, après purification par flash-chromatographie en éluant avec un mélange de cyclohexane et d'acétate d'éthyle (98/2 en volumes), 0,72 g (86%) de [1,3-dibenzyl-imidazol-2-ylidène](1,3-divinyl-1,1,3,3-tétraméthyl-disiloxane)platine (0), sous forme d'une meringue jaune-pâle, dont les caractéristiques sont les suivantes :
- Rf = 0,42 (gel de silice, mélange de 60% de cyclohexane et de 40% d'acétate d'éthyle).
- Spectre de RMN¹H (DMSO): δ 7.55 (m, 1H), 7.40 (m, 1H), 7.58-7.20 (m, 10H), 4.98 (d, 4H), 2.20-1.55 (m, 6H), 0.20 (s, 6H), - 0.50 (s, 6H).

### Etape 2 : trans-diiodo(N-cyclohexylamine)[1,3-dibenzyl-imidazol-2-ylidène]platine (II)

En opérant comme à l'étape 3 de l'exemple 11, mais à partir de 0,325 g (0,516 mmol) du composé obtenu à l'étape 1, dans 45 mL de toluène, 0,131 g (0,516 mmol) de diiode dans 10 mL de toluène et 51,2 µL (0,516 mmol) de cyclohexylamine, à température ambiante pendant 7 h, on obtient, après purification par flash-chromatographie en éluant avec un gradient de mélanges de cyclohexane et d'acétate d'éthyle (de 97/3 à 95/5 en volumes), puis cristallisation dans 5 mL d'oxyde de di*iso*propyle, 90 mg (22%) de *trans*-diiodo(*N*-cyclohexylamine)[1,3-dibenzyl-imidazol-2-ylidène]platine (II), sous forme d'une poudre orangée, dont les caractéristiques sont les suivantes :
- Rf = 0,14 (gel de silice, mélange de 95% de cyclohexane et de 5% d'acétate d'éthyle)
- Spectre de RMN¹H (CDCl₃): δ 7.48 (d, 4H), 7.38 (m, 6H), 6.53 (s, 2H) , 5.62 (s, 4H), 3.28 (m, 1H), 2.98 (m, 2H), 2.28 (d, 2H), 1.75 (d, 2H), 1.62 (d, 1H), 1.32 (q, 2H), 1.20 (q, 2H), 1.12 (d, 1H)

### Exemple 13 : trans-diiodo(N-benzylamine)(1,3-diméthyl-imidazol-2-ylidène) platine (II)

Dans un tricol de 100 mL sous argon, on dissout 0,2 g (0,42 mmol) de (1,3-diméthyl-imidazol-2-ylidène)(1,3-divinyl-1,1,3,3-tétraméthyldisiloxane)platine (0) - qui a été obtenu selon G Berthon-Gelloz et coll., Journal of Organometallic Chemistry (2005), 690(24-25), 6156-68 - dans 40 mL de toluène puis on refroidit à 0°C. On ajoute alors, goutte à goutte à 0°C, successivement une solution de 0,106 g (0,42 mmol) de diiode dans 10 mL de toluène puis 45,8 µL (0,42 mmol) de benzylamine. Le milieu réactionnel est ensuite agité à température ambiante pendant 5 h. Le milieu réactionnel est évaporé à sec. Le résidu ainsi obtenu est purifié par flash-chromatographie en éluant avec un gradient de mélanges de cyclohexane et d'acétate d'éthyle (de 95/5 à 75/25 en volumes). Après cristallisation dans 1 mL d'oxyde de disisopropyle, on obtient 60 mg (22%) de *trans*-diiodo(*N*-benzylamine)(1,3-diméthyl-imidazol-2-ylidène)platine (II), sous forme d'une poudre jaune, dont les caractéristiques sont les suivantes :
- Rf = 0,11 (gel de silice ; mélange de 90% de cyclohexane et 10% d'acétate d'éthyle)
- Spectre de RMN¹H (CDCl₃, 20°), [δ ppm] : 3,24 (m étalé, 2 H), 3,87 (s, 6 H), 4,12 (m, 2 H), 6,81 (s, 2 H), 7,30 - 7,43 (m, 5 H).

### Exemple 14 : trans-diiodo[N-4-(trifluorométhyl)benzylaminel(1,3-diméthyl-imidazol-2-ylidène)platine (II)

On opère comme à l'exemple 13, mais à partir de 0,2 g (0,42 mmol) de (1,3-diméthyl-imidazol-2-ylidène)(1,3-divinyl-1,1,3,3-tétraméthyldisiloxane)platine (0) - qui a été obtenu selon G Berthon-Gelloz et coll., Journal of Organometallic Chemistry (2005), 690(24-25), 6156-68 - une solution de 0,106 g (0,42 mmol) de diiode dans 10 mL de toluène et 59,7 µL (0,42 mmol) de 4-(trifluorométhyl)benzylamine dans 40 mL de toluène, pendant 6 h. Après purification par flash-chromatographie en éluant avec un gradient de mélanges de cyclohexane et d'acétate d'éthyle (de 95/5 à 90/10 en volumes), puis cristallisation dans 2 mL d'oxyde de disisopropyle, on obtient ainsi 201 mg (67%) de *trans*-diiodo[*N*-4-(trifluorométhyl)benzylamine](1,3-diméthyl-imidazol-2-ylidène)platine (II), sous forme de cristaux jaunes, dont les caractéristiques sont les suivantes :
- Rf = 0,14 (gel de silice ; mélange de 90% de cyclohexane et 10% d'acétate d'éthyle)
- Spectre de RMN¹H (CDCl₃, 20°), [δ ppm] : .2,9 (m, 2 H), 3,85 (s, 6 H), 4,23 (m, 2 H), 6,81 (s, 2 H), 7,53 (d, *J*=8.1 Hz, 2 H), 7,66 (d, *J*=8.1 Hz, 2 H).

### Exemple 15 : trans-diiodo(N-cyclohexylamine)[1-méthyl-3-vinyl-imidazol-2-ylidène]platine (II)

### Etape 1 : [1-méthyl-3-vinyl-imidazol-2-ylidène](1,3-divinyl-1,1,3,3-tétraméthyl-disiloxane)platine (0).

En opérant comme à l'étape 2 de l'exemple 10, mais à partir de 0.226 g (1,55 mmol.) de chlorure de 1-méthyl-3-(2-chloroéthyl)-imidazolium - qui a été obtenu selon S. Letaief et coll., Journal of Materials Chemistry (2007), 17(15), 1476-84 - 12.5 mL (1,25 mmol) d'une solution 0,1 M de (1,3-divinyl-1,1,3,3-tétraméthyldisiloxane)platine (0) dans le xylène et 2,5 mL (2,5 mmol) d'une solution 1 M de *tert*-butylate de potassium dans le tétrahydrofurane, pendant 8h. On obtient, après purification par flash-chromatographie en éluant avec un mélange de cyclohexane et d'acétate d'éthyle (97/3 en volumes), 0,414 g (54%) de (1-méthyl-3-vinyl-imidazol-2-ylidène)(1,3-divinyl-1,1,3,3-tétraméthyl-disiloxane)platine (0), sous forme d'une meringue jaune-pâle, dont les caractéristiques sont les suivantes :
- Rf = 0,15 (gel de silice, mélange de 95% de cyclohexane et de 5% d'acétate d'éthyle).

### Etape 2: trans-diiodo(N-cyclohexylamine)(1-méthyl-3-vinyl-imidazol-2-ylidène) platine (II)

En opérant comme à l'étape 3 de l'exemple 11, mais à partir de 0,236 g (0,48 mmol) du composé obtenu à l'étape 1, dans 45 mL de toluène, 0,122 g (0,0,48 mmol) de diiode dans 10 mL de toluène et 55 µL (0,47 mmol) de cyclohexylamine, à température ambiante pendant 6 h, on obtient, après purification par flash-chromatographie en éluant avec un mélange de cyclohexane et d'acétate d'éthyle (de 85/15 en volumes), puis cristallisation dans 2 mL d'oxyde de di*iso*propyle, 161 mg (51%) de *trans*-diiodo(*N*-cyclohexylamine)(1-méthyl-3-vinyl-imidazol-2-ylidène)platine (II), sous forme d'une poudre orangée, dont les caractéristiques sont les suivantes :
- Rf = 0,13 (gel de silice, mélange de 85% de cyclohexane et de 15% d'acétate d'éthyle)
- Spectre de RMN¹H (CDCl₃, 20°), [δ ppm] : 1,08 - 1,43 (m, 5 H), 1,66 (m, 1 H), 1,80 (m, 2 H), 2,29 (m, 2 H), 2,96 (m étalé, 2 H), 3,27 (m, 1 H), 3,91 (s, 3 H), 5,04 (dd, *J*=8.9, 2.1 Hz, 1 H), 5,24 (dd, *J*=15.9, 2.1 Hz, 1 H), 6,87 (d, J=2.4 Hz, 1 H), 7,15 (d, J=2.4 Hz, 1 H), 7,93 (dd, *J*=15.9, 8.9 Hz, 1 H).

### Exemples 16 et 17 : trans-diiodo(N-exo(+)bicylo[2.2.1]hentylamine)(1,3-diméthyl-benzimidazol-2-ylidène)platine (II) et trans-diiodo(N-exo(-)bicyclo[2.2.1]heptyl-amine)(1.3-diméthyl-benzimidazol-2-ylidène)platine (II)

77 mg de *trans*-diiodo(*N-exo*(+/-)bicyclo[2.2.1]heptylamine)(1,3-diméthyl-benzimidazol-2-ylidène)platine (II), obtenus comme à l'exemple 7, sont séparés par CLHP chirale sur une colonne de 350 mm de longueur et 50 mm de diamètre remplie de silice Chiracel OJ FB001 20µM, en éluant avec un mélange de n-heptane, d'ispropanol et de méthanol (80/10/10 en volumes) à un débit de 90 mL/mn. La séparation est suivie par détection UV à 254 nM.

En récupérant la première fraction éluée, on obtient, après concentration à sec sous pression réduite, 37,1 mg de *trans*-diiodo(*N-exo*(-)bicyclo[2.2.1]heptyl-amine)(1,3-diméthyl-benzimidazol-2-ylidène)platine (II), sous forme d'une poudre jaune, dont les caractéristiques sont les suivantes :
- Pouvoir rotatoire α_{D}²⁰ = -4 +/- 0,2 ° (c = 0.916, DMSO)
- Temps de rétention = 11,8 mn (Chiracel OJ 20µM ; 250×4,6 mm ; heptane /méthanol / isopropanol 70/15/15 à un débit de 1 mL/mn).

En récupérant la seconde fraction éluée, on obtient, après concentration à sec sous pression réduite, 32,6 mg de *trans*-düodo(*N-exo*(+)bicyclo[2.2.1]heptyl-amine)(1,3-diméthyl-benzimidazol-2-ylidène)platine (II), sous forme d'une poudre jaune, dont les caractéristiques sont les suivantes :
- Pouvoir rotatoire α_{D}²⁰ = +4,2 +/- 0,2 ° (c = 0.931, DMSO)
- Temps de rétention = 13,6 mn (Chiracel OJ 20µM ; 250x4,6 mm ; heptane /méthanol / isopropanol 70/15/15 à un débit de 1 mL/mn).

### Exemple 18 : trans-diiodo(N-cyclohexylamine)(1,3-dicyclohexylimidazol-2-ylidène) platine(II)

A une solution de complexe (1,3-dicyclohexylimidazol-2-ylidène)(1,3-divinyl-1,1,3,3-tetraméthyldisiloxane)] platine(0) (0,2 g ; 0,32 mmol) - qui a été obtenu selon I. E. Marko et coll., Advanced Synthesis and Catalysis (2004), 346(12), 1429-34 - dans 15 mL de toluène à 0°C est additionnée une solution de diiode (0,09 g ; 0,35 mmol) dans 30 mL de toluène. Par la suite 40 µL de cyclohexylamine (0,35 mmol) sont additionnés au mélange. Le mélange réactionnel est agité pendant 16h à température ambiante puis évaporé à sec. Le résidu obtenu est chromatographié sur silice par élution avec un mélange d'heptane et d'acétate d'éthyle (80/20 en volumes). Les fractions contenant le produit attendu sont rechromatographiées sur colonne de silice en éluant avec un mélange d'heptane et de toluène (40/60 en volumes), on recueille ainsi 0,12 g qui sont recristallisés dans un mélange de dichlorométhane et d'heptane pour donner 0,1 g (40 %) de *trans*-diiodo(*N*-cyclohexylamine)(1,3-dicyclohexylimidazol-2-ylidène) platine(II), sous forme de cristaux jaune-beiges dont les caractéristiques sont les suivantes :
- spectre de RMN ¹H (500 MHz, CDCl₃, 20°C) δ [ppm] = 1.1-1.5 (15H), 1.63 (m, 1H), 1.7-1.8 (4H), 1.88 (m, 4H), 2.31 (m, 6H), 2.92 (br, 2H), 3.28 (m, 1H), 5.20 (m, 2H), 6.82 (s, 2H).
- spectre de masse HRMS (ESI mode positif) calculée pour C₂₁H₃₇I₂N₃Pt.Na : 803.0622 ; trouvée pour C₂₁H₃₇I₂N₃NaPt : 803.0658

### Exemple 19 : trans-diiodo(N-cyclohexylamine)(1-méthyl-3-phényl-1,2,4-triazol-5-ylidène) platine(II)

### Etape 1 : iodure de 1-phényl-4-méthyl-1,2,4-triazolium

A une solution du 1-phényl-1,2,4-triazol - qui a été obtenu selon Antilla, J. C. et al., Journal of Organic Chemistry 2004, 69, 5578-5587 - (0,15 g ; 1 mmol) dans 1 mL d'acétonitrile on ajoute l'iodure de méthyle (0,13 mL ; 2 mmol). Ce mélange est chauffé à 80°C pendant 16 heures, puis est évaporé à sec. Le résidu obtenu est recristallisé dans un minimum d'acétate d'éthyle, permettant ainsi d'obtenir 0.12 g du produit désiré (41%) dont les caractéristiques sont les suivantes :
- spectre de RMN ¹H (500.19 MHz, CDCl₃, 20°C) δ [ppm] = 4.34 (s, 3H), 7.54 (m, 3H), 7.96 (d, 2H), 9.09 (s, 1H), 11.60 (s, 1H).

### Etape 2 : [1-phényl-4-méthyl-1,2,4-triazot-5-ylidène (1,3-divinyl-1,1,3,3-tetraméthyldisiloxane)] platine(0)

A une solution d'iodure de 1-phényl-4-méthyl-1,2,4-triazolium (0,12 g ; 0,42 mmol) dans 20 mL de dichlorométhane est ajouté, à 0°C, du (1,3-divinyl-1,1,3,3-tetraméthyldisiloxane) platine(0) 0.1 M dans le xylène (4,2 mL ; 0,42 mmol) et du *tert-*butylate de potassium (0,07 g ; 0,63 mmol). Le mélange réactionnel est agité pendant 16h à température ambiante puis évaporé à sec. Le résidu obtenu est purifié par chromatographie flash en éluant avec un mélange d'heptane et d'acétate d'éthyle (80 /20 en volumes), on recueille ainsi 0.07 g du produit désiré (31 %), dont les caractéristiques sont les suivantes :
- spectre de RMN ¹H (300 MHz, CDCl₃, 20°C) δ [ppm] = -0.34 (s, 6H), 0.31 (s, 6H), 1.8-2.1 (m, 4H), 2.25 (d, 2H), 3.65 (s, 3H), 7.3 (m, 3H), 7.92-7.96 (m, 2H), 8.19 (s, 1H).

### Etape 3 : trans-diiodo(N-cyclohexylamine)(1-phényl-4-méthyl-1,2,4-triazol-5-ylidène) platine(II)

A une solution de complexe [1-phényl-4-méthyl-1,2,4-triazol-5-ylidène (1,3-divinyl-1,1,3,3-tetraméthyldisiloxane)] platine(0) (0,07 g ; 0,13 mmol) dans 6 mL de toluène à 0°C est additionnée une solution de I₂ (0,04 g ; 0,14 mmol) dans 12 mL de toluène. Par la suite 22 µL de cyclohexylamine, (0,19 mmol) sont additionnés au mélange. Le mélange réactionnel est agité pendant 16h à température ambiante puis évaporé à sec. Le résidu obtenu est purifié par chromatographie flash par élution avec un mélange d'heptane et d'acétate d'éthyle (85/15 en volumes). On recueille ainsi 0.02 g du produit désiré (22 %), dont les caractéristiques sont les suivantes :
- spectre de RMN ¹H (500 MHz, CDCl₃, 20°C) δ [ppm] = 1.1-1.5 (5H), 1.62 (m, 1H), 1.74 (m, 2H), 2.19 (m, 2H), 2.97 (br, 2H), 3.17 (m, 1H), 3.99 (s, 6H, CH₃N), 7.3-7.5 (3H), 8.03 (s, 1H), 8.19 (m, 2H).
- spectre de masse HRMS (ESI mode positif) calculée pour C₁₅H₂₂I₂N₄Pt.Na : 729.9479 ; trouvée pour C₁₅H₂₂I₂N₄NaPt : 729.9509

### Exemple 20 : trans-diiodo(N-cyclohexylamine)[1,3-diméthyl-4-diményl-imidazol-2-ylidène]platine (II)

### Etape 1 : [1,3-diméthyl-4-phényl-imidazol-2-ylidène](1,3-divinyl-1,1,3,3-tétraméthyl-disiloxane)platine (0).

En opérant comme à l'étape 2 de l'exemple 10, mais à partir de 0.375 g (1,25 mmol.) d'iodure de 1,3-diméthyl-4-phényl-imidazolium - qui a été obtenu selon M.R. Grimmett, Science of Synthesis (2002), 12, 325-528 - 12.5 mL (1,25 mmol) d'une solution 0,1 M de (1,3-divinyl-1,1,3,3-tétraméthyldisiloxane) platine (0) dans le xylène et 1,75 mL (1,75 mmol) d'une solution 1 M de *tert*-butylate de potassium dans le tétrahydrofurane, on obtient, après purification par flash-chromatographie en éluant avec un mélange de cyclohexane et d'acétate d'éthyle (98/2 en volumes), 0,595 g (86%) de [1,3-diméthyl-4-phényl-imidazol-2-ylidène](1,3-divinyl-1,1,3,3.tétraméthyl-disiloxane)platine (0), sous forme d'un solide blanc, dont les caractéristiques sont les suivantes :
- Rf = 0,28 (gel de silice, mélange de 90% de cyclohexane et de 10% d'acétate d'éthyle).
- Spectre de RMN¹H (DMSO): δ 7.60-7.40 (m, 6H), 3.50 (s, 6H), 2.20 (m, 2H), 1.90 (m, 2H), 1.70 (m, 2H), 0.25 (s, 6H), - 0.30 (s, 6H).

### Etape 2 : trans-diiodo(N-cyclohexylamine)[1,3-diméthyl-4-phényl-imidazol-2-ylidène]platine (II)

En opérant comme à l'étape 3 de l'exemple 11, mais à partir de 0,3 g (0,542 mmol) du composé obtenu à l'étape 1, dans 40 mL de toluène, 0,138 g (0,542 mmol) de diiode dans 10 mL de toluène et 62 µL (0,5426 mmol) de cyclohexylamine, à température ambiante pendant 7 h, on obtient, après purification par flash-chromatographie en éluant avec un mélange de cyclohexane et d'acétate d'éthyle (90/10 en volumes), puis cristallisation dans 2 mL d'oxyde de di*iso*propyle, 275 mg (70%) de *trans*-diiodo(*N*-cyclohexylamine)[1,3-diméthyl-4-phényl-imidazol-2-ylidène]platine (II), sous forme de cristaux jaunes, dont les caractéristiques sont les suivantes :
- Rf = 0,18 gel de silice, mélange de 90% de cyclohexane et de 10% d'acétate d'éthyle).
- Spectre de RMN¹H (CDCl₃): δ 7.48 (m, 3H), 7.38 (m, 2H), 6.85 (s, 1H), 5.30 (dd, 1H), 3.98 (s, 3H), 3.90 (s, 3H), 3.33 (m, 1H), 2.98 (m, 2H), 2.85 (d, 2H), 1.85 (d, 2H), 1.70 (d, 1H), 1.40 (q, 2H), 1.28 (q, 2H), 1.18 (d, 1H)

### Exemple 21 : trans-diiodo(N-cyclohexylamine)[3-benzyl-1-méthyl-4-phényl-imidazol-2-ylidène]platine (II)

*Etape 1 :* Dans un ballon monocol de 25 mL, on dissout 0,320 g (2,023 mmole) de 1-méthyl-4-phényl-imidazole, qui a été obtenu selon P. Benjes et coll., Heterocycles (1994), 37(2), 735-38, dans 10 mL de toluène puis on ajoute 0,36 mL (3,035 mmole) de bromure de benzyle. Après 5 jours de chauffage au reflux, le milieu réactionnel est concentré sous pression réduite. Le résidu est repris par 50 mL d'oxyde de diisopropyle. Le précipité formé est essoré, lavé deux fois avec 5 mL d'oxyde de diisopropyle, ouis séché sous pression réduite. On obtient ainsi 0,500 g (90%) de bromure de 3-benzyl-1-méthyl-4-phényl-imidazolium, sous forme d'un solide gris-beige, dont les caractéristiques sont les suivantes :
   - Spectre de RMN¹H (DMSO): δ 9.32 (s, 1H), 7.97 (d, 1H), 7.50 (m, 5H), 7.30 (m, 3H), 7.08 (m, 2H), 5.48 (s, 2H), 3.93 (s, 3H)
*Etape* 2 : [3-benzyl-1-méthyl-4-phényl-imidazol-2-ylidène](1,3-divinyl-1,1,3,3-tétraméthyl-disiloxane)platine (0).
   En opérant comme à l'étape 2 de l'exemple 10, mais à partir de 0.439 g (1,33 mmol.) de bromure de 3-benzyl-1-méthyl-4-phényl-imidazolium, obtenu à l'étape 1, 13,3 mL (1,33 mmol) d'une solution 0,1 M de (1,3-divinyl-1,1,3,3-tétraméthyldisiloxane) platine (0) dans le xylène et 1,87 mL (1,87 mmol) d'une solution 1M de *tert*-butylate de potassium dans le tétrahydrofurane, on obtient, après purification par flash-chromatographie en éluant avec un mélange de cyclohexane et d'acétate d'éthyle (98/2 en volumes), 0,485 g (70%) de [3-benzyl-1-méthyt-4-phényl-imidazol-2-ylidène](1,3-divinyl-1,1,3,3-tétraméthyl-disiloxane)platine (0), sous forme d'un solide blanc, dont les caractéristiques sont les suivantes :
   - Rf = 0,43 (gel de silice, mélange de 90% de cyclohexane et de 10% d'acétate d'éthyle).
   - Spectre de RMN¹H (DMSO): δ 7.63 (t, 1H). 7.35 (m, 5H), 7.15 (m, 3H), 6.80 (m, 2H). 5.35 (m, 2H), 3.58 (s, 3H), 2.20-1.50 (m, 6H), 0.20 (m, 6H), - 0.30 (m, 3H), - 0.62 (m, 3H)
*Etape* 3: *trans*-diiodo(*N*-cyclohexylamine)[3-benzyl-1-méthyl-4-phényl-imidazol-2-ylidène]platine (II)
   En opérant comme à l'étape 3 de l'exemple 11, mais à partir de 0,3 g (0,476 mmol) du composé obtenu à l'étape 2, dans 40 mL de toluène, 0,141 g (0,556 mmol) de diiode dans 10 mL de toluène et 54,5 µL (0,476 mmol) de cyclohexylamine, à température ambiante pendant 48 h, on obtient, après purification par flash-chromatographie en éluant avec un gradient de mélanges de cyclohexane et d'acétate d'éthyle (de 95/ à 90/10 en volumes), puis cristallisation dans 2 mL d'oxyde de di*iso*propyle, 83 mg (22%) de *trans*-diiodo(*N*-cyclohexylamine)[3-benzyl-1-méthyl-4-phényl-imidazol-2-ylidène]platine (II), sous forme de cristaux jaunes, dont les caractéristiques sont les suivantes :
   - Rf = 0,21 (gel de silice, mélange de 90% de cyclohexane et de 10% d'acétate d'éthyle).
   - Spectre de RMN¹H (CDCl₃): δ 7.30 (m, 5H), 7.20 (m, 3H), 7.15 (m, 2H), 5.73 (s, 2H), 4.00 (s, 3H), 3.25 (m, 1H), 2.98 (m, 2H), 2.25 (d, 2H), 1.78 (d, 2H), 1.65 (d, 1H,), 1.35 (q, 2H), 1.20 (m, 2H), 1.18 (d, 1H).

### Exemple 22 : trans-diiodo(N-cyclohexylamine)(1,3-diméthyl-4,5-diphényl-imidazol-2-ylidène]platine (II)

*Etape 1 :* Dans un ballon monocol de 25 mL, on dissout 0,240 g (1,024 mmole) de 1-méthyl-4-phényl-imidazole, qui a été obtenu selon W. Collibee et coll., Tetrahedron Letters (1985), 62(6), 1595-96, dans 7 mL de toluène puis on ajoute 100 µL d'iodure de méthyle. Après 15 jours de chauffage au reflux en rajoutant 100 µL d'iodure de méthyle chaque jour, le milieu réactionnel est concentré sous pression réduite. Le résidu est repris par 50 mL d'oxyde de diisopropyle. Le précipité formé est essoré, lavé deux fois avec 5 mL d'oxyde de diisopropyle, ouis séché sous pression réduite. On obtient ainsi 0,370 g (98%) d'iodure de 1,3-diméthyl-4,5-diphényl-imidazolium, sous forme d'un solide jaune pâle, dont les caractéristiques sont les suivantes :
   - Spectre de RMN¹H (DMSO): δ 9.30 (s, 1H), 7.48 (m, 6H), 7.40 (m, 4H), 3.75 (s, 6H)
*Etape* 2: [3-benzyl-1-méthyl-4-phényl-imidazol-2-ylidène](1,3-divinyl-1,1,3,3-tétraméthyl-disiloxane)platine (0).
   En opérant comme à l'étape 2 de l'exemple 10, mais à partir de 0.210 g (0,53 mmol.) d'iodure 1,3-diméthyl-4,5-diphényl-imidazolium, obtenu à l'étape 1, 5,3 mL (0,53 mmol) d'une solution 0,1 M de (1,3-divinyl-1,1,3,3-tétraméthyldisiloxane) platine (0) dans le xylène et 0,74 mL (0,74 mmol) d'une solution 1 M de *tert*-butylate de potassium dans le tétrahydrofurane, on obtient, après purification par flash-chromatographie en éluant avec un mélange de cyclohexane et d'acétate d'éthyle (95/5 en volumes), 0,191 g (57%) de [1,3-diméthyl-4,5-diiphényl-imidazol-2-ylidène](1,3-divinyl-1,1,3,3-tétraméthyl-disiloxane)platine (0), sous forme d'un solide blanc, dont les caractéristiques sont les suivantes :
   - Rf = 0,45 (gel de silice, mélange de 90% de cyclohexane et de 10% d'acétate d'éthyle).
   - Spectre de RMN¹H (DMSO): δ 7.38 (m, 10H), 3.42 (s, 6H), 2.28 (m, 2H), 1.97 (m, 2H), 1.75 (m, 2H), 0.25 (s, 6H), - 0.30 (s, 6H)
*Etape* 3: *trans*-diiodo(*N*-cyclohexylamine)[1,3-diméthyl-4,5-diphényl-imidazol-2-ylidène]platine (II)
   En opérant comme à l'étape 3 de l'exemple 11, mais à partir de 0,19 g (0,30 mmol) du composé obtenu à l'étape 2, dans 40 mL de toluène, 0,077 g (0,30 mmol) de diiode dans 5 mL de toluène et 34,5 µL (0,30 mmol) de cyclohexylamine, à température ambiante pendant 5 h, on obtient, après purification par flash-chromatographie en éluant avec un gradient de mélanges de cyclohexane et d'acétate d'éthyle (de 95/ à 90/10 en volumes), puis cristallisation dans 2 mL d'oxyde de di*iso*propyle, 76 mg (32%) de *trans-*diiodo(*N*-cyclohexylamine)[1,3-diméthyl-4,5-diphényl-imidazol-2-ylidène]platine (II), sous forme d'une poudre jaune, dont les caractéristiques sont les suivantes :
   - Rf = 0,24 (gel de silice, mélange de 90% de cyclohexane et de 10% d'acétate d'éthyle).
   - Spectre de RMN¹H (CDCl₃): δ 7.38 (m, 6H), 7.22 (m, 4H), 3.35 (s, 6H), 3.35 (m, 1H), 2.98 (m, 2H), 2.38 (d, 2H), 1.85 (d, 2H), 1.70 (d, 1H), 1.40 (q, 2H), 1.30 (q, 2H), 1.20 (d, 1H)

### Exemples 23 et 24 : trans-diiodo(N-exo(+)bicyclo[2.2.1]heptylamine)(1,3-diméthyl-imidazot-2-vlidène)platine (II) et trans-diiodo(N-exo(-)bicyclo[2.2.1]heptyl-amine)(1,3-diméthyl-imidazol-2-ylidène)platine (II)

75 mg de *trans*-diiodo(*N*-exo(+/-)bicyclo[2.2.1]heptylamine)(1,3-diméthyl-imidazol-2-ylidène)platine (II), obtenus comme à l'exemple 6, sont séparés par CLHP chirale, en 3 injections successives de 25 mg, sur une colonne de 250 mm de longueur et 20 mm de diamètre remplie de silice Chiracel OJ FB001 10µM, en éluant avec un mélange de n-heptane, d'ispropanol et de méthanol (80/10/10 en volumes) à un débit de 25 mL/mn. La séparation est suivie par détection UV à 254 nM.

En récupérant les premières fractions éluées, on obtient, après concentration à sec sous pression réduite, 22,4 mg de *trans*-diiodo(*N-exo*(-)bicyclo[2.2.1]heptyl-amine)(1,3-diméthyl-imidazol-2-ylidène)platine (II), énantiomériquement pur, sous forme d'une poudre jaune, dont les caractéristiques sont les suivantes :
- Pouvoir rotatoire α_{D}²⁰ =-3,6 +/- 0,5° (c = 0.437, DMSO)
- Temps de rétention = 19,1 mn (Chiracel OJ 20µM; 250x4,6 mm ; heptane /méthanol / isopropanol 80/10/10 à un débit de 1 mL/mn).

En récupérant les secondes fractions éluées, on obtient, après concentration à sec sous pression réduite, 35,6 mg de *trans*-diiodo(*N-exo*(+)bicyclo[2.2.1]heptyl-amine)(1,3-diméthyl-imidazol-2-ylidène)platine (II), à 98,7% de pureté énantiomérique, sous forme d'une poudre jaune, dont les caractéristiques sont les suivantes :
- Pouvoir rotatoire α_{D}²⁰ = +4,6 +/- 0,5 °(c = 0.461, DMSO)
- Temps de rétention = 20,9 mn (Chiracel OJ 20µM; 250×4,6 mm ; heptane /méthanol / isopropanol 80/10/10 à un débit de 1 mL/mn).

### Exemple 25 : trans-diiodo(N-exo(+/-)bicyclo[2.2.1]heptylamine) (1,3,7,9-tétraméthylxanthine-8-ylidène) platine(II)

A une solution de complexe [1,3,7,9-tétraméthylxanthine-8-ylidène (1,3-divinyl-1,1,3,3-tetraméthyldisiloxane)] platine(0) , obtenu à l'étape 2 de l'exemple 9, (0.06 g, 0.1 mmol) dans 5 mL de toluène à 0°C est ajoutée une solution de I₂ (0.03 g, 0.1 mmol) dans 8 mL de toluène. Par la suite 26 µL de *exo*-2-norbornanamine (pour un total de 0.22 mmol, en deux additions successives) sont additionnés au mélange. Le mélange réactionnel est agité pendant 16h à température ambiante puis évaporé à sec. Le résidu obtenu est purifié par chromatographie flash par élution avec un mélange d'heptane et d'acétate d'éthyle (70/30 en volumes). On recueille ainsi 0.022 g du produit désiré (29 %), dont les caractéristiques sont les suivantes :
RMN ¹H (300 MHz, CDCl₃, 20°C) δ [ppm] = 1.1-1.9 (8H), 2.35 (m, 1H), 2.42 (m, 1H), 3.0 (br, 2H), 3.38 (s, 3H), 3.5 (br, 1H), 3.78 (s, 3H), 4.20 (s, 3H), 4.32 (s, 3H).
HRMS (ESI mode négatif) calculée pour C₁₆H₂₅I₂N₅O₂Pt : 767.9745; trouvée pour C₁₆H₂₄I₂N₅O₂Pt : 766.9705.

### Exemple 26 : trans-diiodo(N-cyclohexylamine)[3-benzyl-4,5-diphényl-1-méthyl-imidazol-2-ylidène]platine (II)

### Etape 1 : bromure de 3-benzyl-4,5-diphényl-1-méthyl-imidazolium

Dans un ballon monocol de 25 mL, on dissout 0,240 g (1,024 mmol) de 4,5-diphényl-1-méthyl-imidazole, qui a été obtenu selon Zeitschrift fuer Naturforschung, B : Chemical Sciences (2003), 58(4), 305-310, dans 10 mL de toluène puis on ajoute 183 µL (1,536 mmol) de bromure de benzyle. Après 2 jours de chauffage au reflux, le milieu réactionnel est concentré sous pression réduite. Le résidu est repris par 50 mL d'oxyde de diisopropyle. Le précipité formé est essoré, lavé deux fois avec 5 mL d'oxyde de diisopropyle, puis séché sous pression réduite. On obtient ainsi 0,480 g (100%) de bromure de 3-benzyl-4,5-diphényl-1-méthyl-imidazolium, sous forme d'un solide jaune, utilisé tel quel à l'étape suivante, dont la caractéristique est la suivante :
- Rf = 0,18 (gel de silice, mélange de 90% de dichlorométhane et de 10% de méthanol).

### Etape 2: [3-benzyl-4,5-diphényl-1-méthyl-imidazol-2-ylidène](1,3-divinyl-1,1,3,3-tétraméthyl-disiloxane)platine (0).

En opérant comme à l'étape 2 de l'exemple 10, mais à partir de 0.3 g (0,74 mmol) de bromure de 3-benzyl-1-méthyl-4,5-diphényl-imidazolium, obtenu à l'étape 1, 7,4 mL (0,74 mmol) d'une solution 0,1 M de (1,3-divinyl-1,1,3,3-tétraméthyldisiloxane) platine (0) dans le xylène et 1,04 mL (1,04 mmol) d'une solution 1M de *tert*-butylate de potassium dans le tétrahydrofurane, on obtient, après purification par flash-chromatographie en éluant des mélanges de cyclohexane et d'acétate d'éthyle (99/1 puis 98/2 en volumes), 0,211 g (40%) de [3-benzyl-4,5-diphényl-1-méthyl-imidazol-2-ylidène](1,3-divinyl-1,1,3,3-tétraméthyl-disiloxane)platine (0), sous forme d'un solide blanc, dont la caractéristique est la suivante :
- Rf = 0,46 (gel de silice, mélange de 90% de cyclohexane et de 10% d'acétate d'éthyle).

### Etape 3: trans-diiodo(N-cyclohexylamine)[3-benzyl-4,5-diphényl-1-méthyl-imidazol-2-ylidène]platine (II)

En opérant comme à l'étape 3 de l'exemple 11, mais à partir de 0,1 g (0,181 mmol) du composé obtenu à l'étape 2, dans 20 mL de toluène, 91 mg (0, 36 mmol) de diiode dans 5 mL de toluène et 20,8 µL (0,181 mmol) de cyclohexylamine, à température ambiante pendant 24 h, on obtient, après purification par flash-chromatographie en éluant avec un gradient de mélanges de cyclohexane et d'acétate d'éthyle (de 95/5 à 90/10 en volumes), puis cristallisation dans 2 mL d'oxyde de di*iso*propyle, 79 mg (50%) de *trans*-diiodo(*N*-cyclohexylamine)[3-benzyl-4,5-diphényl-1-méthyl-imidazol-2-ylidène] platine (II), sous forme d'une poudre jaune, dont les caractéristiques sont les suivantes :
- Rf = 0,23 (gel de silice, mélange de 90% de cyclohexane et de 10% d'acétate d'éthyle).
- Spectre de RMN¹H (CDCl₃): δ ppm : 1.10 - 1.44 (m, 5 H) 1.67 (dt, *J*=12.7, 3.3 Hz, 1 H) 1.80(dt, *J*=13.4, 3.5 Hz, 2 H) 2.29 (d, J=12.1 Hz, 2 H) 2.88 - 3.09 (m, 2 H) 3.21 - 3.36 (m, 1 H) 3.94 (s, 3 H)5.75 (s, 2 H) 6.97 (d, *J*=7.0 Hz, 2 H) 7.13 - 7.27 (m, 10 H) 7.32 -7.37 (m,3H)

### Exemple 27 : trans-diiodo[N-(1-méthyl-pipéridin-4-ylamine)](1-méthyl-3-benzyl-imidazol-2-ylidène) platine(II)

A une solution de complexe [1-méthyl-3-benzyl-imidazol-2-ylidène](1,3-divinyl-1,1,3,3-tetraméthyldisiloxane)] platine(0), obtenu à l'étape 1 de l'exemple 5, (0.296 g, 0.535 mmol) dans 5 mL de tétrahydrofurane à 0°C est additionnée une solution de diiode (0,142 g, 0.562 mmol) dans 5 mL de tétrahydrofurane. Par la suite une solution de 67,2 mg (0,589 mmol) de 4-amino-1-méthyl-pipéridine dans 10 mL de tétrahydrofurane est ajoutée au mélange. Le mélange réactionnel est agité pendant 16h à température ambiante puis évaporé à sec. Le résidu obtenu est purifié par cristallisation dans un mélange de pentane et d'oxyde de diéthyle (80/20 en volumes), on obtient ainsi 0.187 g (48%) de *trans*-diiodo[*N*-(1-méthyl-pipéridin-4-ylamine)](1-méthyl-3-benzyl-imidazol-2-ylidène) platine(II), sous forme de fins cristaux jaunes, dont les caractéristiques sont les suivantes :
- spectre de RMN ¹H (400 MHz, CDCl₃) δ ppm : 1.39 - 1.61 (m, 2 H) 1.98 (t, J=11.0 Hz, 2 H) 2.11 (d, J=13.4 Hz, 2 H) 2.17 (s, 3 H) 2.61 - 2.99 (m, 4 H) 3.18 (br. s., 1 H) 3.74 (s, 3 H) 5.44 (s, 2 H) 6.42 (d, J=1.5 Hz, 1 H) 6.63 (d, J=1.8 Hz, 1 H) 7.15 - 7.28 (m, 3 H) 7.25 - 7.35 (m, 2 H).

### Exemple 28 : trans-diiodo(N-pentan-3-ylamie)(1-méthyl-3-benzyl-imidazol-2-ylidène) platine(II)

A une solution de complexe [1-méthyl-3-benzyl-imidazol-2-ylidène](1,3-divinyl-1,1,3,3-tetraméthyldisiloxane)] platine(0), obtenu à l'étape 1 de l'exemple 5, (0,2 g, 0.379 mmol) dans 5 mL de tétrahydrofurane à 0°C est additionnée une solution de diiode (96,2 mg, 0.361 mmol) dans 5 mL de tétrahydrofurane. Par la suite une solution de 46,3 µL (0,397 mmol) de 3-aminopentane dans 2 mL de tétrahydrofurane est ajoutée au mélange. Le mélange réactionnel est agité pendant 18 h à température ambiante puis évaporé à sec. Le résidu obtenu est purifié par cristallisation dans 40 mL d'hexane, on obtient ainsi 0.187 g (48%) de *trans*-diiodo(*N-*pentan-3-ylamine)(1-méthyl-3-benzyl-imidazol-2-ylidène) platine(II), sous forme de cristaux jaunes, dont les caractéristiques sont les suivantes :
- spectre de RMN¹H (400 MHz, CDCl₃) δ ppm : 1.05 (t, J=7.5 Hz, 6 H) 1.66 - 1.88 (m, 4 H) 2.88 - 2.99 (m, 2 H) 3.34 - 3.44 (m, 1 H) 3.95 (s, 3 H) 5.65 (s, 2 H) 6.62 (d, J=2.0 Hz, 1 H) 6.83 (d, *J*=2.2 Hz, 1 H) 7.36 - 7.45 (m, 3 H) 7.48 - 7.54 (m, 2 H).

### Exemple 29 : trans-diiodo(N-4-amino-tétrahydrocyrane)(1-méthyl-3-benzyl-imidazol-2-ylidène) platine(II)

A une solution de complexe [1-méthyl-3-benzyl-imidazol-2-ylidène](1,3-divinyl-1,1,3,3-tetraméthyldisiloxane)] platine(0), obtenu à l'étape 1 de l'exemple 5, (0,2 g, 0.361 mmol) dans 5 mL de tétrahydrofurane à 0°C est additionnée une solution de diiode (96,2 mg, 0.379 mmol) dans 5 mL de tétrahydrofurane. Par la suite une solution de 54,7 mg (0,397 mmol) de chlorhydrate de 4-amino-tétrahydrohydropyrane dans 2 mL de tétrahydrofurane et 0,1 mL de diisopropyléthylamine est ajoutée au mélange. Le mélange réactionnel est agité pendant 18 h à température ambiante puis évaporé à sec. Le résidu obtenu est purifié par flash chromatographie sur gel de silice en éluant avec du dichlorométhane. On obtient ainsi 0.320 g (82%) de *trans*-diiodo(*N*-4-amino-tétrahydropyrane)(1-méthyl-3-benzyl-imidazol-2-ylidène) platine(ll), sous forme d'une poudre jaune, dont les caractéristiques sont les suivantes :
- spectre de RMN ¹H (400 MHz, CDCl₃) δ ppm : 1.54 - 1.72 (m, 2 H) 2.20 - 2.35 (m, 2 H) 2.94 - 3.20 (m, 2 H) 3.37 - 3.64 (m, 3 H) 3.94 (s, 3 H) 3.97 - 4.09 (m, 2 H) 5.63 (s, 2 H) 6.62 (d, *J*=2.2 Hz, 1 H) 6.84 (d, J=2.2 Hz, 1 H) 7.35 - 7.46 (m, 3 H) 7.45 - 7.52 (m, 2 H).

### Exemple 30 : trans-diiodo(N-cyclopentylamine)(1-méthyl-3-benzyl-imidazol-2-ylidène) platine(II)

A une solution de complexe [1-méthyl-3-benzyl-imidazol-2-ylidène](1,3-divinyl-1,1,3,3-tetraméthyldisiloxane)] platine(0), obtenu à l'étape 1 de l'exemple 5, (0,2 g, 0.361 mmol) dans 5 mL de tétrahydrofurane à 0°C est additionnée une solution de diiode (96,2 mg, 0.379 mmol) dans 5 mL de tétrahydrofurane. Par la suite une solution de 39,2 µL (0,397 mmol) de cyclopentylamine dans 2 mL de tétrahydrofurane est ajoutée au mélange. Le mélange réactionnel est agité pendant 18 h à température ambiante puis évaporé à sec. Le résidu obtenu est purifié par flash chromatographie sur gel de silice en éluant avec un mélange d'heptane et d'acétate d'éthyle (90/10 en volumes). On obtient ainsi 82 mg (32%) de *trans*-diiodo(*N*-cyclopentylamine)(1-méthyl-3-benzyl-imidazol-2-ylidène) platine(ll), sous forme d'une poudre jaune, dont les caractéristiques sont les suivantes :
- spectre de RMN ¹H (400 MHz, CDCl₃) δ ppm : 1.62 - 1.87 (m, 6 H) 2.05 - 2.20 (m, 2 H) 2.92 - 3.18 (m, 2 H) 3.84 - 3.99 (m, 4 H) 5.65 (s, 2 H) 6.62 (d, J=2.0 Hz, 1 H) 6.83 (d, *J*=2.0 Hz, 1 H) 7.35 - 7.46 (m, 3 H) 7.51 (d, J=6.8 Hz, 2 H).

### Exemple 31 : trans-diiodo(N-morpholine)(1-méthyl-3-benzyl-imidazol-2-ylidène) platine(II)

A une solution de complexe [1-méthyl-3-benzyl-imidazol-2-ylidène](1,3-divinyl-1,1,3,3-tetraméthyldisiloxane)] platine(0), obtenu à l'étape 1 de l'exemple 5, (0,2 g, 0.361 mmol) dans 5 mL de tétrahydrofurane à 0°C est additionnée une solution de diiode (96,2 mg, 0.379 mmol) dans 5 mL de tétrahydrofurane. Par la suite une solution de 35,2 µL (0,397 mmol) de morpholine dans 2 mL de tétrahydrofurane est ajoutée au mélange. Le mélange réactionnel est agité pendant 18 h à température ambiante puis évaporé à sec. Le résidu obtenu est purifié par flash chromatographie sur gel de silice en éluant avec un gradient de mélanges d'heptane et d'acétate d'éthyle (de 95/5 à 85/15 en volumes). On obtient ainsi 15 mg (6%) de *trans*-düodo(*N-*morpholine)(1-méthyl-3-benzyl-imidazol-2-ylidène) platine(II), sous forme d'une poudre jaune, dont les caractéristiques sont les suivantes :
- spectre de RMN ¹H (400 MHz, CDCl₃) δ ppm : 2.97 (d, J=13.2 Hz, 2 H) 3.29 - 3.43 (m, 1 H) 3.55 - 3.74 (m, 4 H) 3.88 (d, *J*=10.7 Hz, 2 H) 3.92 (s, 3 H) 5.62 (s, 2 H) 6.62 (d, *J*=2.0 Hz, 1 H) 6.83 (d, *J*=2.0 Hz, 1 H) 7.36 - 7.47 (m, 3 H) 7.46 - 7.52 (m, 2 H).
- LC/MS : Colonne ACQUITY BEH C18 1,7 µmde 2,1 x 50 mm, à 50°C.

| | |
|---|---|
| Solvants : | A = eau contenant 0,1% d'acide formique ; B = acétonitrile contenant 0,1% d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 1,09 mn | |

### Exemple 32 : trans-diiodo(N-cyclohexylamine)[3-(3-méthoxybenzyl)-1-méthyl-imidazol-2-vlidène]platine (II)

### Etape 1: [3-(3-méthoxybenryl)-1-méthyl-imidazol-2-ylidène](1,3-divinyl-1,1,3,3-tétraméthyldisiloxane)platine (0).

Dans un tricol de 100 mL, sous atmosphère d'argon, on dissout 0.564 g (1,99 mmol) de bromure de 3-(3-méthoxybenzyl)-l-méthyl-imidazolium, qui peut être obtenu selon J. Agr. Food Chem. (2007), 55(22), 9142-8, dans 50 mL de dicholorométhane, puis on ajoute 0,231 g (0,996 mmol) d'oxyde d'argent. Après 1 h d'agitation à température ambiante, on ajoute 20,1 mL (2,01 mmol) d'une solution 0,1 M de (1,3-divinyl-1,1,3,3-tétraméthyldisiloxane) platine (0) dans le xylène, puis on agite pendant une nuit à température ambiante. Après filtration sur Célite des sels d'argent insolubles, le milieu réactionnel est concentré à sec sous pression réduite. Le résidu est mis à cristalliser pendant une nuit au congélateur à -20°, puis les cristaux formés sont essorés, lavés au pentane et séchés sous pression réduite (20 mbars). On obtient ainsi 0,434 g (37%) de [3-(3-méthoxybenzyl)-1-méthyl-imidazol-2-ylidène](1,3-divinyl-1,1,3,3-tétraméthyldisiloxane)platine (0), sous forme de cristaux blancs, dont les caractéristiques sont les suivantes :
- LC/MS: Colonne ACQUITY BEH C18 1,7 µm de 2,1 x 50 mm, à 50°C.

| | |
|---|---|
| Solvants : | A = eau contenant 0,1% d'acide formique ; B = acétonitrile contenant 0,1% d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 1,31 mn | |

### Etape 2 : trans-diiodo(N-cyclohexylamine)[3-(3-méthoxybenzyl)-1-méthyl-imidazol-2-ylidène]platine (II).

En opérant comme à l'étape 1 de l'exemple 5, mais à partir de 0,2 g (0,342 mmol) du composé obtenu à l'étape 1, dans 5 mL de tétrahydrofurane, 91,3 mg (0,36 mmol) de diiode dans 5 mL de tétrahydrofrane et 41,1 µL (0,374 mmol) de cyclohexylamine, à température ambiante pendant une nuit, on obtient, après purification par flash-chromatographie en éluant avec un gradient de mélanges de cyclohexane et d'acétate d'éthyle ( de 90/10 à 60/40 en volumes), 100 mg (39%) de *trans*-diiodo(*N-*cyclohexylamine)[3-(3-méthoxybenzyl)-1-méthyl-imidazol-2-ylidène]platine (II), sous forme d'une poudre jaune, dont les caractéristiques sont les suivantes :
- Spectre de RMN¹H (CDCl₃): δ ppm 1.10 - 1.47 (m, 5 H) 1.69 (br.d, *J*=12.9 Hz, 1 H) 1.82 (br.d, *J*=13.2 Hz, 2 H) 2.34 (d, *J*=13.2 Hz, 2 H) 2.86 - 3.09 (m, 2 H) 3.25 - 3.37 (m, 1 H) 3.85 (s, 3 H) 3.95 (s, 3 H) 5.62 (s, 2 H) 6.65 (s, 1 H) 6.84 (s, 1 H) 6.93 (d, *J*=8.1 Hz, 1 H) 7.06 (d, *J*=7.2 Hz, 1 H) 7.11 (s, 1 H) 7.32 - 7.36 (1 H).

### Exemple 33 : trans-diiodo(N-cyclohexylamine)[1-méthyl-3-(3-trifluorométhyl-benzyl)imidazol-2-ylidène]platine (II)

### Etape 1 : bromure de 1-méthyl-3-(3-trifluorométhyl-benzyl)-imidazolium

Dans un ballon monocol de 25 mL, on dissout 1,4 g (17,05 mmole) de 1-méthylimidazole dans 15 mL de 1,2-dichloroéthane puis on ajoute 4,89 g (20,46 mmole) de bromure de 3-trifluorométhyl-benzyle. Après 1 nuit de chauffage à 80°, on ajoute 50 mL d'oxyde de diéthyle. L'huile, qui décante alors, est cristallisée dans un mélange d'acétate d'éthyle et de tétrahydrofurane (2/1 en volumes). Les cristaux ainsi obtenus sont essorés puis séchés sous pression réduite. On obtient ainsi 3,06 g (56%) de bromure de 1-méthyl-3-(3-trifluorométhy-lbenryl)-imidazolium, sous forme de cristaux blancs cassés, utilisés tels quels à l'étape suivante, dont la caractéristique est la suivante :
- LC/MS : Colonne ACQUITY BEH C18 1,7 µm de 2,1 x 50 mm, à 50°C.

| | |
|---|---|
| Solvants : | A = eau contenant 0,1% d'acide formique ; B = acétonitrile contenant 0,1% d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 0,40 mn | |

### Etape 2 : [1-méthyl-3-(3-trifluorométhyl-benzyl)-imidazol-2-ylidène](1,3-divinyl-1,1,3,3-tétraméthyl-disiloxane)platine (0).

En opérant comme à l'étape 1 de l'exemple 32, mais à partir de 0,5 g (1,87 mmol.) de bromure de 1-méthyl-3-(3-trifluorométhyl-benzyl)imidazolium, obtenu à l'étape précédente, et de 0,217 g (0,935 mmol) d'oxyde d'argent dans 50 mL de dichlorométhane, puis de 18,9 mL (1,89 mmol) d'une solution 0,1 M de (1,3-divinyl-1,1,3,3-tétraméthyldisiloxane) platine (0) dans le xylène, on obtient, après purification par cristallisation dans le pentane, 0,513 g (44%) de [1-méthyl-3-(3-trifluorométhyl-benzyl)imidazol-2-ylidène](1,3-divinyl-1,1,3,3-tétraméthyl-disiloxane)platine (0), sous forme de fins cristaux blancs, dont la caractéristique est la suivante :
- Rf = 0,42 (gel de silice, mélange de 90% de cyclohexane et de 10% d'acétate d'éthyle).

### Etape 3: trans-diiodo(N-cyclohexylamine)[1-méthyl-3-(3-trifluorométhyl-benzyl-imidazol-2-ylidène]platine (II)

En opérant comme à l'étape 3 de l'exemple 11, mais à partir de 0,2 g (0,314 mmol) du composé obtenu à l'étape 2, dans 20 mL de tétrahydrofurane, 83,7 mg (0, 33 mmol) de diiode dans 5 mL de tétrahydrofurane et 39,53 µL (0,345 mmol) de cyclohexylamine, à température ambiante pendant 24 h, on obtient, après purification par flash-chromatographie en éluant avec un mélange de cyclohexane et d'acétate d'éthyle (90/10 en volumes), puis cristallisation dans 2 mL de pentane, 100 mg (40%) de *trans-*diiodo(*N*-cyclohexylamine)[1-méthyl-3-(3-trifluorométhyl-benzyl)imidazol-2-ylidène]platine (II), sous forme de fins cristaux jaunes, dont les caractéristiques sont les suivantes :
- Rf = 0,18 (gel de silice, mélange de 90% de cyclohexane et de 10% d'acétate d'éthyle).
- Spectre de RMN¹H (400 MHz, CDCl₃): δ ppm : 1.09 - 1.46 (m, 5 H) 1.69 (ddd, *J*=13.1, 3.2, 3.0 Hz, 1 H) 1.82 (ddd, J=13.5, 3.5, 3.2 Hz, 2 H) 2.32 (dd, J=12.6, 2.7 Hz, 2 H) 2.82 - 3.09 (m, 2 H) 3.20 - 3.38 (m, 1 H) 3.96 (s, 3 H) 5.73 (s, 2 H) 6.66 (d, *J*=2.0 Hz, 1 H) 6.89 (d, *J*=2.0 Hz, 1 H) 7.55 (t, *J*=7.8 Hz, 1 H) 7.65 (d, *J*=7.7 Hz, 1 H) 7.72 (d, *J*=7.7 Hz, 1 H) 7.76 (s, 1 H)

### Exemple 34 : trans-dinitrato(N-cyclohexylamine)[1-méthyl-3-(4-trifluorométhyl-benzyl)imidazol-2-ylidène]platine (II)

Dans un tricol de 50 mL sous atmosphère d'argon, 80 mg (0,102 mmol) de *trans-*diiodo(*N*.cyclohexylamine)[1-méthyl-3-(4-trifluorométhyl-benzyl)imidazol-2-ylidène]platine (II), obtenu à l'exemple 11, sont dissous dans 20 mL de dichlorométhane puis on ajoute 36,2 mg (0,207 mmol) de nitrate d'argent et on agite une nuit à température ambiante Les sels d'argent précipités sont filtrés, et le filtrat est concentré à sec. Le résidu obtenu est cristallisé dans un mélange d'heptane et d'acétate d'éthyle (1/1 en volumes). On obtient ainsi 9 mg (13%) de *trans*-dinitrato(*N*-cyclohexylamine)[1-méthyl-3-(4-trifluorométhyl-benzyl)imidazol-2-ylidène]platine (II), sous forme de fins cristaux blancs dont les caractéristiques sont les suivantes :
- Spectre de RMN¹H (400 MHz, CDCl₃): δ ppm : 1.14 - 1.26 (m, 1 H) 1.27 - 1.48 (m, 4 H) 1.67 - 1.77 (m, 1 H) 1.81 - 1.90 (m, 2 H) 2.30 - 2.41 (m, 2 H) 2.87 (br. s., 1 H) 3.32 - 3.44 (m, 2 H) 4.21 (s, 3 H) 5.88 (s, 2 H) 6.74 (d, *J*=2.0 Hz, 1 H) 6.94 (d, *J*=2.0 Hz, 1 H) 7.46 (d, *J*=8.1 Hz, 2 H) 7.70 (d, *J*=8.1 Hz, 2 H)
- LC/MS : Colonne ACQUITY BEH C18 1,7 µm de 2,1 x 50 mm, à 50°C.

| | |
|---|---|
| Solvants : | A = eau contenant 0,1% d'acide formique ; B = acétonitrile contenant 0,1 % d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 0,51 mn | |

### Exemple 35 : trans-diiodo(N-cyclohexylamine)[3-(3,4-diméthoxybenzyl)-1-méthyl-imidazol-2-ylidène]platine (II)

### Etape 1 : bromure de (3,4-diméthoxybenzyl)-1-méthyl-imidazolium

Dans un ballon monocol de 25 mL, on dissout 0,35 g (4,26 mmole) de 1-méthylimidazole dans 10 mL de tétrahydrofurane puis on ajoute 1,18 g (5,12 mmole) de bromure de 3,4-diméthoxybenzyle. Après 1 nuit de chauffage à reflux, on ajoute 50 mL d'oxyde de diéthyle. Le précipité formé est essoré puis lavé à l'oxyde de diéthyle. On obtient ainsi 1,25 g (94%) de bromure de 3-(3,4-diméthoxybenzyl)-1-méthyl-imidazofium, sous forme d'une poudre beige clair, utilisée telle quelle à l'étape suivante, dont la caractéristique est la suivante :
- LC/MS : Colonne ACQUITY BEH C181,7 µm de 2,1 x 50 mm, à 50°C.

| | |
|---|---|
| Solvants : | A = eau contenant 0,1% d'acide formique ; B = acétonitrile contenant 0,1% d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 0,38 mn | |

### Etape 2: [3-(3,4-diméthoxybenzyl)-1-méthyl-imidazol-2-ylidènej(1,3-divinyl-1,1,3,3-tétraméthyldisiloxane)platine (0).

En opérant comme à l'étape 1 de l'exemple 32, mais à partir de 0,753 g (2,235 mmol.) de bromure de 3-(3,4-diméthoxybenzyl)-1-méthyl-imidazolium, obtenu à l'étape précédente, et de 0,259 g (1,118 mmol) d'oxyde d'argent dans 100 mL de dichlorométhane, puis de 24,6 mL (2,46 mmol) d'une solution 0,1 M de (1,3-divinyl-1,1,3,3-tétraméthyldisiloxane) platine (0) dans le xylène, on obtient, après purification par cristallisation dans le pentane, 0,525 g (38%) de [3-(3,4-diméthoxybenryl)-1-méthyl-imidazol-2-ylidène](1,3-divinyl-1,1,3,3-tétraméthyl-disiloxane)platine (0), sous forme de fins cristaux blancs, dont la caractéristique est la suivante :
- LC/MS : Colonne ACQUITY BEH C18 1,7 µm de 2,1 x 50 mm, à 50°C.

| | |
|---|---|
| Solvants : | A = eau contenant 0,1% d'acide formique ; B = acétonitrile contenant 0,1% d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 1,28 mn | |

### Etape 3: trans-diiodo(N-cyclohexylamine)[3-(3,4-diméthoxybenzyl)-1-méthyl-imidazol-2-ylidène]platine (II)

En opérant comme à l'étape 3 de l'exemple 11, mais à partir de 0,4 g (0,652 mmol) du composé obtenu à l'étape 2, dans 35 mL de tétrahydrofurane, 174 mg (0,686 mmol) de diiode dans 5 mL de tétrahydrofurane et 82 µL (0,717 mmol) de cyclohexylamine, à température ambiante pendant 24 h, on obtient, après purification par cristallisation dans un mélange de pentane et de tétrahydrofurane (10/1 en volumes), 256 mg (50%) de *trans*-diiodo(*N*-cyclohexylamine)[3-(3,4-diméthoxybenzyl)-1-méthyl-imidazol-2-ylidène]platine (II), sous forme de fins cristaux jaunes, dont les caractéristiques sont les suivantes :
- Rf = 0,27 (gel de silice, mélange de 90% de cyclohexane et de 10% d'acétate d'éthyle).
- Spectre de RMN¹H (400 MHz, CDCl₃): δ ppm : 1.07 - 1.42 (m, 5 H) 1.63 (ddd, *J*=12.9, 3.6, 3.4 Hz, 1 H) 1.77 (dt, *J*=13.5, 3.4 Hz, 2 H) 2.29 (d, *J*=13.6 Hz, 2 H) 2.84 - 3.04 (m, 2 H) 3.22 - 3.32 (m, 1 H) 3.86 (s, 3H) 3.88 (s, 3 H) 3.89 (s, 3 H) 5.53 (s, 2 H) 6.58 (d, *J*=2.0 Hz, 1 H) 6.77 (d, *J*=2.2 Hz, 1 H) 6.84 (d, *J*=8.1 Hz, 1 H) 6.95 (dd, *J*=8.1, 2.0 Hz, 1 H) 7.12 (d, *J*=2.0 Hz, 1 H)

### Exemple 36 : trans-diiodo(N-morpholine)(1-méthyl-3-(4-trifluorométhyl-benzyl)-imidazol-2-ylidène) platine(II)

A une solution de complexe [1-méthyl-3-(4-trifluorométhyl-benzyl)-imidazol-2-ylidène](1,3-divinyl-1,1,3,3-tetraméthyldisiloxane)] platine(0), obtenu à l'étape 2 de l'exemple 11, (0,5 g, 0.804 mmol) dans 5 mL de tétrahydrofurane à 0°C est additionnée une solution de diiode (214,3 mg, 0,844 mmol) dans 5 mL de tétrahydrofurane. Par la suite une solution de 77,4 µL (0,884 mmol) de morpholine dans 2 mL de tétrahydrofurane est ajoutée au mélange. Le mélange réactionnel est agité pendant 18 h à température ambiante puis évaporé à sec. Le résidu obtenu est purifié par flash chromatographie sur gel de silice en éluant avec un mélange d'heptane et d'acétate d'éthyle (85/15 en volumes), on obtient ainsi 260 mg (42%) de *trans*-diiodo(*N-*morpholine)(1-méthyl-3-(4-trifluorométhyl-benzyl)-imidazol-2-ylidène) platine(II), sous forme d'une poudre jaune, dont les caractéristiques sont les suivantes :
- spectre de RMN ¹H (400 MHz, CDCl₃) δ ppm : 2.96 (d*, J*=12.5 Hz, 2 H) 3.21 - 3.52 (m, 1 1 H) 3.51 - 3.74 (m, 4 H) 3.87 (d, *J*=9.6 Hz, 2 H) 3.94 (s, 3 H) 5.71 (s, 2 H) 6.64 (s, 1 H) 6.89 (s, 1 H) 7.55 - 7.64 (m, 2 H) 7.65 - 7.74 (m, 2 H)
- LC/MS : Colonne ACQUITY BEH C18 1,7 µm de 2,1 x 50 mm, à 50°C.

| | |
|---|---|
| Solvants : | A = eau contenant 0,1% d'acide formique ; B = acétonitrile contenant 0, 1 % d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 1,16 mn | |

### Exemple 37 : trans-diiodo(N-tétrahydropyran-4-ylamine)(1-méthyl-3-(4-trifluorométhyl-benzyl)-imidazol-2-ylidène) platine(II)

A une solution de (1-méthy)-3-(4-trifluorométhyl-benzyl)-imidazol-2-ylidéne](1,3-divinyl-1,1,3,3-tetraméthyldisiloxane)] plafine(0), obtenu à l'étape 2 de l'exemple 11, (0,4 g, 0.643 mmol) dans 5 mL de tétrahydrofurane à 0°C est additionnée une solution de diiode (171,5 mg, 0,676 mmol) dans 5 mL de tétrahydrofurane. Par la suite une solution de 71,6 mg (0,708 mmol) de chlorure de 4-amino-tétrahydropyrane dans 2 mL de tétrahydrofurane et 0,1 mL de diisopropyléthylamine est ajoutée au mélange. Le mélange réactionnel est agité pendant une nuit à température ambiante puis évaporé à sec. Le résidu obtenu est purifié par flash chromatographie sur gel de silice en éluant avec un mélange d'heptane et d'acétate d'éthyle (80/20 en volumes), on obtient ainsi 180 mg (35%) de *trans*-diiodo(*N*-tétrahydropyran-4-ylamine)(1-méthyl-3-(4-trifluorométhyl-benzyl)-imidazol-2-ylidène) platine(II), sous forme d'une poudre jaune, dont les caractéristiques sont les suivantes :
- spectre de RMN ¹H (400 MHz, CDCl₃) δ ppm : 1.61 - 1.68 (m, 2 H) 2.26 (d, J=14.0 Hz, 2 H) 2.96 - 3.18 (m, 2 H) 3.35 - 3.62 (m, 3 H) 3.96 (s, 3 H) 3.99 - 4.08 (m, 2 H) 5.73 (s, 2 H) 6.65 (s, 1 H) 6.90 (s, 1 H) 7.54 - 7.65 (m, 2 H) 7.66 - 7.73 (m, 2 H)
- LC/MS : Colonne ACQUITY BEH C18 1,7 µm de 2,1 x 50 mm, à 50°C.

| | |
|---|---|
| Solvants : | A = eau contenant 0,1% d'acide formique ; B = acétonitrile contenant 0,1% d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 1,12 mn | |

### Exemple 38 : trans-dibromo(N-cyclohexylamine)[1-méthyl-3-(4-trifluorométhyl-benzyl)imidazol-2-ylidène]platine (II)

Dans un tricol de 25 mL, 300 mg (0,482 mmol) de [1-méthyl-3-(4-trifluorométhyl-benzyl)-imidazol-2-ylidène](1,3-divinyl-1,1,3,3-tetraméthyldisiloxane)] platine(0), obtenu à l'étape 2 de l'exemple 11, sont dissous dans 5 mL de tétrahydrofurane, on ajoute une solution de 24,9 µL (0,482 mmol) de dibrome dans 2 mL de tétrahydrofurane. Puis après décoloration presque totale de la solution précédente, on ajoute 55,2 µL (0,482 mmol) de cyclohexylamine. Après une nuit d'agitation à température ambiante, le milieu réactionnel est concentré sous pression réduite. Le résidu obtenu est purifié par flash-chromatographie sur une colonne d'alumine, en éluant avec un gradient de mélanges d'heptane et d'acétate d'éthyle (de 90/10 à 70/30 en volumes). On obtient ainsi 20 mg (6%) de *trans*-dibromo(*N-*cyclohexylamine)[1-méthyl-3-(4-trifluorométhyl-benzyl)imidazol-2-ylidène]platine (II), sous forme d'une poudre jaune d'or dont les caractéristiques sont les suivantes :
- Spectre de RMN¹H (400 MHz, CDCl₃): δ ppm : 1.10 - 1.48 (m, 5 H) 1.63 - 1.73 (m, 1 H) 1.75 - 1.87 (m, 2 H) 2.34 (d, *J*=13.8 Hz, 2 H) 2.78 - 3.06 (m, 2 H) 3.12 - 3.31 (m, 1 H) 4.10 (s, 3 H) 5.85 (s, 2 H) 6.69 (s, 1 H) 6.89 (s, 1 H) 7.57 - 7.64 (m, 2 H) 7.64 - 7.71 (m, 2 H)
- LC/MS : Colonne ACQUITY BEH C18 1,7 µm de 2,1 x 50 mm, à 50°C.

| | |
|---|---|
| Solvants : | A = eau contenant 0,1% d'acide formique ; B = acétonitrile contenant 0,1% d'acide formique. |
| Gradient : | de 5 à 50% de B en 0,8 mn, puis de 50 à 100% de B en 0,4 mn, puis 0,65 mn à 100% de B, puis de 100 à 5% de B en 0,15 mn |
| Débit : | 1 mL/mn |
| Temps de rétention = 1,49 mn | |

Produit de référence de la publication de S. Ray, R. Mohan, J. Singh, M. Samantaray, M. Shaikh, D. Panda and P. Ghosh « Anticancer and Antimicrobial Metallopharmaceutical Agents Based on Palladium, Gold and Silver N-Heterocyclic Carbene Complexes » J. Am. Chem. Soc.Vol 129, no.48 pages 15042-15053 ; (2007.12.05):
*trans*-diiodo(pyridine)(1-benzyl-3-*tert*-butylimidazol-2-ylidène) platine(II)

### Etape 1 : chlorure de 1-benzyl-3-tert-butylimidazolium

Une solution de 1-*tert*-butylimidazole, qui a été obtenu selon A. J. Arduengo U.S. Patent (2001) No. 6,177,575, (0,31 g ; 2,5 mmol) dans le chlorure de benzyle (0,3 mL ; 2,5 mmol) est agitée à température ambiante pendant 48h. Les volatiles sont ensuite évaporés sous vide. Le résidu obtenu est trituré dans de l'éther éthylique puis filtré permettant ainsi d'obtenir 0,54 g (87 %) de chlorure de 1-benzyl-3-*tert*-butylimidazolium, dont les caractéristiques sont les suivantes :
- RMN ¹H (300 MHz, CDCl₃) δ ppm : 1.73 (s, 9H), 5.71 (s, 2H), 7.15 (m, 1H), 7.25 (m, 1H), 7.38 (m, 3H), 7.52 (m, 2H), 11.41 (s, 1H).

### Etape 2: [1-benzyl-3-tert-butylimidazol-2-ylidène(1,3-divinyl-1,1,3,3-tetraméthyl-disiloxane)] platine(0)

A une solution de chlorure de 1-benzyl-3-*tert-*butylimidazolium (0,18 g ; 0,7 mmol) dans 10 mL de chloroforme est ajouté une solution de 1,3-divinyl-1,1,3,3-tetraméthyldisiloxane platine(0) 0,1 M dans le xylène (7 mL ; 0,7 mmol). Le mélange est refroidi à 0°C et 0,12 g de *tert*-butylate de potassium (1,03 mmol) sont ajoutés. Le mélange réactionnel est agité pendant 16 h à température ambiante puis évaporé à sec. Le résidu obtenu est purifié par chromatographie flash par élution avec un mélange d'heptane et d'acétate d'éthyle (95/5 en volumes). On recueille ainsi 0,17 g (42 %) de [1-benzyl-3-*tert-*butylimidazol-2-ylidène (1,3-divinyl-1,1,3,3-tetraméthyldisiloxane)] platine(0) sous forme d'un mélange 1:1 de deux isomères.
- RMN ¹H (300 MHz, CDCl₃) δ ppm : -0.45 (s, 6H), -0.27 (s, 6H), 0.30 (s, 6H), 0.32 (s, 6H), 1.61 (s, 9H), 1.64 (s, 9H), 1.8-1.9 (m, 8H), 2.1-2.3 (m, 4H), 5.16 (s, 2H), 5.28 (s, 2H), 6.84 (br s, 1H), 6.90 (br s, 1H), 7.08 (m, 4H), 7.20-7.35 (m, 6H).

### Etape 3 : trans-diiodo(pyridine)(1-benzyl-3-tert-butylimidazol-2-ylidène) platine(II)

A une solution de (1-benzyl-3-*tert*-butylimidazol-2-ylidène)(1,3-divinyl-1,1,3,3-tetraméthyldisiloxane) platine(0) (0,07 g ; 0,12 mmol), obtenu à l'étape précédente, dans 5 mL de toluène à 0°C est ajoutée une solution de diiode (0,03 g ; 0,13 mmol) dans 11 mL de toluène. Par la suite 15 µL de pyridine (0,12 mmol) sont additionnés au mélange. Le mélange réactionnel est agité pendant 16h à température ambiante puis évaporé à sec. Le résidu obtenu est purifié par chromatographie flash par élution avec un gradient de mélanges d'heptane et d'acétate d'éthyle (80 / 20 à 70 / 30 en volumes), on recueille ainsi 0,07 g (80%) de (1-benzyl-3-*tert*-butylimidazol-2-ylidène)(1,3-divinyl-1,1,3,3-tetraméthyldisiloxane) platine(0), dont les caractéristiques sont les suivantes :
- RMN ¹H (300 MHz, CDCl₃) δ ppm : 2.08 (s, 9H), 5.99 (s, 2H), 6.57 (d, 1H), 7.02 (d, 1H), 7.3-7.5 (m, 5H), 7.53 (m, 2H), 7.71 (m, 1H), 9.06 (m, 2H).
- Spectre de masse HRMS (ESI mode positif) calculé pour C₁₉H₂₃l₂N₃Pt.Na : 764.9527 ; trouvé pour C₁₉H₂₃l₂N₃NaPt : 764.9525.

Le tableau qui suit illustre les structures chimiques de quelques exemples de composés selon l'invention. Dans ce tableau :
- Me, représente méthyle,
- Ph et Bn représentent respectivement les groupes phényle et benzyle.

**Tableau**

| **N°** | **R1** | **R2** | **R3** | **V** | **RR'NH** | **X** | **MM** (masse moléculaire) |
|---|---|---|---|---|---|---|---|
| **1** | Me | Me | H | CH | 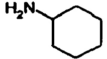 | I | 644,21 |
| **2** | Me | Me | H | N | 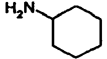 | I | 645,20 |
| **3** | Me | 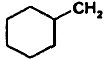 | H | CH | 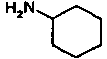 | I | 726,36 |
| **4** | Me | Ph | H | CH | 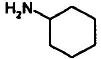 | I | 706,28 |
| **5** | Me | Bn | H | CH | 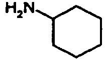 | I | 720,31 |
| **6** | Me | Me | H | CH | 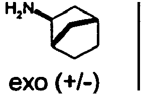 | I | 656,22 |
| **7** | Me | Me | 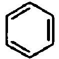 | | 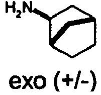 | I | 706,28 |
| **8** | Me | Me | 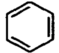 | | 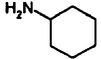 | I | 694,27 |
| **9** | Me | Me | 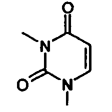 | | 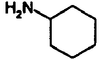 | I | 756,30 |
| **10** | Me | 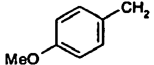 | H | CH | 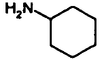 | I | 750,33 |
| **11** | Me | 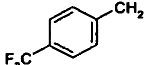 | H | CH | 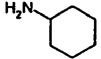 | I | 788,31 |
| **12** | Bn | Bn | H | CH | 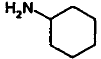 | I | 796,41 |
| **13** | Me | Me | H | CH | H₂N-Bn | I | 652,19 |
| **14** | Me | Me | H | CH | 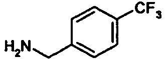 | I | 720,19 |
| **15** | Me | CH=CH₂ | H | CH | 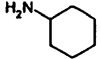 | I | 656,22 |
| **16** | Me | Me | 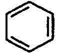 | | 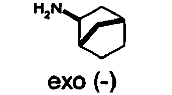 | I | 706,28 |
| **17** | Me | Me | 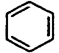 | | 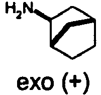 | I | 706,28 |
| **18** | 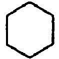 | 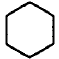 | H | CH | 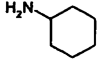 | I | 780,45 |
| **19** | Me | Ph | H | N | 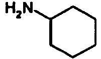 | I | 707.27 |
| **20** | Me | Me | Ph | CH | 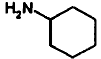 | I | 720.31 |
| **21** | Me | Bn | Ph | CH | 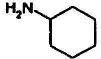 | I | 796.41 |

| **N°** | **R1** | **R2** | **R3** | **V** | **RR'NH** | **X** | **MM** (masse moléculair e) |
|---|---|---|---|---|---|---|---|
| **22** | Me | Me | Ph | C-Ph | 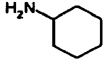 | I | 796.41 |
| **23** | Me | Me | H | CH | 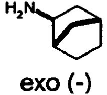 | I | 656.22 |
| **24** | Me | Me | H | CH | 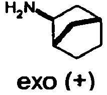 | I | 656.22 |
| **25** | Me | Me | 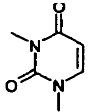 | | 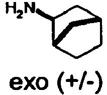 | I | 768.31 |
| **26** | Me | Bn | Ph | C-Ph | 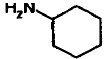 | I | 872,51 |
| **27** | Me | Bn | H | CH | 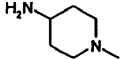 | I | 735,32 |
| **28** | Me | Bn | H | CH | 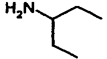 | I | 07,29 |
| **29** | Me | Bn | H | CH | 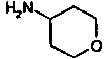 | I | 722,28 |
| **30** | Me | Bn | H | CH | 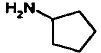 | I | 705,27 |
| **31** | Me | Bn | H | CH | 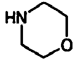 | I | 707,24 |
| **32** | Me | 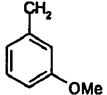 | H | CH | 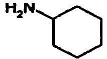 | I | 749,33 |
| **33** | Me | 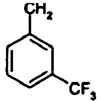 | H | CH | 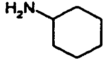 | I | 788.31 |
| **34** | Me | 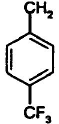 | H | CH | 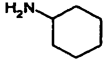 | ONO₂ | 657,5 |
| **35** | Me | 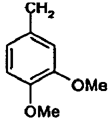 | H | CH | 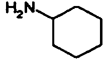 | I | 794,39 |
| **36** | Me | 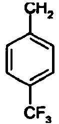 | H | CH | 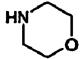 | I | 776,25 |
| **37** | Me | 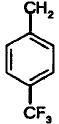 | H | CH | 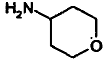 | I | 790,28 |
| **38** | Me | 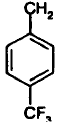 | H | CH | 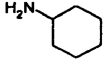 | Br | 694.31 |

Les composés selon l'invention ont fait l'objet d'essais pharmacologiques permettant de déterminer leur effet inhibiteur de cellules sensibles aux dérivés du platine.

Des essais ont consisté à mesurer l'activité *in vitro* des composés de l'invention sur les souches H460 et CEM, ainsi que sur les souches A2780, A2780 résistantes au DDP, CH1, CH1 résistantes au DPP et SKOV3. De nombreuses tumeurs sont résistantes aux composés de platines. Deux mécanismes principaux sont en causes :
Premier mécanisme : La résistance des tumeurs est attribuée à des niveaux inadéquats de composés platine susceptibles d'atteindre l'ADN.
   Les composés Platine pénètrent dans la cellule soit par l'intermédiaire de transporteurs, tels le transporteur de cuivre CRT1, ou bien par diffusion passive à travers la membrane plasmique. A l'intérieur de la cellule, les composés Platine forment de variétés chimiques aqueuses réactives, réagissant particulièrement avec des molécules contenant du soufre, tels le glutathion ou les metallothioneines. Enfin, les composés Platine sont exportés activement de la cellule à travers les exporteurs de cuivre ATP7A et ATP7B, ou bien par la pompe exportatrice GS-X (Glutathion-S-X). L'absence de récepteurs CTR1, ou bien des niveaux élevés de glutathion, ou bien encore de fortes expressions des exporteurs actifs peuvent être impliqués dans les mécanismes de résistances innées ou acquises des cellules tumorales vis-à-vis des composés Platine.
Second mécanisme: mécanismes de résistance des cellules tumorales, postérieurs à la liaison des composés Platine avec l'ADN.
   Dans le noyau, les composés Platine se lient de façon covalente à 2 guanine adjacentes : on parle de formation d'adduits. Mais les dégats causés par ces adduits peuvent être contournés par la cellule, qui peut utiliser plusieurs mécanismes de réparation: réparation par excision de nucléotide (nucleotide-excision repair ou NER), inactivation ou délétion du mécanisme de réparation des disparités de l'ADN (DNA mismatch repair ou MMR), contournement des adduits par certaines polymérases ou bien encoree faiblesse du processus de mort cellulaire programmée (apoptose). Tous ces mécanismes peuvent être à l'origine d'une résistance observée chez de nombreuses cellules tumorales aux composés Platine.
Les complexe de Platine de l'invention sont évalués sur des cellules qui expriment un ou plusieurs de ces mécanismes :
**CCRF-CEM** est une leucémie T humaine (Cancer 1965, 18: 522-529). L'oxaliplatine induit des lésions bifonctionnelles, tells que des adduits inter- et intra-brins d'ADN sur ces cellules CCRF-CEM (Mol Pharmacol 2000, 58: 920-927). Les cellules CCRF-CEM ne disposent pas de la protéine MLH1, une des deux principales protéines impliquées dans le mécanisme de MMR dans les cancers humains. Cette déficience rend particulièrement résistants ces cellules aux agents alkylants et aux composés Platine.
**NCI-H460** est un cancer du poumon humain non à petite cellule, possédant une mutation du gène K-*ras*. Cette cellule est sensible aux composés Platine. Dans cette lignée cellulaire, le cisplatine forme plus d'adduits que l'oxaliplatine à la même concentration et malgré tout, le pourcentage de réparation est plus élevé pour l'oxaliplatine (DNA Repair 2006, 5: 219-225).
**SK-OV-3** est un cancer ovarien humain. Comme la cellule CCRF-CEM, elle ne dispose pas de la protéine MLH1 (Mol Pharmacol 2000, 58: 920-927; BMC cancer 2006;6:201). Cette cellule possède un mécanisme de résistance intrinsèque au cisplatine, lié à des taux élevés de glutathion (Br J Cancer 1998;78:175-180). Cette lignée cellulaire est communément utilisée pour explorer les effets des niveaux élevés de glutathion sur la résistance vis-à-vis des composés Platine (Br J Cancer 1996, 74: 380-386).
**A2780 et A2780/DDP** sont des lignées de cancer ovarien humain. A2780 n'exprime pas le gène de la protéine MLH1 (BMC cancer 2006;6: 201). La résistance de la lignée A2780/DDP est de type acquise et fait intervenir différents mécanismes: - diminution de la capacité d'internalisation du cisplatine, - contournement réplicatif (celui-ci est définit comme la possibilité pour un complexe réplicatif de synthétiser de l'ADN au-delà du site de dommage) (Cancer Res 1994;54:3500-3505), - détoxification active par le glutathion (Exp Oncol 2005;27:191-195), - faible activité des Caspase-3, enzymes intervenant dans les processus de mort cellulaire programmée (J Cancer Res Clin Oncol 2004;130:423-428) et enfin, fort efflux nucléaire des composés Platine (Oncol Rep 2004;12:1365-1370).
**CH1 et CH1 CisR,** sont des cancers ovariens humains, respectivement sensibles et résistants au cisplatine. La sensibilité relative de la cellule CH1 peut être au moins partiellement attribuée à une déficience d'un gène qui intervient dans les processus de réparation et qui est capable de retirer les adduits (Chemico-Biological Interactions, 1999; 123:11-29). La cellule CH1cisR est résistante au cisplatine à travers un mécanisme imparfaitement connu de réparation de l'AND, ou bien d'augmentation de la tolérance de la cellule aux dommages causes par le cisplatine à l'ADN de cette cellule (Mol Pharmacol 2003;63:933-944, Cancer Res 52: 3857-3864).

### Les tests de Cytotoxicité:

Pour les cellules adhérentes: La prolifération cellulaire est évaluée par incorporation de ¹⁴C-thymidine dans l'ADN des cellules. A549, CH1, CH1/DDP et H460 sont cultivées en milieu Dulbecco's modified Eagle's medium (DMEM), SK-OV-3 dans un milieu Mc-Coy, A2780 et A2780/DDP dans un milieu RPMI1640. Tous les milieux contiennent 10% de sérum de veau foetal, 2 mM de L-glutamine, 100 unités/ml de pénicilline et 100 mg/ml de streptomycine. L'incubation est réalisée à 37°C dans une atmosphère de 95% air/5% CO₂.

Les cellules, en croissance exponentielles, sont introduites dans les puits d'une plaque 96 puits Cytostar, contenant un liquide scintillant (Cytostar-T scintillating microplate, GE Healthcare Bio-Sciences AB, Uppsala, Sweden), à la densité de 5.10³ à 10⁴ cellules par puits, à raison de 180µl par puits. Après 4 heures d'incubation, les composés à tester sont ajoutés. Les composés sont préalablement préparés en solution stock à la concentration de 10mM dans le DMSO. Une série de dilutions est réalisée, avant que les composés soient mis au contact des cellules sous un volume de 10µl. Après 72 heures d'incubation à 37°C dans une atmosphère de 95% air/5% CO_{2,} 0.1µCi/puits de ¹⁴C-thymidine sont ajoutés. 24h après (total 96h de traitement), l'incorporation de ¹⁴C-thymidine est enregistrée dans un compteur à scintillation adaptée, ( MicroBeta radioactivity counting, Perkin-Elmer Life Sciences, Boston, MA, USA). Les valeurs moyennes obtenues dans les groupes expérimentaux sont divisées par les valeurs moyennes obtenues dans les groupes contrôles pour obtenir des pourcentages par rapports aux contrôles.

Pour les cellules non-adhérentes CCRF-CEM, la viabilité cellulaire est mesurée par le test de luminescence CellTiter-Glo^{®} Luminescent Cell Viability Assay (Promega, Madison, WI, USA). Quand il est ajouté aux cellules, le réactif produit une luminescence en présence de l'ATP des cellules vivantes. Les résultats sont évalués sur un lecteur adapté (Envision, Perkin-Elmer Life Sciences, Boston, MA, USA).

Les cellules CCRF-CEM sont cultivées en milieu RPMI1640 contenant 10% de sérum de veau foetal, 2 mM de L-glutamine, 100 unités/ml de pénicilline et 100 mg/ml de streptomycine, à 37°C dans une atmosphère de 95% air/5% CO₂. Les cellules, en croissance exponentielles, sont introduites dans les puits d'une plaque 96 puits à la densité de 5.10³ cellules par puits dans une plaque opaque (Greiner), ceci sous un volume de 150µl. Après 4h d'incubation à 37°C dans une atmosphère of 95% air/5% CO₂, les composés (concentrés 20 fois) sont ajoutés sous un volume de 8µl. Les composés sont préalablement préparés en solution stock à la concentration de 10mM dans le DMSO. Une série de dilutions est réalisée, avant que les composés soient mis au contact des cellules. Après 96h d'incubation à 37°C, 100µl d'agents (CellTiter-Glo) sont ajoutés. Après lyse des cellules, la libération d'ATP est évaluée sur le lecteur Envision.

Les valeurs d'IC₅₀ sont évaluées à partir de courbes présentant le pourcentage de viabilité vs la concentration du composé testé, en utilisant le logiciel Biostat speed. L'IC₅₀ est définie comme la concentration du compose qui inhibe 50% de la prolifération cellulaire ou 50% de la viabilité cellulaire.

La viabilité cellulaire peut aussi être évaluée par le kit « Titer 96 assay » (Promega, Madison, Wisconsin, USA), qui utilise le 3-(4,5-dimethylthiazol-2-yl)-5-(3-carboxymethoxyphenyl)-2-(4-sulfophenyl)-2H-tetrazolium] (MTS) (Cancer Commun. 1991;3:207-212) et ceci quelque soit le type cellulaire. Ce test est un des plus communément utilisé dans la littérature. Il utilise une méthode d'absorption colorimétrique basé sur la capacité des enzymes déshydrogénases des cellules vivantes de produire le formazan, un composé brunâtre détectable à 490nm. Pour cela, en fin d'incubation, 20 µl de réactif MTS est ajouté aux puits de culture et la plaque incubée 1-3 heures supplémentaires. Une mesure du signal bruit de fond est évalué à partir de puits contenant les cellules auxquelles on a ajouté 20µl de Triton X100 en fin d'incubation. La survie cellulaire est évaluée par la mesure de l'absorbance à 490 nm en utilisant un compteur adapté (Victor2 Wallac plate reader, Perkin Elmer, Boston, Massachusetts, USA).

L'activité inhibitrice vis à vis de ces souches est donnée par la concentration qui inhibe 50% de l'activité de chacune d'entre elles.

Les Cl₅₀ de certains des composés selon l'invention sont comprises entre 1 µM et 5 µM sur les souches sensibles au cisplatine ou à l'oxaliplatine
Le tableau ci-dessous montre les résultats obtenus avec les exemples décrits précédemment :

| Exemple | CEM IC₅₀ µM | H460 IC₅₀ µM | A2780 IC₅₀ µM | A2780/DDP IC₅₀ µM | CH1 IC₅₀ µM | CH1/DDP IC₅₀ µM | SKOV3 IC₅₀ µM |
|---|---|---|---|---|---|---|---|
| **Cisplatine** | 3 | 2.4 | 9 | > 10 | 8 | >10 | 6.1 |
| **Carboplatine** | 3.3 | 9.7 | >10 | >10 | >10 | >10 | >10 |
| **Oxaliplatine** | 0.9 | 4 | 3.9 | 17.3 | 4.2 | 6.2 | >10 |
| **1** | 2.8 | 5.4 | nd | nd | nd | nd | nd |
| | 1.9 | 3.0 | | | | | |
| **2** | 1.5 | 2.5 | nd | nd | nd | nd | nd |
| **3** | 2.0 | 3.8 | 1.0 | 1.2 | 2.3 | 2.0 | 5.3 |
| **4** | 1.5 | 2.3 | nd | nd | nd | nd | nd |
| **5** | 1.2 | 1.7 | 0.9 | 1.8 | 2.4 | 2.4 | 6.5 |
| | 1.4 | 2.7 | | | | | |
| **6** | 1.7 | 2.8 | nd | nd | nd | nd | nd |
| **7** | 1.3 | 1.9 | 0.4 | 1.8 | 2.5 | 2.4 | 7.0 |
| **8** | 3.1 | 1.9 | nd | nd | nd | nd | nd |
| **9** | 2.7 | 1.6 | 1.8 | 1.4 | 2.2 | 2.1 | > 10 |
| **10** | 1.6 | 3.8 | 1.6 | 2.3 | nd | nd | 5.2 |
| **11** | 1.0 | 1.5 | 0.8 | 1.1 | nd | nd | 2.6 |
| **12** | 1.3 | 3.4 | 0.7 | 1.2 | nd | nd | 2.8 |
| **13** | 1.7 | 3.0 | nd | nd | nd | nd | nd |
| **14** | 1.7 | 2.6 | nd | nd | nd | nd | nd |
| **15** | 2.1 | 3.8 | nd | nd | nd | nd | nd |
| **16** | 1.4 | 4.1 | 1.1 | 2.7 | nd | nd | 4.1 |
| **17** | 1.8 | 1.1 | 1.1 | 2.2 | nd | nd | 5.0 |
| **18** | 1.4 | 2.7 | nd | nd | nd | nd | nd |
| **19** | 1.0 | 2.3 | nd | nd | nd | nd | nd |
| **20** | 2.1 | 4.4 | nd | nd | nd | nd | nd |
| **21** | 0.9 | 1.9 | nd | nd | nd | nd | nd |
| **22** | 1.2 | 1.8 | nd | nd | nd | nd | nd |
| **23** | 1.6 | 2.3 | nd | nd | nd | nd | nd |
| **24** | 0.9 | 2.1 | nd | nd | nd | nd | nd |
| **25** | 1.0 | 1.5 | nd | nd | nd | nd | nd |
| **26** | 1,2 | 2,9 | nd | nd | nd | nd | nd |
| **27** | 1,6 | 1,7 | nd | nd | nd | nd | nd |
| **28** | 1,4 | 1,6 | nd | nd | nd | nd | nd |
| **39** | 0,5 | 0,9 | nd | nd | nd | nd | nd |
| **30** | 1,1 | 1,3 | nd | nd | nd | nd | nd |
| **31** | 0,7 | 0,9 | nd | nd | nd | nd | nd |
| **32** | 1,2 | 1,4 | nd | nd | nd | nd | nd |
| **33** | 0,7 | 0,8 | nd | nd | nd | nd | nd |
| **34** | 1,4 | 3,1 | nd | nd | nd | nd | nd |
| **35** | 1,3 | 2,7 | nd | nd | nd | nd | nd |
| **36** | 0,6 | 0,9 | nd | nd | nd | nd | nd |
| **37** | 0,9 | 1,4 | nd | nd | nd | nd | nd |
| **38** | 2,2 | 3 | nd | nd | nd | nd | nd |
| **Produit de référence** | 3,9 | 4,4 | nd | nd | nd | nd | nd |

D'autres essais consistant à mesurer l'activité *in vivo* des composés de l'invention, notamment sur leucémies L1210 et L1210/DDP, ont été effectués.

Les composés selon l'invention peuvent donc être utilisés pour la préparation de médicaments, en particulier de médicaments inhibiteurs de la croissance de leucémies ou tumeurs solides chez l'homme, notamment de tumeurs solides ou liquides qui ne répondent pas de manière satisfaisante à une chimiothérapie à base de cisplatine, de carboplatine ou d'oxaliplatine.

Ainsi, selon un autre de ses aspects, l'invention a pour objet des médicaments qui comprennent un composé de formule (I), ou encore un hydrate ou un solvat du composé de formule (I).

Ces médicaments trouvent leur emploi en thérapeutique, notamment dans le traitement des cancers. Ils sont notamment actifs dans le traitement des cancers solides comme par exemple les cancers du tube digestif tels que les cancers gastriques, colorectaux, les cancers du poumon, du sein de la prostate ou les tumeurs liquides comme les leucémies.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, un hydrate ou solvat dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration sous-cutanée, intramusculaire, intra-veineuse, le principe actif de formule (I) ci-dessus, ou son solvat ou hydrate éventuel, peut être administré sous forme unitaire d'administration, en mélange avec des excipients pharmaceutiques classiques, aux animaux et aux êtres humains pour le traitement des troubles ou des maladies cancéreuses.

Les formes unitaires d'administration appropriées comprennent les formes d'administration parentérales notamment sous-cutanée, intramusculaire ou intraveineuse.

A titre d'exemple, une forme unitaire d'administration d'un composé selon l'invention sous forme de solution peut comprendre les composants suivants :

| | |
|---|---|
| Composé selon l'invention | 10 à 100 mg |
| Eau pour préparation injectable | 2 à 20ml |

Selon une autre forme de mise en oeuvre de l'invention le composé peut se présenter sous forme d'un lyophilisat.

On préfère utiliser les composés selon l'invention par voie intraveineuse à des doses comprises entre 10 et 200 mg/m². Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou hydrates ou solvats.

## Revendications

1. Complexes Platine - Carbène N-Hétérocyclique (NHC) portant en trans du
carbène un ligand azoté et répondant à la formule générale (I) 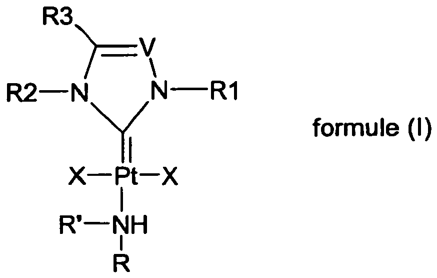 dans laquelle
- R₁ et/ou R₂ représentent indépendamment l'un de l'autre un groupe aryle ou aralkyle chacun éventuellement substitué, un groupe alkyle en C1-C6 linéaire ou ramifié, cycloalkyle monocyclique en C3-C7, alkényle en C2-C6 linéaire ou ramifié
- ou bien R' représente un atome d'hydrogène et R représente un groupe choisi parmi les groupes cycloalkyle, hétérocycloalkyle mono ou bicyclique en C3-C8 ou benzyle éventuellement substitué
- ou bien R et R' forment ensemble avec NH un hétérocycloalkyle en mono ou bicyclique en C3-C8
- V représente un atome d'azote ou un radical C-R₄
- R₃ et/ou R₄ représentent l'hydrogène, un groupe phényle ou R₃ et R₄ peuvent aussi former ensembles un radical alkylène en C3-C6, hétéroalkylène en C3-C6 avec un ou plusieurs hétéroatomes azotés, les atomes de carbones du radical hétéroalkylène pouvant être modifiés sous forme de radical carbonyle
- X représente l'iode, le brome, le chlore ou un groupe nitrato (-ONO2).

2. Composé de formule (I) selon la revendication 1, **caractérisé en ce que**
- R1 et/ou R2 représente indépendamment l'un de l'autre un groupe alkyle en C1-C4,
- et/ou V représente N ou C-R₄
- et/ou R3 et/ou R4 représente l'hydrogène ou forment ensemble un radical alkénylène
- et/ou R représente un motif cyclohexyle ou norbornyle et R' est H et/ou R et R' forment avec NH une morpholine
- et/ou X est l'iode.

3. Composé de formule (1) selon la revendication 1, **caractérisé en ce que**
- R1 et/ou R2 représente indépendamment l'un de l'autre un benzyle et/ou un alkyle ;
- et/ou V représente N ou C-R₄
- et/ou R3 et/ou R4 représente l'hydrogène ou forment ensemble un radical alkénylène ;
- et/ou R représente un motif cyclohexyle ou norbornyle et R' est H et/ou R et R' forment avec NH une morpholine
- et/ou X est l'iode.

4. Composé de formule (I) selon la revendication 1, **caractérisé en ce que**
- R1 et/ou R2 représente indépendamment l'un de l'autre un groupe cyclohexyleméthylène et/ou un groupe alkyle,
- et/ou V représente N ou C-R₄
- et/ou R3 et/ou R4 représente l'hydrogène ou forment ensemble un radical alkénylène
- et/ou R représente un motif cyclohexyle ou norbornyle et R' est H et/ou R et R' forment avec NH une morpholine
- et/ou X est l'iode.

5. Composés selon la revendication 1 ou 3 où le groupe benzyle est subtitué par un motif CF3 et/ou méthoxy.

6. Composés selon la revendication 1 sous forme d'hydrates ou de solvats

7. Médicament, **caractérisé en ce qu'**il comprend un composé de formule (I) selon l'une quelconque des revendications 1 à 3, un hydrate ou un solvat du composé de formule (I).

8. Composition pharmaceutique, **caractérisée en ce qu'**elle comprend un composé de formule (I) selon l'une quelconque des revendications 1 à 5, ou un sel pharmaceutiquement acceptable, un hydrate ou un solvat de ce composé, ainsi qu'au moins un excipient pharmaceutiquement acceptable.

9. Utilisation d'un composé de formule (I) selon l'une quelconque des revendications 1 à 4 pour la préparation d'un médicament destiné au traitement du cancer.

## Claims

1. N-Heterocyclic carbene (NHC)-platinum complexes bearing in trans of the carbene a nitrogen-containing ligand and corresponding to the general formula (I) 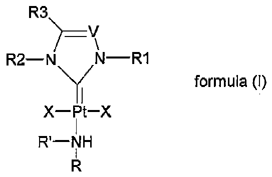 in which
- R₁ and/or R₂ represent independently of each other an aryl or aralkyl group, each optionally substituted, a linear or branched C1-C6 alkyl group, a monocyclic C3-C7 cycloalkyl group or a linear or branched C2-C6 alkenyl group
- or R' represents a hydrogen atom and R represents a group chosen from C3-C8 mono- or bicyclic cycloalkyl or heterocycloalkyl groups or an optionally substituted benzyl group
- or R and R' form together with NH a C3-C8 mono-or bicyclic heterocycloalkyl
- V represents a nitrogen atom or a C-R₄ radical
- R₃ and/or R₄ represent hydrogen, a phenyl group or R₃ and R₄ may also form together a C3-C6 alkylene or C3-C6 heteroalkylene radical with one or more nitrogen-based heteroatoms, it being possible for the carbon atoms of the heteroalkylene radical to be modified in the form of a carbonyl radical
- X represents iodine, bromine, chlorine or a nitrato (-ONO2) group.

2. Compound of formula (I) according to Claim 1, **characterized in that**
- R1 and/or R2 represents independently of each other a C1-C4 alkyl group,
- and/or V represents N or C-R₄
- and/or R3 and/or R4 represents hydrogen or form together an alkenylene radical
- and/or R represents a cyclohexyl or norbornyl unit and R' is H and/or R and R' form with NH a morpholine
- and/or X is iodine.

3. Compound of formula (I) according to Claim 1, **characterized in that**
- R1 and/or R2 represents independently of each other a benzyl and/or an alkyl;
- and/or V represents N or C-R₄
- and/or R3 and/or R4 represents hydrogen or form together an alkenylene radical;
- and/or R represents a cyclohexyl or norbornyl unit and R' is H and/or R and R' form with NH a morpholine
- and/or X is iodine.

4. Compound of formula (I) according to Claim 1, **characterized in that**
- R1 and/or R2 represents independently of each other a cyclohexylmethylene group and/or an alkyl group,
- and/or V represents N or C-R₄
- and/or R3 and/or R4 represents hydrogen or form together an alkenylene radical
- and/or R represents a cyclohexyl or norbornyl unit and R' is H and/or R and R' form with NH a morpholine
- and/or X is iodine.

5. Compounds according to Claim 1 or 3, where the benzyl group is substituted with a CF3 and/or methoxy unit.

6. Compound according to Claim 1, in the form of hydrates or solvates.

7. Medicament, **characterized in that** it comprises a compound of formula (I) according to any one of Claims 1 to 3, a hydrate or a solvate of the compound of formula (I).

8. Pharmaceutical composition, **characterized in that** it comprises a compound of formula (I) according to any one of Claims 1 to 5, or a pharmaceutically acceptable salt, a hydrate or a solvate of this compound, and at least one pharmaceutically acceptable excipient.

9. Use of a compound of formula (I) according to any one of Claims 1 to 4, for the preparation of a medicament intended for the treatment of cancer.

## Patentansprüche

1. Platin-Komplexe von N-heterozyklischem Carben (NHC), die in trans des Carbens einen stickstoffhaltigen Liganden tragen und der folgenden allgemeinen Formel (I) entsprechen 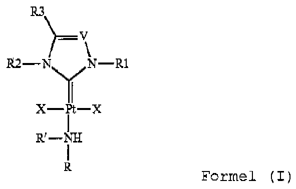 worin
- R₁ und/oder R₂ unabhängig voneinander für eine Aryl- oder Aralkylgruppe, die jeweils gegebenenfalls substituiert ist, eine gerade oder verzweigte C1-C6-Alkylgruppe, eine monozyklische C3-C7-Cycloalkylgruppe, eine gerade oder verzweigte C2-C6-Alkenylgruppe stehen
- oder R' für ein wasserstoffatom steht und R für eine Gruppe steht, die aus Cycloalkyl-, mono- oder bizyklischen C3-C8-Heterocycloalkyl- oder gegebenenfalls substituierten Benzylgruppen ausgewählt wird,
- oder R und R' zusammen mit NH ein mono- oder bizyklisches C3-C8-Heterocycloalkyl bilden
- V für ein Stickstoffatom oder einen Rest C-R₄ steht
- R₃ und/oder R₄ für Wasserstoff, eine Phenylgruppe stehen oder R₃ und R₄ auch zusammen einen C3-C6-Alkylenrest, C3-C6-Heteroalkylenrest mit einem oder mehreren Stickstoff-Heteroatomen bilden können, wobei die Kohlenstoffatome des Heteroalkylenrests in Form des Carbonylrests modifiziert sein können,
- X für Iod, Brom, Chlor oder eine Nitratestergruppe (-ONO2) steht.

2. Verbindung der Formel (I) nach Anspruch 1, **dadurch gekennzeichnet, dass**
- R₁ und/oder R₂ unabhängig voneinander für eine C1-C4-Alkylgruppe stehen,
- und/oder V für N oder C-R₄ steht
- und/oder R₃ und/oder R₄ für Wasserstoff stehen oder zusammen einen Alkenylenrest bilden
- und/oder R für ein Cyclohexyl- oder Norbornylmotiv steht und R' H ist und/oder R und R' zusammen mit NH ein Morpholin bilden
- und/oder X Iod ist.

3. Verbindung der Formel (I) nach Anspruch 1, **dadurch gekennzeichnet, dass**
- R₁ und/oder R₂ unabhängig voneinander für ein Benzyl und/oder ein Alkyl stehen,
- und/oder V für N oder C-R₄ steht
- und/oder R₃ und/oder R₄ für Wasserstoff stehen oder zusammen einen Alkenylenrest bilden
- und/oder R für ein Cyclohexyl- oder Norbornylmotiv steht und R' H ist und/oder R und R' zusammen mit NH ein Morpholin bilden
- und/oder X Iod ist.

4. Verbindung der Formel (I) nach Anspruch 1, **dadurch gekennzeichnet, dass**
- R₁ und/oder R₂ unabhängig voneinander für eine Cyclohexylmethylengruppe und/oder eine Alkylgruppe stehen,
- und/oder V für N oder C-R₄ steht
- und/oder R₃ und/oder R₄ für Wasserstoff stehen oder zusammen einen Alkenylenrest bilden
- und/oder R für ein Cyclohexyl- oder Norbornylmotiv steht und R' H ist und/oder R und R' zusammen mit NH ein Morpholin bilden
- und/oder X Iod ist.

5. Verbindungen nach Anspruch 1 oder 3, worin die Benzylgruppe mit einem CF3- oder Methoxy-Motiv substituiert ist.

6. Verbindungen nach Anspruch 1 in Form von Hydraten oder Solvaten.

7. Arzneimittel, **dadurch gekennzeichnet, dass** es eine Verbindung der Formel (I) nach einem der Ansprüche 1 bis 3, ein Hydrat oder ein Solvat der Verbindung der Formel (I) umfasst.

8. Pharmazeutische Zusammensetzung, **dadurch gekennzeichnet, dass** sie eine Verbindung der Formel (I) nach einem der Ansprüche 1 bis 5 oder ein pharmazeutisch annehmbares Salz, ein Hydrat oder ein Solvat dieser Verbindung sowie mindestens einen pharmazeutisch annehmbaren Excipienten umfasst.

9. Verwendung einer Verbindung der Formel (I) nach einem der Ansprüche 1 bis 4 zur Herstellung eines Arzneimittels für die Behandlung von Krebs.
